# EUROPÄISCHE PATENTANMELDUNG

(11) **EP 4 389 881 A1**
(43) Veröffentlichungstag der Anmeldung: **26.06.2024**
(21) Anmeldenummer: 23213199.5
(22) Anmeldetag: 30.11.2023
(51) Int. Cl.: C12N 9/04, C12P 7/18

(54) **GENETISCH MODIFIZIERTER MIKROORGANISMUS UND DESSEN VERWENDUNG ZUR HERSTELLUNG VON D-CHIRO-INOSITOL**

(30) Priorität: 15.12.2022 DE 102022004733
(71) Anmelder: Forschungszentrum Jülich GmbH, 52425 Jülich (DE)
(72) Erfinder: Ramp, Paul, 52062 Aachen (DE); Bott, Michael, 52428 Jülich (DE)

(57) **Zusammenfassung**

Die Erfindung betrifft einen genetisch modifizierten Mikroorganismus zur Herstellung von D-chiro-Inositol aus myo-Inositol oder Glukosebausteine enthaltenden Zuckern sowie Vektoren enthaltend wenigstens eine NAD⁺- und eine NADPH-abhängige Dehydrogenase zur Umsetzung von myo-Inositol zu D-chiro-Inositol. Weiter betrifft die vorliegende Erfindung Vektoren enthaltend wenigstens eine NAD⁺- und eine NADPH-abhängige Dehydrogenase zur Umsetzung von myo-Inositol zu D-chiro-Inositol. Ferner betrifft die vorliegende Erfindung ein Verfahren zur Herstellung von D-chiro-Inositol. Außerdem betrifft die vorliegende Erfindung Verwendungen erfindungsgemäß genetisch modifizierten Mikroorganismen, Vektoren, Proteine und sie kodierende Nukleinsäuresequenzen zur Herstellung von D-chiro-Inositol.

## Beschreibung

Die vorliegende Erfindung betrifft neue genetisch modifizierte Mikroorganismen, welche eine NAD⁺-abhängige und eine NADPH-abhängige Dehydrogenase exprimieren und welche Inositole aufnehmen und exportieren können und somit in neuen Verfahren zur Herstellung von D-*chiro*-Inositol (DCI) eingesetzt werden können. Die Erfindung betrifft außerdem neue Vektoren zur Herstellung der erfindungsgemäßen Mikroorganismen.

DCI ist ein Cyclitol (C₆H₁₂O₆) und eines von neun verschiedenen Isomeren von Inositol, umfassend *Myo-, Scyllo-, Allo-, Muco-, Epi-, Neo-, D-Chiro-, L-Chiro-* und cis-Inositol (Löpez-Gambero, A. J. et al. The biomedical uses of inositols: A nutraceutical approach to metabolic dysfunction in aging and neurodegenerative diseases. Biomedicine. 8, 295, 2020*;* Thomas, M. P. et al., "The Other" Inositols and Their Phosphates: Synthesis, Biology, and Medicine (with Recent Advances in myo-Inositol Chemistry). Angew Chem Int Edit. 55, 1614-1650, 2016*).*

Zusammen mit myo-Inositol (MI) reguliert DCI mehrere Stoffwechselwege und hormonelle Signalwege im menschlichen Körper, da sie an Zellwachstum, Überleben und Reproduktion beteiligt sind (Chiofalo, B. et al. Fasting as possible complementary approach for polycystic ovary syndrome: Hope or hype? Med Hypotheses. 105, 1-3, 2017*;* Dinicola, S. et al., The rationale of the myo-inositol and D-chiro-inositol combined treatment for polycystic ovary syndrome. J Clin Pharmacol. 54, 1079-1092, 2014*;* Kachhawa, G. et al., Efficacy of myo-inositol and D-chiro-inositol combination on menstrual cycle regulation and improving insulin resistance in young women with polycystic ovary syndrome: A randomized open-label study. Int J Gyncecol Obstet. Doi: 10. 1002/ijgo. 139712021, 2021*;* Vitale, S. G. et al., Myo-inositol supplementation to prevent gestational diabetes in overweight non-obese women: bioelectrical impedance analysis, metabolic aspects, obstetric and neonatal outcomes-a randomized and open-label, placebo-controlled clinical trial. Int J Food Sci Nutr. 72, 670-6792021, 2021).

Als Bestandteil von Inosit-Phosphoglykanen (IPGs) fungieren sowohl MI als auch DCI (MI-IPG und DCI-IPG) als Botenstoffe, die an der Insulinsignalübertragung beteiligt sind, wobei DCI-IPG hauptsächlich an der Regulierung der Glykogensynthese (GS) und der Aufrechterhaltung der Insulinempfindlichkeit beteiligt ist (Lamer, J., D-chiro-inositol-its functional role in insulin action and its deficit in insulin resistance. Int J Exp Diabetes Res. 3, 47-602002, 2002*;* Ortmeyer, H. K. et al., Chiroinositol deficiency and insulin resistance. I. Urinary excretion rate of chiroinositol is directly associated with insulin resistance in spontaneously diabetic rhesus monkeys. Endocrinology. 132, 640-6451993, 1993). Insulinresistente Personen, wie z. B. solche mit Typ-2-Diabetes oder Frauen mit polyzystischem Ovarialsyndrom (PCOS), einer facettenreichen Erkrankung, die durch ovarielle Dysfunktion, Unfruchtbarkeit, Hyperandrogenismus und namensgebende polyzystische Ovarien gekennzeichnet ist, weisen eine gestörte Freisetzung von DCI-IPG auf (Homburg, R., Polycystic ovary syndrome. Best Pract Res CI Ga. 22, 261-274.2008, 2008*;* Lamer, J. et al., D-chiro-inositol glycans in insulin signaling and insulin resistance. Mol Med. 16, 543-5522010, 2010*).*

DCI ist als Bestandteil von Inositol-Phosphoglykanen somit an der Regulierung der Glykogensynthese (GS) beteiligt und ist Teil eines Mediators, der an der Aufrechterhaltung der Insulinempfindlichkeit beteiligt ist (Nestler, J. E. et al., Reflections on inositol(s) for PCOS therapy: steps toward success. Gynecological Endocrinology. 31, 501-505, 2015*;* Paul, C., et al., Inositol's and other nutraceuticals' synergistic actions counteract insulin resistance in polycystic ovarian syndrome and metabolic syndrome: state-of-the-art and future perspectives. Gynecological Endocrinology. 32, 431-438, 2016*).* Klinische Studien, welche die gesundheitsfördernden Eigenschaften von DCI untersuchten, konnten zeigen, dass die orale Verabreichung niedriger Konzentrationen von DCI die allgemeine Insulinsensitivität im Gewebe von Typ-2-Diabetikern erhöhen, den Blutdruck, die Serumandrogene und die Plasmatriglyceride senken sowie den Eisprung bei Patientinnen mit polyzystischem Ovarialsyndrom (PCOS) fördern kann (Monastra, G. et al. Combining treatment with myo-inositol and D-chiro-inositol (40: 1) is effective in restoring ovary function and metabolic balance in PCOS patients. Gynecological Endocrinology. 33, 1-9, 2017; *Ortmeyer, H. K. et al.,* 1993).

Ein effektives Herstellungsverfahren für DCI ist erstrebenswert, da es als vielversprechender therapeutischer Kandidat für die Behandlung von Stoffwechselkrankheiten im Zusammenhang mit Insulinresistenz, Typ-2-Diabetes und PCOS angesehen wird (Gambioli, R. et al. The use of D-chiro-Inositol in clinical practice. European Review for Medical and Pharmacological Sciences. 25, 438-46, 2021)*.*

DCI wird hauptsächlich aus Pflanzen gewonnen, die reich an dessen 3-O-methyliertem Derivat D-Pinitol (3-O-Methyl-D-*chiro*-Inositol) sind, z. B. aus Hülsenfrüchten und Kiefernholz (Sanchez-Hidalgo, M. et al., D-Pinitol: A cyclitol with versatile biological and pharmacological activities. Phytochemistry Reviews. 20, 211-224, 2021*).*

Die industrielle Herstellung von DCI erfolgt durch Entfernung der 3-O-Methylgruppe von D-Pinitol aus Sojabohnen durch chemische Hydrolyse mit hochkonzentrierter Salzsäure, wie z.B. in US5827896A beschrieben.

DCI kann ebenfalls durch Hydrolyse des Antibiotikums Kasugamycin mit hochkonzentrierter Salzsäure gewonnen werden, wie z.B. in US5091596A und US5714643 beschrieben.

Die Hauptnachteile derartiger Verfahren der sauren Hydrolyse von D-Pinitol und Kasugamycin zur Gewinnung von DCI sind, dass D-Pinitol und Kasugamycin als Rohstoffe sehr teuer sind. Darüber hinaus führt die chemische Hydrolyse unter Verwendung hoher Säurekonzentrationen zu unerwünschten Nebenprodukten und chemischen Abfällen, die hohe Kosten für die ökologisch gerechte Abfallentsorgung verursachen.

Geeignete biotechnologische Herstellverfahren zur wirtschaftlichen und effizienten Herstellung von DCI in ökonomisch relevanten Mengen stehen bislang nicht zur Verfügung.

Bekannt ist, dass einige mikrobielle Spezies Inositole als einzelne Kohlenstoff- und Energiequelle nutzen können und MI und DCI durch Inositol-Dehydrogenasen enzymatisch umsetzen. In *B*. *subtilis* zum Beispiel werden MI und DCI zunächst über spezifische Transporter aufgenommen und dann von der Inositol-2-Dehydrogenase IolG zu den jeweiligen Keto-Produkten oxidiert. Darin oxidiert IolG in einer reversiblen Reaktion sowohl MI zu 2-Keto-*Myo-*Inositol (2KMI) als auch DCI zu 1-Keto-D-Chiro-Inositol (1 KDCI). Die Inosose-Isomerase IolI ist dafür zuständig, dass 2KMI und 1KDCI reversibel isomerisiert werden können. 2KMI wird dann über einen weiteren Abbauweg degradiert und als Energiequelle genutzt. Aufgrund der reversiblen Umsetzungsreaktion ist IolG darin grundsätzlich in der Lage, 1 KDCI unter Verwendung von NADH zurück zu DCI zu reduzieren und durch Verwendung der Inositol-Dehydrogenase IolG und der Inosose-Isomerase IolI besteht somit grundsätzlich eine Möglichkeit zur biotechnologischen Herstellung von DCI aus MI. Unter Verwendung eines gentechnisch veränderten *B*. subtilis-Stammes, der Inositole nicht abbauen kann und IolG und IolI überexprimiert, wurde mit diesem Verfahren allerdings lediglich die Herstellung von DCI aus MI mit einer Ausbeute von 6 % erreicht (Yoshida, K.-i. et al., Genetic modification of Bacillus subtilis for production of D-chiro-inositol, an investigational drug candidate for treatment of type 2 diabetes and polycystic ovary syndrome. Appl Environ Microbiol. 72, 1310-1315, 2006*;* Yoshida, K.-i. et al., Myo-Inositol catabolism in Bacillus subtilis. J. Biol. Chem. 283, 10415-10424, 2008).

Der maßgebliche Nachteil dieses Verfahrens liegt darin, dass eine effiziente Herstellung von DCI in ökonomisch relevanten Mengen damit nicht möglich ist. Zum einen basiert die diesem Verfahren zugrundeliegende enzymatische Reaktion darauf, dass die eingesetzte Inositol-Dehydrogenase zwei entgegengesetzte Reaktionen katalysiert, nämlich die bevorzugte Oxidation von MI zu 2KMI unter Nutzung von NAD⁺ als Kofaktor und dessen Reduktion zu NADH, aber auch die nicht begünstigte Reduktion des Keto-Zwischenprodukts 1 KDCI. Außerdem ist ein zusätzlicher Reaktionsschritt der Isomerisierung unter Verwendung der Inosose-Isomerase IolI erforderlich. Enzymatische Tests mit gereinigtem IolI zeigten ein Reaktionsgleichgewicht mit einem molaren Verhältnis von 77:23 zugunsten von 2KMI. Aufgrund des ungünstigen Reaktionsgleichgewichts, welches zu etwa 85 bis 87% auf der Seite der Ausgangsstoffe MI bzw. 2KMI liegt, wird der gewünschte Prozess der Umsetzung zu DCI behindert. Ein theoretischer Wert für eine Reaktionseffizienz auf Basis eines Enzym Assays wird mit maximal 13 bis 15 % angegeben. Allerdins müssten für die Erzielung einer solchen Effizienz extrem hohe Konzentration an Ausgangsstoffen (etwa 150 g/L MI) eingesetzt werden, um ökonomisch relevante Ausbeuten an DCI zu erhalten. Damit ist dieses Verfahren wirtschaftlich uninteressant.

Auch die US9725739B2 und die korrespondierende EP2902492A1 beschreiben ein mikrobielles Herstellverfahren zur Herstellung von DCI aus MI unter Verwendung einer transformierten Wirtszelle, die einen Inositol-Transporter, eine Inositol-Dehydrogenase und eine Inosose-Isomerase exprimiert und unterliegt damit den gleichen Nachteilen wie vorstehend beschrieben.

Außerdem bekannt ist die Inositol-2-Dehydrogenase IolW aus *B*. *subtilis,* die Keto-Inositol zu Inositol reduziert und dabei NADPH zu NADP⁺ oxidiert. Dabei setzt diese Inositol-Dehydrogenase IolW 2KMI lediglich zu scyllo-Inositol um, nicht zu DCI (P. Ramp et al., Metabolic engineering of Corynebacterium glutamicum for production of scyllo-inositol, a drug candidate against Alzheimer's disease, Metabolic Engineering 67 (2021) 173-185*).*

Pupel, P. et al., Two-step D-ononitol epimerization pathway in Medicago truncatula. The Plant Journal. 100, 237-250, 2019 beschreiben eine NAD⁺-abhängige Epimerase bzw. Dehydrogenase MtOEPa (*Medicago truncatula* Ononitol Epimerase a), welche Ononitol jedoch zu Methyl-1-keto-D-chiro-Inositol oxidiert, also einem Keto-Intermediat. Ferner wird eine weitere Epimerase beschrieben, nämlich eine NADPH-abhängige Dehydrogenase MtOEPb (*Medicago truncatula* Ononitol Epimerase b), welche das Keto-Intermediat zu D-Pinitol reduziert. Die Enzyme MtOEPa und MtOEPb wurden lediglich jeweils einzeln nach Expression in einem *Escherichia coli* Stamm aufgereinigt und ihre Aktivität für Ononitol und D-Pinitol charakterisiert.

Eine Aufgabe der vorliegenden Erfindung besteht somit darin, ein verbessertes Verfahren zur Bereitstellung von D-chiro-Inositol (DCI) zur Verfügung zu stellen, welches insbesondere die beschriebenen Nachteile des Stands der Technik überwindet. Das neue Verfahren soll den Einsatz unerwünschter hoher Säurekonzentrationen sowie das Auftreten unerwünschter Nebenprodukte und unerwünschter chemischer Abfälle, sowie die damit verbundenen hohen Kosten für ökologisch gerechte Abfallentsorgung vermeiden. Eine weitere Aufgabe der vorliegenden Erfindung ist es, ein neues biotechnologisches Herstellverfahren bereitzustellen, das sich gegenüber den bekannten mikrobiellen oder enzymatischen Prozessen durch eine gesteigerte Effizienz auszeichnet. Eine weitere Aufgabe der vorliegenden Erfindung ist die Bereitstellung eines Verfahrens, welches hinsichtlich der eingesetzten Ausgangsstoffe eine gewisse Variabilität ermöglicht und den Einsatz kostengünstiger und leicht verfügbarer Ausgangsstoffe erlaubt. Eine weitere Aufgabe der vorliegenden Erfindung ist die Bereitstellung geeigneter Vektoren und die Bereitstellung geeigneter genetisch veränderter Mikroorganismen, die eine enzymatische (in-vitro) oder biotechnologische (in-vivo) Herstellung von D-chiro-Inositol ermöglicht.

Problematisch bei der biotechnologischen Herstellung von D-chiro-Inositol ist dabei, die Ausgangssubstrate preiswert sind bzw. beim Einsatz von synthetisch hergestellten Ausgangsstoffen (z. B. Stoffwechselintermediaten oder Vorstufen) diese trotz möglicherweise hoher Bereitstellungskosten einen ökonomischen Herstellprozess ermöglichen müssen. Darüber hinaus müssen die Ausgangsstoffe (z. B. Stoffwechselintermediaten oder Vorstufen) zur Herstellung in lebenden Mikroorganismen auch von diesen aufgenommen und in die Zelle transportiert oder aus ihr heraus exportiert werden können. Außerdem muss bedacht werden, dass eine biotechnologische Herstellung von hochwertigen Metaboliten in Mikroorganismen oft eine heterologe Expression von Genen aus höheren Organismen, beispielsweise Pflanzen, erfordert. Zudem kann es problematisch sein, dass eine Vielzahl von Genen in dem zur Herstellung genutzten Mikroorganismus stabil, ggf. unter Selektionsdruck, eingebracht und über viele Replikationszyklen bei der biotechnologischen Herstellung erhalten werden müssen. Mikroorganismen mit mehr als 2 Vektoren oder mehr als 2 Genen pro Vektor sind häufig nicht stabil. Zudem sind Herstellungsprozesse unter Einsatz eines oder mehrerer sehr teurer Antibiotika nicht wirtschaftlich interessant und implizieren darüber hinaus ein Risikopotential für die Entstehung von resistenten Keimen in der Umwelt (z.B. über Abwässer). Ferner sollte das gewünschte Produkt, hier D-chiro-Inositol, oder seine Vorstufen nicht durch zelleigene Aktivitäten in den bereitzustellenden Mikroorganismen (Katabolismus) wieder zersetzt werden.

In diesem Zusammenhang ist es eine weitere Aufgabe der vorliegenden Erfindung die Voraussetzungen zu schaffen und Systeme bereitzustellen, die eine Herstellung von biologischen Katalysatoren, also Enzymen, ermöglicht mit denen auch eine rein enzymatische, also in-vitro-Herstellung von D-chiro-Inositol möglich ist.

Die oben beschriebenen Aufgaben werden durch die vorliegende Erfindung, wie nachfolgend ausgeführt, in vorteilhafter Weise gelöst.

Es folgt zunächst die kurze Beschreibung der vorliegenden Erfindung, ohne dass der Gegenstand der Erfindung dadurch limitiert wird.

Gegenstand der vorliegenden Erfindung ist die Bereitstellung eines genetisch modifizierten Mikroorganismus, der in der Lage ist myo-Inositol (MI) effizient zu D*-chiro-*Inositol (DCI) umzusetzen. Dabei umfasst die vorliegende Erfindung einen genetisch modifizierten Mikroorganismus, der eine NAD⁺-abhängige Dehydrogenase-Aktivität zur Umsetzung von *myo*-Inositol zu 1-Keto-D-*chiro*-Inositol und eine NADPH-abhängige Dehydrogenase-Aktivität zur Umsetzung von 1-Keto-D-*chiro*-Inositol zur D-*chiro*-Inositol exprimiert.

Erfindungsgemäß umfasst ist auch ein genetisch modifizierter Mikroorganismus, der zusätzlich Inositol-Transporter aufweist und/oder eine Nukleinsäuresequenz kodierend für Inositol-Tranporter exprimiert.

Gegenstand der vorliegenden Erfindung ist auch ein genetisch modifizierter Mikroorganismus, bei dem zusätzlich der Abbau von Inositolen ausgeschaltet ist, derart dass die Aktivität der am Abbau von Inositolen beteiligten Enzyme ausgeschaltet oder die für sie kodierenden Nukleinsäuren teilweise oder ganz aus dem Genom deletiert sind.

Gegenstand der vorliegenden Erfindung ist auch ein Vektor enthaltend eine Nukleinsäuresequenz kodierend für eine NAD⁺-abhängige Dehydrogenase mit einer Aktivität zur Umsetzung von myo-Inositol zu 1-Keto-D-chiro-Inositol und/oder eine Nukleinsäuresequenz kodierend für eine NADPH-abhängige Dehydrogenase mit einer Aktivität zur Umsetzung von 1-Keto-D-*chiro*-Inositol zur D-*chiro*-Inositol. Erfindungsgemäß umfasst ist auch ein Vektor, der zusätzlich Inositol-Transporter, aufweist und/oder eine Nukleinsäuresequenz kodierend für Inositol-Transporter, exprimiert. Weiter ist ein Vektor Gegenstand der vorliegenden Erfindung, der zur poly-cistronen Genexpression in Mikroorganismen, ausgewählt aus der Gruppe enthaltend coryneforme Bakterien und Enterobacteriaceae, geeignet ist, wobei er wenigstens zwei Expressionskassetten aufweist, enthaltend jeweils wenigstens einen Promotor, einen LacO-Operator, eine multiple-clonig-site (MCS), einen Transkriptionsterminator und eine Ribosomenbindungsstelle, wobei die Expressionskassetten für eine gleichgerichtete oder gegengerichtete Expression kodierender Nukleinsäuresequenzen angeordnet sind.

Gegenstand der vorliegenden Erfindung ist auch ein Verfahren zur biotechnologischen Herstellung von D-*chiro*-Inositol, umfassend
a) die Bereitstellung eines erfindungsgemäßen genetisch modifizierten Mikroorganismus,
b) die Kultivierung des erfindungsgemäß genetisch modifizierten Mikroorganismus aus a) in einem Medium welches ein geeignetes Ausgangssubstrat für die Bildung von D-*chiro*-Inositol enthält, ausgewählt aus *myo-*Inositol, Glukose oder Glukosebausteine enthaltenden Zuckern,
c) die Isolierung, und optional die Aufreinigung, des gebildeten D-*chiro*-Inositols (DCI).

Das erfindungsgemäße Verfahren beruht dabei auf zwei Umsetzungsschritten, die von zwei verschiedenen Enzymen katalysiert werden. Die Gesamtreaktion wird dabei durch die bevorzugten Reaktionen der Reduktion von NAD⁺ zu NADH und der Oxidation von NADPH zu NADP⁺ bei der Umwandlung von MI zu DCI angetrieben.
Die Reaktion ist im folgenden Schema dargestellt:

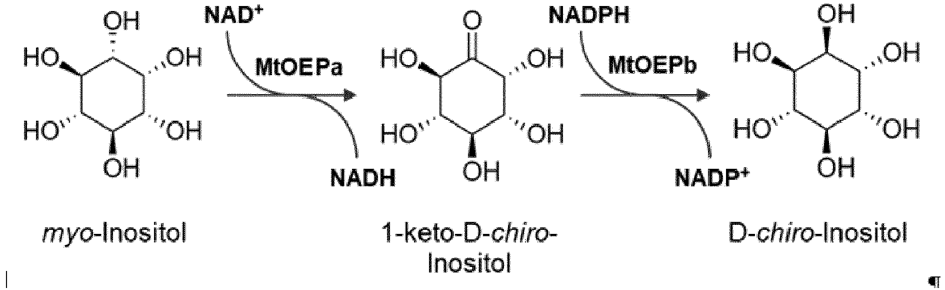

Als Ausgangsstoff des erfindungsgemäßen Verfahrens wird im allgemeinen myo-Inositol (MI) eingesetzt. Es ist jedoch erfindungsgemäß ebenfalls möglich, Glukose selbst oder Glukose enthaltende Zucker wie z.B. Saccharose oder Maltose als Ausgangsmaterial der Umsetzung zu DCI einzusetzen.

Die vorliegende Erfindung umfasst somit auch ein Verfahren der zuvor genannten Art, umfassend eine enzymatische Umsetzung ausgehend von Glukosebausteinen enthaltenden Zuckern, wie Saccharose, Glukose und Maltose, zu *myo-*Inositol, die während Schritt b) stattfindet. Durch die vorliegende Erfindung wird so eine höhere Variabilität bei der Auswahl der Ausgangsstoffe hin zu leicht verfügbaren und kostengünstigen Ausgangsstoffen ermöglicht, und damit insgesamt ein wirtschaftliches Verfahren zur Verfügung gestellt.

Gegenstand der vorliegenden Erfindung sind auch die Verwendung der erfindungsgemäßen genetisch veränderten Mikroorganismen und Vektoren sowie die erfindungsgemäße Verwendung der zuvor genannten Proteinen und Nukleinsäuresequenzen kodierend diese Proteine zur enzymatischen oder biotechnologischen Herstellung von DCI.

Weitere vorteilhafte Ausgestaltungen der Erfindung ergeben sich aus den Ansprüchen und darauf rückbezogene Unteransprüche.

Gegenstand der vorliegenden Erfindung ist ein genetisch modifizierter Mikroorganismus, der eine NAD⁺-abhängige Dehydrogenase-Aktivität zur Umsetzung von *myo-*Inositol zu 1-Keto-D-*chiro*-Inositol und eine NADPH-abhängige Dehydrogenase-Aktivität zur Umsetzung von 1-Keto-D-*chiro*-Inositol zur D-*chiro*-Inositol exprimiert.

In einer Variante der vorliegenden Erfindung ist auch ein genetisch modifizierter Mikroorganismus der zuvor genannten Art umfasst, der zusätzlich Inositol-Transporter aufweist und/oder Nukleinsäuresequenzen kodierend für Inositol-Transporter exprimiert. Dies gewährleistet die ausreichend hohe und schnelle Aufnahme geeigneter Substrate, wie myo-Inositol oder andere Vorstufen, wie unter anderen Glukosehaltige Zuckerbausteine, in die genetisch modifizierte Mikroorganismenzelle zur Umsetzung und Herstellung von D-chiro-Inositol.

In einer vorteilhaften Variante der vorliegenden Erfindung ist ein genetisch modifizierter Mikroorganismus umfasst, bei dem beispielsweise ein Inositol-Transporter ausgewählt aus der Gruppe enthaltend IolT1, IolT2, IolT, IolF und YfiG oder deren Homologe, exprimiert und/oder eine Nukleinsäuresequenz kodierend für Inositol-Transporter, ausgewählt aus der Gruppe enthaltend IolT1, IolT2, IolT, IolF und YfiG oder Homologe, aufweist. Coryneforme Bakterien, wie beispielsweise Corynebacterium glutamicum, weisen chromosomal-kodiert in den Genloci cg0223 (lolT1) und cg3387 (IolT2) einen Inositol-Transporter auf. In Bacillus subtilis sind die Inositol-Transporter IolT und IolF und YfiG bekannt. Aus Salmonella typhimurium sind IolT1 und iolT2 als Inositol-Transporter bekannt. Und aus Agrobacterium tumefaciens sind die Inositol-Tranporter mit den Genloci 5935 und 2525 bekannt. Vorteilhaft sind hier erfindungsgemäß Inositol-Transporter, wie beispielsweise ein myo-Inositol/Proton-Symporter IolT1 und/oder myo-Inositol/Proton-Symporter lolT2 oder deren Homologe aus Corynebacterium glutamicum und/oder eine Nukleinsäuresequenz kodierend für Inositol-Transporter, wie beispielsweise myo-Inositol/Proton-Symporter iolT1 und/oder myo-Inositol/Proton-Symporter iolT2 oder deren Homologe aus Corynebacterium glutamicum. Alle zuvor genannten Transporter ermöglichen in erfindungsgemäß vorteilhafter Weise ein Wachstum der erfindungsgemäß genetisch modifizieren Mikroorganismen in einem Kulturmedium ausgehend von oder enthaltend myo-Inositol.

In einer vorteilhaften Variante der vorliegenden Erfindung können in den genetisch modifizierten Mikroorganismen heterologe Nukleinsäuresequenzen kodierend für Inositol-Transporter exprimiert werden. Beispielsweise ist dies vorteilhaft, wenn der eingesetzte Mikroorganismus keine eigenen oder keine von einem gemeinschaftlich verwandtschaftlichen Ausgangsstamm (homologen) Nukleinsäuresequenzen aufweist. Vorteilhaft im Sinne der Erfindung ist hier die Vektor-kodierte Expression eines heterologen Inositol-Transporters in einem erfindungsgemäß genetisch modifizierten Mikroorganismus. Dies erfolgt in vorteilhafter Weise durch heterologe Expression der besagten kodierenden Nukleinsäuresequenzen mit geeigneten Expressionsvektoren in den erfindungsgemäß genetisch modifizierten Mikroorganismen. Erfindungsgemäß umfasst sind beispielsweise pEKEx2, pMKEx2 für coryneforme Bakterien oder pET, pET-DUET1 für Enterobacteriaceae. Diese vorherigen Auflistungen sind jedoch nur beispielhaft und nicht limitierend für die vorliegende Erfindung. Für jeden erfindungsgemäß vorteilhaften Mikroorganismus können entsprechend etablierte Vektorsysteme, die dem Fachmann bekannt sind, eingesetzt werden, die dann die erfindungsgemäßen kodierenden Nukleinsäuresequenzen tragen.

In einer weiteren vorteilhaften Variante der vorliegenden Erfindung sind genetisch modifizierte Mikroorganismen umfasst, die einen myo-Inositol/Proton-Symporter, wie beispielsweise IolT1 oder lolT2 gemäß SEQ ID NO. 7 oder 8 und deren Homologe aus coryneformen Bakterien aufweisen bzw. exprimieren. In einer besonders vorteilhaften Variante der vorliegenden Erfindung sind genetisch modifizierte Mikroorganismen umfasst, die einen myo-Inositol/Proton-Symporter IolT1 gemäß SEQ ID NO. 7 und dessen Homologe aus coryneformen Bakterien exprimieren.

Erfindungsgemäß gleichermaßen vorteilhaft ist die homologe Expression eines Inositol-Transporters in einem erfindungsgemäß genetisch modifizierten Mikroorganismus, die sowohl Vektor-kodiert oder chromosomal-kodiert erfolgen kann.

Regulationsmechanismen bezüglich der Expression von chromosomal-kodierten Nukleinsäuresequenzen für einen Inositol-Transporter sind dabei aufzuheben bzw. auszuschalten. Beispielsweise befindet sich IolT1 in Corynebacterium glutamicum im Genlocus cg0223 und seine Expression wird durch einen Regulator IolR (cg0196) kontrolliert, d.h. reprimiert. Erfindungsgemäß wird in einem genetisch modifizierten coryneformen Mikroorganismus durch die Deletion des Genlocus cg0196-cg0212, welcher die kodierende Nukleinsäuresequenz für besagten Repressor (lolR; cg0196) enthält, ausgeschaltet. Dies wiederum resultiert in der konstitutiven (homologen) Expression des Inositol-Transporters IolT1 mit den beschriebenen Vorteilen, u.a. des effektiven Transports von myo-Inositol in die Zellen des erfindungsgemäß genetische modifizierten Mikroorganismus.

In einer weiteren vorteilhaften Variante der vorliegenden Erfindung sind genetisch modifizierte coryneforme Mikroorganismen umfasst, die einen myo-Inositol/Proton-Symporter, wie beispielsweise IolT1 oder lolT2 gemäß SEQ ID NO. 7 oder 8 und deren Homologe aufweisen bzw. exprimieren. In einer besonders vorteilhaften Variante der vorliegenden Erfindung sind genetisch modifizierte coryneforme Mikroorganismen umfasst, die einen myo-Inositol/Proton-Symporter IolT1 gemäß SEQ ID NO. 7 und dessen Homologe exprimieren.

Unter "homolog" im Sinne der Erfindung ist zu verstehen, dass die erfindungsgemäßen Proteine und die sie kodierenden Nukleinsäuresequenzen verwandtschaftlich von einem gemeinsamen Ausgangsstamm abstammen. "Homolog" wird erfindungsgemäß synonym benutzt mit dem Begriff "nicht heterolog" und "nicht rekombinant".

In einer weiteren vorteilhaften Variante des erfindungsgemäß genetisch modifizierten Mikroorganismus ist zusätzlich der Abbau von Inositolen ausgeschaltet, und zwar derart, dass die Aktivität der am Abbau von Inositolen beteiligten Enzyme ausgeschaltet oder die für sie kodierenden Nukleinsäuren teilweise oder ganz aus dem Genom deletiert sind.

Somit wird in der Zelle des erfindungsgemäß genetisch modifizierten Mikroorganismus in vorteilhafter Weise überhaupt erste eine Bildung des gewünschten D-chiro-Inositols ermöglicht, welches anderenfalls unverzüglich wieder katabolisiert würde.

Zu den für die vorliegende Erfindung relevanten Inositolen und deren Isomeren, die nicht abgebaut werden sollen, sind bevorzugt *myo-*Inositol, 1-Keto-D-*chiro*-Inositol und D-*chiro*-Inositol zu verstehen. In weiteren vorteilhaften Varianten der erfindungsgemäß genetisch modifizierten Mikroorganismen ist zusätzlich auch der Abbau von Inositolen und deren Isomeren ausgeschaltet, ausgewählt aus der Gruppe enthaltend 2-Keto-*myo-*inositol, *scyllo*-inositol, L*-chiro*-Inositol, D-Ononitol und D-Pinitol. Dabei handelt es sich um eine nicht abschließende Aufzählung an Verbindungen.

Die vorliegende Erfindung fasst in einer weiteren vorteilhaften Variante einen genetisch modifizierten Mikroorganismus, bei dem die Genloci der Nukleinsäuresequenzen cg0196-cg0212, cg2312-cg2313 und cg3389-cg3392 teilweise oder ganz aus dem Genom deletiert sind.

Gegenstand der vorliegenden Erfindung ist auch ein genetisch modifizierter Mikroorganismus, der eine Aminosäuresequenz einer NAD⁺-abhängigen Dehydrogenase mit einer Identität von wenigstens 80% zur Aminosäuresequenz gemäß SEQ ID NO. 1 und eine Aminosäuresequenz einer NADPH-abhängigen Dehydrogenase mit einer Identität von wenigstens 80% zur Aminosäuresequenz gemäß SEQ ID NO. 2 aufweist.

Ebenfalls erfindungsgemäß umfasst ist ein genetisch modifizierter Mikroorganismus, der eine NAD⁺-abhängige Dehydrogenase kodiert durch eine Nukleinsäuresequenz mit einer Identität von wenigstens 70% gemäß SEQ ID NO. 3 und eine NADPH-abhängige Dehydrogenase kodiert durch eine Nukleinsäuresequenz mit einer Identität von wenigstens 70% gemäß SEQ ID NO. 4 aufweist.

In einer vorteilhaften Variante des erfindungsgemäß genetisch modifizierten Mikroorganismus weist dieser eine Aminosäuresequenz der NAD⁺-abhängigen Dehydrogenase gemäß SEQ ID NO. 1 und eine Aminosäuresequenz der NADPH-abhängigen Dehydrogenase gemäß SEQ ID NO. 2 aufweist.

In einer weiteren Variante der vorliegenden Erfindung stammt die Aminosäuresequenz der NAD⁺-abhängigen Dehydrogenase gemäß SEQ ID NO. 1 und die Aminosäuresequenz der NADPH-abhängigen Dehydrogenase gemäß SEQ ID NO. 2 aus Pflanzen, bevorzugt Medicago truncatula.

Alle aus Pflanzen oder anderen heterologen Systemen abgeleiteten Gene können für die Expression in Mikroorganismen, wie beispielsweise in coryneformen Bakterien oder Enterobacteriaceae, an die bakterielle Codonverwendung (Codon-Usage) dieser Mikroorganismen angepasst und optimiert werden. Der Anteil heterologer Nukleinsäuren wird dadurch erfindungsgemäß reduziert und die Expression in der Bakterienzelle in vorteilhafter Weise unterstützt. Denn es ist bekannt, dass verschiedene Spezies Varianten des universellen genetischen Codes mit unterschiedlicher Häufigkeit verwenden, was letztendlich auf unterschiedliche tRNA-Konzentrationen innerhalb der Zelle zurückzuführen ist. Selten verwendete Codons können die Translation ausbremsen, während häufiger genutzte Codons die Translation beschleunigen können.

Erfindungsgemäß werden daher die heterologen Gene mit der Codonverwendung spezifisch für den Zielorganismus synthetisiert. Hierzu wird die Aminosäuresequenz des heterologen Proteins von Interesse in die DNA-Sequenz mit spezifischer Codonverwendung umgeschrieben.

Erfindungsgemäß umfasst ist auch ein genetisch modifizierter Mikroorganismus der zuvor genannten Art ausgewählt aus der Gruppe enthaltend coryneforme Bakterien, bevorzugt Corynebacteriaceae oder Brevibacteriaceae, Bacillaceae, Enterobacteriaceae, Zymomonadaceae, Methylobacteriaceae, Burkholderiaceae, Vibrionaceae, Clostridiaceae, Pseudomonadaceae und Acetobacteraceae. In einer weiteren Variante sind genetisch modifizierte Mikroorganismen umfasst, ausgewählt aus der Gruppe enthaltend Corynebacterium, Brevibacterium, Bacillus, Lactobacillus, Lactococcus, Escherichia, Zymomonas, Methylobacterium, Ralstonia, Vibrio, Clostridium, Pseudomonas und Gluconobacter. In einer weiteren vorteilhaften Variante handelt es sich erfindungsgemäß um einen genetisch modifizierten Mikroorganismus basierend auf dem Stamm Escherichia coli BL21 (DE3).

Eine weitere vorteilhafte Variante umfasst einen genetisch modifizierten Mikroorganismus gemäß der vorliegenden Erfindung, ausgewählt aus der Gruppe enthaltend Corynebacterium glutamicum, Corynebacterium acetoglutamicum, Brevibacterium flavum, Brevibacterium lactofermentum und Brevibacterium divaricatum. In einer weiteren vorteilhaften Variante handelt es sich erfindungsgemäß um einen genetisch modifizierten Mikroorganismus, ausgewählt aus der Gruppe enthaltend Corynebacterium glutamicum ATCC13032, Corynebacterium glutamicum MB001(DE3), Corynebacterium acetoglutamicum ATCC15806, Corynebacterium acetoacidophilum ATCC13870, Brevibacterium flavum ATCC14067, Brevibacterium lactofermentum ATCC13869 und Brevibacterium divaricatum ATCC14020.

In einer vorteilhaften Variante der vorliegenden Erfindung ist ein genetisch modifizierter Mikroorganismus umfasst, der eine NAD⁺-abhängige Dehydrogenase kodiert durch eine Nukleinsäuresequenz gemäß SEQ ID NO. 5 und eine NADPH-abhängige Dehydrogenase kodiert durch eine Nukleinsäuresequenz gemäß SEQ ID NO. 6 exprimiert. Dabei handelt es sich um eine vorteilhafte Variante, in der die zuvor genannten Nukleinsäuresequenzen aus Pflanzen an die Codon-Verwendung (Codon-Usage) coryneformer Bakterien optimal adaptiert sind. Für *Corynebacterium glutamicum* ist eine Datenbank über die Codonverwendung verfügbar unter http://www.kazusa.or.jp/codon/cgibin/showcodon.cgi?species=196627&aa=1&style=N.

Im Sinne der vorliegenden Erfindung wird sowohl ausgehend von diese Codon-optimierten Nukleinsäuresequenz als auch des pflanzlichen Gens die erfindungsgemäße Enzymaktivität gleichermaßen in den erfindunsgemäß genetisch modifizierten Mikroorganismen exprimiert und ist somit nicht limitierend für die vorliegende Erfindung. Die Bezeichnung MtOEPa, OEPa, NAD⁺-abhängige Dehydrogenase und MI-Dehydrogenase sowohl für die erfindungsgemäßen Nukleinsäuresequenzen und der durch sie kodierten NAD⁺-abhängige Dehydrogenase, welche die Umsetzung von *myo*-Inositol zu 1-Keto-D-*chiro*-Inositol katalysiert (MI-Dehydrogenase), synonym verwendet.

Im Sinne der vorliegenden Erfindung wird sowohl ausgehend von diese Codon-optimierten Nukleinsäuresequenz als auch des pflanzlichen Gens die erfindungsgemäße Enzymaktivität gleichermaßen in den erfindunsgemäß genetisch modifizierten Mikroorganismen exprimiert und ist somit nicht limitierend für die vorliegende Erfindung. Die Bezeichnung MtOEPb, OEPb, NADPH-abhängige Dehydrogenase und 1KDCI-Dehydrogenase sowohl für die erfindungsgemäßen Nukleinsäuresequenzen und der durch sie kodierten NADPH-abhängige Dehydrogenase, welche die Umsetzung von 1-Keto-D-*chiro*-Inositol zur D-*chiro*-Inositol katalysiert (1 KDCI-Dehydrogenase), synonym verwendet.

Gegenstand der vorliegenden Erfindung sind auch Varianten eines genetisch modifizierten Mikroorganismus, der zusätzlich zu den zuvor beschriebenen Modifikationen eine gesteigerte enzymatische Aktivität einer myo-Inositol-1-Phosphat-Synthase (Ino1) aufweist, so dass der erfindungsgemäß genetisch modifizierte Mikroorganismus ausgehend von Glukosebausteinen enthaltenden Zuckern, wie Saccharose, Glukose oder auch Maltose, diese über Glukose-6-Phosphat zu myo-Inositol-1-Phosphat umsetzt.

Im Sinne der vorliegenden Erfindung ist "gesteigert" gleichbedeutend mit "erhöht", oder "verbessert", "verändert" oder "dereguliert" zu verstehen und wird synonym verwendet. "Gesteigert" im Sinne der vorliegenden Erfindung meint beispielsweise die gesteigerte Genexpression eines Gens im Vergleich zu der Expression des jeweiligen Ausgangsgens im unveränderten, natürlicherweise nicht gesteigerten Zustand. Gleiches ist im Sinne der Erfindung bezüglich einer erhöhten Enzymaktivität gemeint.

Eine weitere Variante eines erfindungsgemäß genetisch modifizierten Mikroorganismus zeichnet sich dadurch aus, dass eine gesteigerte Genexpression auf Veränderungen beruht, ausgewählt aus der Gruppe enthaltend a) Veränderung der Regulation oder von Signalstrukturen zur Genexpression, b) Veränderung der Transkriptionsaktivität der kodierenden Nukleinsäuresequenz, oder c) Erhöhung der Genkopienzahl der kodierenden Nukleinsäuresequenz, d) Erhöhung der Stabilität der mRNA abgeleitet von der kodierenden Nukleinsäuresequenz, oder e) eine Veränderung der katalytischen Aktivität und/oder Substratspezifität der erfindungsgemäß eingesetzen Enzyme für die Herstellung von D-chiro-Inositol, oder f) eine Kombination von a) - e).

Erfindungsgemäß umfasst sind dabei Veränderung der Signalstrukturen der Genexpression, wie beispielsweise durch Veränderung der Repressorgene, Aktivatorgene, Operatoren, Promotoren; Attenuatoren, Ribosomenbindungsstellen, des Startkodons, Terminatoren. Ebenso umfasst sind das Einführen eines stärkeren Promoters, wie beispielsweise des tac-Promotors oder eines IPTG-induzierbaren Promotors. Das Einführen eines stärkeren Promoters, wie z. B. den tac-Promoter (Amann et al (Gene 1988 69:301-15), oder Promotoren aus der Gruppe der bei Patek et al (Microbiology 1996 142:1297) beschriebenen Promotoren ist bevorzugt, aber nicht limitierend für die vorliegende ERfindung. Weitere Beispiele finden sich in der WO 96/15246 oder in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Pátek et al. (Microbiology 142: 1297 (1996)), in Knippers ("Molekulare Genetik", 6th Edition, Georg Thieme Verlag, Stuttgart, Germany, 1995) oder auch bei Winnacker ("Gene und Klone", VCH Verlagsgesellschaft, Weinheim, Germany, 1990).

Eine erhöhte Kopienzahl der erfindungsgemäß kodierenden Nukleinsäuresequenz kann in weiteren Varianten der Erfindung chromosomal-kodiert und/oder extrachromosomal-kodiert, bevorzugt Vektor-kodiert, weiter bevorzugt Plasmid-kodiert, vorliegen. Erfindungsgemäß eignen sich als Vektorsysteme solche, die in coryneformen Bakterien und Enterobacteriaceae repliziert werden. Zahlreiche bekannte Plasmidvektoren wie z. B. pZ1 (Menkel et al., Applied and Environmental Microbiology (1989) 64: 549-554), pEKEx1 (Eikmanns et al., Gene 102:93-98 (1991)) oder pHS2-1 (Sonnen et al., Gene 107:69-74 (1991)) beruhen auf den kryptischen Plasmiden pHM1519, pBL1 oder pGA1. Andere Plasmidvektoren wie z. B. solche, die auf pCG4 (US-A 4,489, 160), oder pNG2 (Serwold-Davis et al., FEMS Microbiology Letters 66, 119-124 (1990)), oder pAG1 (US-A 5, 158,891) beruhen, können in gleicher Weise verwendet werden (O. Kirchner 2003, J. Biotechnol. 104:287-99). Ebenso können Vektoren mit regulierbarer Expression benutzt werden, wie zum Beispiel pEKEx2 (B. Eikmanns, 1991 Gene 102:93-8; O. Kirchner 2003, J. Biotechnol. 104:287-99) oder pMKEx2 (Kortmann, Maike, et al. "A chromosomally encoded T 7 RNA polymerase-dependent gene expression system for C orynebacterium glutamicum: construction and comparative evaluation at the single-cell level"; Microbial biotechnology 8.2 (2015): 253-265. ).

Auch kann eine kodierende Nukleinsäuresequenz durch Integration in das Chromosom in einfacher Kopie (P. Vasicova 1999, J. Bacteriol. 181:6188-91), oder mehrfacher Kopie (D. Reinscheid 1994 Appl. Environ Microbiol 60:126-132) exprimiert werden.

Die Transformation eines gewünschten Mikroorganismus mit einem Vektor zur Erhöhung der Kopienzahl erfolgt durch Konjugation oder Elektroporation des gewünschten Stammes von beispielsweise C. glutamicum. Die Methode der Konjugation ist beispielsweise bei Schäfer et al. (Applied and Environmental Microbiology (1994) 60:756-759) beschrieben. Methoden zur Transformation sind beispielsweise bei Tauch et al. (FEMS Microbiological Letters (1994)123:343-347) beschrieben.

Die Erhöhung der mRNA-Stabilität kann beispielsweise durch Mutation der terminalen Positionen erreicht werden, welche den Abbruch der Transkription steuern. Maßnahmen, die zu einer Veränderung der katalytischen Eigenschaften von Enzymproteinen führen, insbesondere zu einer veränderten Substratspezifität sind aus dem Stand der Technik bekannt. Neben erfindungsgemäß bevorzugten partiellen oder kompletten Deletionen regulatorischer Strukturen sind erfindungsgemäß auch Veränderungen, wie z. B. Transitionen, Transversionen oder Insertionen umfasst, sowie Methoden der gerichteten Evolution. Anleitungen zur Erzeugung derartiger Veränderungen können bekannten Lehrbüchern (R. Knippers "Molekulare Genetik", 8. Auflage, 2001, Georg Thieme Verlag, Stuttgart, Deutschland) entnommen werden.

In weiteren Varianten des erfindungsgemäß genetisch modifizierten Mikroorganismus weist dieser zusätzlich eine Aktivität einer putativen myo-Inositol-1-Phosphat-Phosphatase (Inositol-Monophosphatase) auf, so dass er myo-Inositol-1-Phosphat zu freiem myo-Inositol umsetzt. Die erfindungsgemäß vorteilhaften Mikroorganismen enthalten alle wenigstens ein, manchmal mehrere Gene kodierend für Enzyme mit Inositol-Monophosphatasen-Aktivität, die konstitutiv exprimiert werden.

Die vorliegende Erfindung löst so mit der Bereitstellung der genetisch modifizierten Mikroorganismen der zuvor beschriebenen Art in vorteilhafter Weise das Problem, dass nicht nur synthetisch hergestellte und somit teure Ausgangssubstanzen für die Herstellung von D-chiro-Inositol eingesetzt werden müssen. Ein Vorteil der vorliegenden Erfindung ist insbesondere, dass hinsichtlich der eingesetzten Ausgangsstoffe als Substrate für die biotechnologische Herstellung von D-chiro-Inositol eine große Varianz möglich ist. Besonders vorteilhaft ist es, dass durch die vorliegende Erfindung neben myo-Inositol auch der Einsatz kostengünstiger und leicht verfügbarer Ausgangsstoffe aus Glukosebausteinen, wie Saccharose, Glukose oder Maltose, für Herstellung von D-chiro-Inositol eingesetzt werden können, wodurch Nachteile aus dem Stand der Technik aufgehoben und bestehende Probleme in vorteilhafter Weise gelöst werden.

In einer Variante der vorliegenden Erfindung weist der erfindungsgemäße genetisch modifizierte Mikroorganismus unter andern eine myo-Inositol-1-Phosphat-Synthase (Ino1) mit einer Aminosäuresequenz von wenigstens 80% Identität gemäß SEQ ID NO: 11 bzw. eine Nukleinsäuresequenz (ino1) kodierend für eine myo-Inositol-1-Phosphat-Synthase von wenigstens 70% Identität gemäß SEQ ID NO: 12 auf.

In einer vorteilhaften Variante des erfindungsgemäßen genetisch veränderten Mikroorganismus weist dieser eine gesteigerte Expression des Gens (ino1) für eine myo-Inositol-1-Phosphat-Synthase (Ino1) auf, die beispielsweise durch eine erhöhte Kopienzahl der kodierenden Nukleinsäuresequenz begründet ist. Dabei kann die erhöhte Kopienzahl chromosomal-kodiert und/oder extrachromosomal-kodiert, bevorzugt Vektor-kodiert, vorliegen.

In einer weiteren Variante der vorliegenden Erfindung weist der erfindungsgemäße genetisch modifizierte Mikroorganismus der zuvor beschriebenen Art eine putative myo-Inositol-1-Phosphat-Phosphatase (Inositol-Monophosphatase) mit einer Aminosäuresequenz von wenigstens 80% Identität gemäß SEQ ID NO: 13 bzw. eine Nukleinsäuresequenz kodierend für eine putative myo-Inositol-1-Phosphat-Phosphatase (Inositol-Monophosphatase) von wenigstens 70% Identität gemäß SEQ ID NO: 14.

Gegenstand der vorliegenden Erfindung sind genetisch modifizierte Mikroorganismen der erfindungsgemäßen Art, die die Nukleinsäuresequenzen kodierend für eine NAD⁺-abhängige Dehydrogenase-Aktivität, für eine NADPH-abhängige Dehydrogenase-Aktivität, für Inositol-Transporter, für eine myo-Inositol-1-Phosphat-Synthase (Ino1) und/oder für eine putative myo-Inositol-1-Phosphat-Phosphatase (Inositol-Monophosphatase) chromosomal-kodiert und/oder extrachromosomal-kodiert, bevorzugt Vektor-kodiert, aufweisen.

Dabei ist erfindungsgemäß die Expression sowohl homologer als auch heterologer kodierender Nukleinsäuresequenzen umfasst. Plasmid-kodiert ist die Expression sowohl homologer als auch heterologer Nukleinsäuresequenzen umfasst. Chromosomal-kodierte Nukleinsäuresequenzen basieren vorteilhafter Weise auf homologen Genen oder zumindest auf für den jeweiligen Mikroorganismus und seine Codon-Usage optimierten Nukleinsäuresequenzen.

Im Sinne der vorliegenden Erfindung ist neben der Variante der Plasmid-kodierten Expression der erfindungsgemäßen Nukleinsäuresequenzen auch die weitere Möglichkeit für die Produktion von D-chiro-Inositol erfindungsgemäß umfasst, die eine Plasmid-freie Expression der entsprechenden Gene durch Integration in das Genom des erfindungsgemäßen Mikroorganismus umfasst. Hierfür werden unter anderen die beiden erfindungsgemäßen für die NAD⁺- und NADPH-abhängige Dehydrogenase kodierenden Nukleinsäuresequenzen mit zwei 500-1000 bp langen HomologieFlanken entsprechend der Integrationsstelle in pK19mobsacB kloniert. Die Integration kann über zweifache homologe Rekombination nach *Niebisch und Bott 2001* (https://doi.org/10.1007/s002030100262) mit dem Vektor pK19mobsacB (Schäfer et al., 1994) (https://doi.org/10.1016/0378-1119(94)90324-7) erfolgen.

Erfindungsgemäß liegen in einer vorteilhaften Variante genetisch modifizierter coryneformer Mikroorganismen beispielsweise die Gene kodierend für Inositol-Transporter oder Inositol-Monophosphatase chromosomal-kodiert vor und werden konstitutiv exprimiert. Sofern ihre chromosomale Expression übergeordnet durch Regulatoren reguliert wird, wird diese Regulation durch entsprechende genetische Modifikationen dereguliert, was ebenfalls in einer konstitutiven Expression resultiert. Ebenso liegen in einer weiteren vorteilhaften Variante genetisch modifizierter coryneformer Mikroorganismen beispielsweise die Gene kodierend für eine myo-Inositol-1-Phosphat-Synthase (Ino1) und/oder für eine putative myo-Inositol-1-Phosphat-Phosphatase (Inositol-Monophosphatase) chromosomal-kodiert vor und werden konstitutiv exprimiert. In einen besonders vorteilhaften Variante liegt das Gen kodierend für eine myo-Inositol-1-Phosphat-Synthase (Ino1) zusätzlich extrachromosomal, also Vektor- oder Plasmid-kodiert, vor, um eine ausreichend hohe, also gesteigerte enzymatische Aktivität der myo-Inositol-1-Phosphat-Synthase (Ino1) zu erreichen.

In einer vorteilhaften Variante der vorliegenden Erfindung ist ein genetisch modifizierter Mikroorganismus umfasst,
a) der eine NAD⁺-abhängige Dehydrogenase-Aktivität zur Umsetzung von *myo-*Inositol zu 1-Keto-D-*chiro*-Inositol und eine NADPH-abhängige Dehydrogenase-Aktivität zur Umsetzung von 1-Keto-D-*chiro*-Inositol zur D-*chiro*-Inositol exprimiert und
b) der zusätzlich Inositol-Transporter aufweist und/oder eine Nukleinsäuresequenz kodierend für Inositol-Transporter exprimiert, und
c) bei dem die Aktivität der am Abbau von Inositolen beteiligten Enzyme ausgeschaltet, oder bei dem die sie kodierenden Nukleinsäuresequenzen teilweise oder ganz aus dem Genom deletiert sind, und
d) der zusätzlich eine gesteigerte enzymatische Aktivität einer myo-Inositol-1-Phosphat-Synthase (Ino1) aufweist, und
e) der zusätzlich eine Aktivität einer putativen myo-Inositol-1-Phosphat Phosphatase (Inositol-Monophosphatase) aufweist.

In einer weiteren vorteilhaften Variante der vorliegenden Erfindung ist ein genetisch modifizierter Mikroorganismus umfasst,
a) der eine NAD⁺-abhängige Dehydrogenase-Aktivität mit einer Identität von wenigstens 80% zur Aminosäuresequenz gemäß SEQ ID NO. 1 zur Umsetzung von *myo-*Inositol zu 1-Keto-D-*chiro*-Inositol und eine NADPH-abhängige Dehydrogenase-Aktivität mit einer Identität von wenigstens 80% zur Aminosäuresequenz gemäß SEQ ID NO. 2 zur Umsetzung von 1-Keto-D-chiro-Inositol zur D-*chiro*-Inositol exprimiert, bzw.
b) der eine NAD⁺-abhängige Dehydrogenase kodiert durch eine Nukleinsäuresequenz mit einer Identität von wenigstens 70% gemäß SEQ ID NO. 3 und eine NADPH-abhängige Dehydrogenase kodiert durch eine Nukleinsäuresequenz mit einer Identität von wenigstens 70% gemäß SEQ ID NO. 4 aufweist, und
c) der Inositol-Transporter, ausgewählt aus der Gruppe enthaltend IolT1, IolT2, IolT, iolF und YfiG, aufweist und/oder eine Nukleinsäuresequenz kodierend für diese Inositol-Transporter exprimiert, und
d) bei dem die kodierenden Nukleinsäuresequenzen für den am Abbau von Inositolen beteiligten Enzyme teilweise oder ganz aus dem Genom deletiert sind, und
e) der zusätzlich eine gesteigerte enzymatische Aktivität einer myo-Inositol-1-Phosphat-Synthase (Ino1) mit einer Aminosäuresequenz von wenigstens 80% Identität gemäß SEQ ID NO: 11 aufweist, bzw.
f) der zusätzlich eine Nukleinsäuresequenz kodierend für eine myo-Inositol-1-Phosphat-Synthase (Ino1) von wenigstens 70% Identität gemäß SEQ ID NO: 12 in erhöhter Kopienzahl exprimiert, und
g) der zusätzlich eine Aktivität einer putativen myo-Inositol-1-Phosphat Phosphatase (Inositol-Monophosphatase) mit einer Aminosäuresequenz von wenigstens 80% Identität gemäß SEQ ID NO: 13 aufweist, bzw.
h) der zusätzlich eine Nukleinsäuresequenz kodierend für eine putative myo-Inositol-1-Phosphat-Phosphatase (Inositol-Monophosphatase) von wenigstens 70% Identität gemäß SEQ ID NO: 14. exprimiert.

In Varianten des zuvor beschriebenen genetisch modifizierten Mikroorganismus handelt es sich bei Merkmal c) um eigene (homologe) oder heterologe Nukleinsäuresequenzen kodierend für einen Inositol-Transporter. Die homologen Nukleinsäuresequenzen liegen dabei vorteilhafter Weise chromosomal-kodiert vor. Die heterologen Nukleinsäuresequenzen liegen dabei vorteilhafter Weise extrachromosomal, also Vektor- oder Plasmid-kodiert, vor. Vorteilhafte Inositol-Transporter sind IolT1 bzw. lolT2 aus coryneformen Bakterien sowie IolT, iolF oder YfiG aus Bacillaceae.

In einer weiteren vorteilhaften Variante der vorliegenden Erfindung ist ein genetisch modifizierter Mikroorganismus der Gattung coryneformer Bakterien umfasst,
a) der eine NAD⁺-abhängige Dehydrogenase-Aktivität mit einer Identität von wenigstens 80% zur Aminosäuresequenz gemäß SEQ ID NO. 1 zur Umsetzung von *myo-*Inositol zu 1-Keto-D-*chiro*-Inositol und eine NADPH-abhängige Dehydrogenase-Aktivität mit einer Identität von wenigstens 80% zur Aminosäuresequenz gemäß SEQ ID NO. 2 zur Umsetzung von 1-Keto-D-*chiro*-Inositol zur D-*chiro*-Inositol exprimiert, bzw.
b) der eine NAD⁺-abhängige Dehydrogenase kodiert durch eine Nukleinsäuresequenz mit einer Identität von wenigstens 70% gemäß SEQ ID NO. 3 und eine NADPH-abhängige Dehydrogenase kodiert durch eine Nukleinsäuresequenz mit einer Identität von wenigstens 70% gemäß SEQ ID NO. 4 aufweist, und
c) der eine Nukleinsäuresequenz kodierend für einen myo-Inositol/Proton-Symporter (lolT1) gemäß SEQ ID NO. 7 exprimiert, und
d) der eine Nukleinsäuresequenz kodierend für einen myo-Inositol/Proton-Symporter (IolT2) gemäß SEQ ID NO. 8 exprimiert, und
e) bei dem die Nukleinsäuresequenzen cg0196-cg0212, cg2312-cg2313 und cg3389-cg3392 teilweise oder ganz aus dem Genom deletiert sind, und
f) der zusätzlich eine gesteigerte enzymatische Aktivität einer myo-Inositol-1-Phosphat-Synthase (Ino1) mit einer Aminosäuresequenz von wenigstens 80% Identität gemäß SEQ ID NO: 11 aufweist, bzw.
g) der zusätzlich eine Nukleinsäuresequenz kodierend für eine myo-Inositol-1-Phosphat-Synthase (Ino1) von wenigstens 70% Identität gemäß SEQ ID NO: 12 in erhöhter Kopienzahl exprimiert, und
h) der zusätzlich eine Aktivität einer putativen myo-Inositol-1-Phosphat Phosphatase (Inositol-Monophosphatase) mit einer Aminosäuresequenz von wenigstens 80% Identität gemäß SEQ ID NO: 13 aufweist, bzw.
i) der zusätzlich eine Nukleinsäuresequenz kodierend für eine putative myo-Inositol-1-Phosphat-Phosphatase (Inositol-Monophosphatase) von wenigstens 70% Identität gemäß SEQ ID NO: 14 exprimiert.

In einer weiteren vorteilhaften Variante der vorliegenden Erfindung ist ein genetisch modifizierter Mikroorganismus von Corynebacterium, vorteilhaft Corynebacterium glutamicum MB001(DE3) umfasst,
a) der eine NAD⁺-abhängige Dehydrogenase-Aktivität gemäß SEQ ID NO. 1 zur Umsetzung von *myo-*Inositol zu 1-Keto-D-*chiro*-Inositol und eine NADPH-abhängige Dehydrogenase-Aktivität gemäß SEQ ID NO. 2 zur Umsetzung von 1-Keto-D-*chiro*-Inositol zur D-*chiro*-Inositol exprimiert, bzw.
b) der eine NAD⁺-abhängige Dehydrogenase kodiert durch eine Nukleinsäuresequenz gemäß SEQ ID NO. 5 und eine NADPH-abhängige Dehydrogenase kodiert durch eine Nukleinsäuresequenz gemäß SEQ ID NO. 6 aufweist, und
c) der eine Nukleinsäuresequenz kodierend für einen myo-Inositol/Proton-Symporter (lolT1) gemäß SEQ ID NO. 7 exprimiert, und
d) der eine Nukleinsäuresequenz kodierend für einen myo-Inositol/Proton-Symporter (IolT2) gemäß SEQ ID NO. 8 exprimiert, und
e) bei dem die Nukleinsäuren cg0196-cg0212, cg2312-cg2313 und cg3389-cg3392 teilweise oder ganz aus dem Genom deletiert sind, und
f) der zusätzlich eine gesteigerte enzymatische Aktivität einer myo-Inositol-1-Phosphat-Synthase (Ino1) mit einer Aminosäuresequenz gemäß SEQ ID NO: 11 aufweist, bzw.
g) der zusätzlich eine Nukleinsäuresequenz kodierend für eine myo-Inositol-1-Phosphat-Synthase (Ino1) gemäß SEQ ID NO: 12 in erhöhter Kopienzahl exprimiert, und
h) der zusätzlich eine Aktivität einer putativen myo-Inositol-1-Phosphat Phosphatase (Inositol-Monophosphatase) mit einer Aminosäuresequenz gemäß SEQ ID NO: 13 aufweist, bzw.
i) der zusätzlich eine Nukleinsäuresequenz kodierend für eine putativen myo-Inositol-1-Phosphat-Phosphatase (Inositol-Monophosphatase) gemäß SEQ ID NO: 14 exprimiert.

In einer weiteren vorteilhaften Variante der vorliegenden Erfindung ist ein genetisch modifizierter Mikroorganismus der Gattung Escherichia, vorteilhaft E. coli BL21 (DE3) umfasst,
a) der eine NAD⁺-abhängige Dehydrogenase-Aktivität mit einer Identität von wenigstens 80% zur Aminosäuresequenz gemäß SEQ ID NO. 1 zur Umsetzung von *myo-*Inositol zu 1-Keto-D-*chiro*-Inositol und eine NADPH-abhängige Dehydrogenase-Aktivität mit einer Identität von wenigstens 80% zur Aminosäuresequenz gemäß SEQ ID NO. 2 zur Umsetzung von 1-Keto-D-*chiro*-Inositol zur D-*chiro*-Inositol exprimiert, bzw.
b) der eine NAD⁺-abhängige Dehydrogenase kodiert durch eine Nukleinsäuresequenz mit einer Identität von wenigstens 70% gemäß SEQ ID NO. 3 und eine NADPH-abhängige Dehydrogenase kodiert durch eine Nukleinsäuresequenz mit einer Identität von wenigstens 70% gemäß SEQ ID NO. 4 aufweist, und
c) der Inositol-Transporter, ausgewählt aus der Gruppe enthaltend IolT1, IolT2, IolT, iolF und YfiG, aufweist und/oder eine Nukleinsäuresequenz kodierend für diese Inositol-Transporter exprimiert, und
d) bei dem die kodierenden Nukleinsäuren für den am Abbau von Inositolen beteiligten Enzyme teilweise oder ganz aus dem Genom deletiert sind, und
e) der zusätzlich eine gesteigerte enzymatische Aktivität einer myo-Inositol-1-Phosphat-Synthase (Ino1) mit einer Aminosäuresequenz von wenigstens 80% Identität gemäß SEQ ID NO: 11 aufweist, bzw.
f) der zusätzlich eine Nukleinsäuresequenz kodierend für eine myo-Inositol-1-Phosphat-Synthase (Ino1) von wenigstens 70% Identität gemäß SEQ ID NO: 12 in erhöhter Kopienzahl exprimiert, und
g) der zusätzlich eine Aktivität einer putativen myo-Inositol-1-Phosphat Phosphatase (Inositol-Monophosphatase) mit einer Aminosäuresequenz von wenigstens 80% Identität gemäß SEQ ID NO: 13 aufweist, bzw.
h) der zusätzlich eine Nukleinsäuresequenz kodierend für eine putative myo-Inositol-1-Phosphat-Phosphatase (Inositol-Monophosphatase) von wenigstens 70% Identität gemäß SEQ ID NO: 14 exprimiert.

In Varianten des zuvor beschriebenen genetisch modifizierten Mikroorganismus handelt es sich bei Merkmal c) um eigene (homologe) oder heterologe Nukleinsäuresequenzen kodierend für einen Inositol-Transporter. Die homologen Nukleinsäuresequenzen liegen dabei vorteilhafter Weise chromosomal-kodiert vor. Die heterologen Nukleinsäuresequenzen liegen dabei vorteilhafter Weise extrachromosomal, also Vektor- oder Plasmid-kodiert, vor. Vorteilhafte Inositol-Transporter sind IolT1 bzw. lolT2 aus coryneformen Bakterien sowie IolT, iolF oder YfiG aus Bacillaceae.

Gegenstand der vorliegenden Erfindung ist auch ein Vektor enthaltend eine Nukleinsäuresequenz kodierend für eine NAD⁺-abhängige Dehydrogenase mit einer Aktivität zur Umsetzung von *myo*-Inositol zu 1-Keto-D-*chiro*-Inositol und/oder eine Nukleinsäuresequenz kodierend für eine NADPH-abhängige Dehydrogenase mit einer Aktivität zur Umsetzung von 1-Keto-D-*chiro*-Inositol zur D-chiro-Inositol.

Erfindungsgemäß denkbar sind dabei zwei verschiedene Vektoren mit unterschiedlichen Antibiotika-Selektionsmarkern und je einer kodierenden Nukleinsäuresequenz der zuvor genannten Art, die zur Expression in einem erfindungsgemäßen genetisch modifizierten Mikroorganismus geeignet sind. Oder in einer anderen Variante handelt es sich um einen Vektor mit einem Antibiotika-Selektionsmarker, der mehrere kodierende Nukleinsäuresequenzen zur Expression in einem erfindungsgemäßen genetisch modifizierten Mikroorganismus bereitstellt. Im Sinne der Erfindung kann ein Vektor auch ein Plasmid sein.

In einer Variante der vorliegenden Erfindung ist auch ein Vektor umfasst, der zusätzlich Inositol-Transporter exprimiert und/oder eine Nukleinsäuresequenz kodierend für Inositol-Transporter aufweist. In einer vorteilhaften Variante der vorliegenden Erfindung ist ein Vektor umfasst, bei dem beispielsweise ein Inositol-Transporter ausgewählt aus der Gruppe enthaltend IolT1, IolT2, IolT, IolF und YfiG oder deren Homologe, exprimiert und/oder eine Nukleinsäuresequenz kodierend für Inositol-Transporter, ausgewählt aus der Gruppe enthaltend IolT1, IolT2, IolT, IolF und YfiG oder Homologe, aufweist. Coryneforme Bakterien, wie beispielsweise Corynebacterium glutamicum, weisen chromosomal-kodiert in den Genloci cg0223 (lolT1) und cg3387 (IolT2) einen Inositol-Transporter auf. In Bacillus subtilis sind die Inositol-Transporter IolT und IolF und YfiG bekannt. Aus Salmonella typhimurium sind IolT1 und iolT2 als Inositol-Transporter bekannt. Und aus Agrobacterium tumefaciens sind die Inositol-Tranporter mit den Genloci 5935 und 2525 bekannt. Vorteilhaft sind hier erfindungsgemäß Vektoren enthaltend Inositol-Transporter, wie beispielsweise ein myo-Inositol/Proton-Symporter IolT1 und/oder myo-Inositol/Proton-Symporter lolT2 oder deren Homologe aus Corynebacterium glutamicum und/oder eine Nukleinsäuresequenz kodierend für Inositol-Transporter, wie beispielsweise myo-Inositol/Proton-Symporter iolT1 und/oder myo-Inositol/Proton-Symporter iolT2 oder deren Homologe aus Corynebacterium glutamicum. Alle zuvor genannten Transporter ermöglichen in erfindungsgemäß vorteilhafter Weise ein Wachstum der erfindungsgemäß genetisch modifizieren Mikroorganismen in einem Kulturmedium ausgehend von oder enthaltend myo-Inositol.

Eine weitere Variante der vorliegenden Erfindung umfasst auch einen Vektor, der eine NAD⁺-abhängige Dehydrogenase kodiert durch eine Nukleinsäuresequenz mit einer Identität von wenigstens 70% gemäß SEQ ID NO. 3 und/oder eine NADPH-abhängige Dehydrogenase kodiert durch eine Nukleinsäuresequenz mit einer Identität von wenigstens 70% gemäß SEQ ID NO. 4 aufweist.

In einer weiteren Variante der vorliegenden Erfindung ist ein Vektor umfasst, wobei die eine NAD⁺-abhängige Dehydrogenase kodierende DNA-Sequenz und die eine NADPH-abhängige Dehydrogenase kodierende DNA-Sequenz aus Pflanzen stammen, bevorzugt aus Medicago truncatula. Alle aus Pflanzen oder anderen heterologen Systemen abgeleiteten Gene können für die Expression in Mikroorganismen, wie beispielsweise in coryneformen Bakterien oder Enterobacteriaceae, an die bakterielle Codonverwendung (Codon-Usage) dieser Mikroorganismen angepasst und optimiert werden. Dies wurde bereits zuvor ausführlich erläutert. Vektoren enthaltend solche an die Codon-Usage des jeweiligen Mikroorganismus, in dem der Vektor exprimiert werden soll, entsprechend angepassten kodierenden Nukleinsäuresequenzen sind alle von der vorliegenden Erfindung umfasst.

Erfindungsgemäß sind alle Varianten des Vektors zur Verwendung und zur Expression in Mikroorganismen ausgewählt aus der Gruppe enthaltend Corynebacteriaceae, Brevibacteriaceae und Enterobacteriaceae geeignet. Vorteilhaft sind coryneforme Bakterien, wie Corynebacterien und Brevibacterien. Bei coryneformen Bakterien, bevorzugt der Gattung Corynebacterium, handelt es sich um einen "Generally Recognized As Safe" (GRAS)-Organismus, welcher in allen industriellen Bereichen eingesetzt werden kann. Coryneforme Bakterien erreichen auf definierten Medien hohe Wachstumsraten und Biomasseerträge (Grünberger et al., 2012) und es existiert umfangreiche Erfahrung im industriellen Einsatz coryneformer Bakterien (Becker et al., 2012). Besonders vorteilhaft ist Corynebacterium glutamicum und besonders vorteilhaft ist Corynebacterium glutamicum MB001 (DE3) sowie Escherichia coli und besonders vorteilhaft Eschericha coli BL21 (DE3) als Wirt für einen erfindungsgemäßen Vektor.

Gegenstand der vorliegenden Erfindung ist auch ein Vektor, der eine NAD⁺-abhängige Dehydrogenase kodiert durch eine Nukleinsäuresequenz gemäß SEQ ID NO. 5 und/oder eine NADPH-abhängige Dehydrogenase kodiert durch eine Nukleinsäuresequenz gemäß SEQ ID NO. 6 aufweist. Dabei handelt es sich bei den SEQ ID NO. 5 und 6 um kodierende Nukleinsäuresequenzen, die an die Codon-Usage coryneformer Bakterien angepasst sind. Dies wurde bereits zuvor ausführlich erläutert. Vektoren enthaltend solche an die Codon-Usage des jeweiligen Mikroorganismus, in dem der Vektor exprimiert werden soll, entsprechend angepassten kodierenden Nukleinsäuresequenzen sind alle von der vorliegenden Erfindung umfasst. Jedoch erfüllen auch alle anderen Varianten der erfindungsgemäßen Nukleinsäuresequenzen und erfindungsgemäßen Vektoren, enthaltend besagte erfindungsgemäßen Nukleinsäuresequenzen, alle vorteilhaften Eigenschaften der vorliegenden Erfindung. Die an die Codon-Usage coryneformer Bakterien angepassten Varianten der Nukleinsäuresequenzen sind somit nicht limitierend für die vorliegende Erfindung.

Erfindungsgemäß umfasst sind auch ein Vektor, der Nukleinsäuresequenzen kodierend für Inositol-Transporter, vorteilhafter Weise ausgewählt aus der Gruppe enthaltend IolT1, IolT2, IolT, iolF und YfiG aufweist.

In einer weiteren Variante der vorliegenden Erfindung ist auch ein Vektor umfasst, der eine Nukleinsäuresequenz kodierend für myo-Inositol-1-Phosphat-Synthase (Ino1) von wenigstens 70% Identität gemäß SEQ ID NO: 12 aufweist.

Dabei kann es sich im Sinne der vorliegenden Erfindung um verschieden Vektoren handeln, die in den erfindungsgemäß genetisch modifizierten Mikroorganismen repliziert und exprimiert werden. Zum anderen kann es sich erfindungsgemäß auch um Vektoren handeln, die alle der zuvor genannten erfindungsgemäß vorteilhaften kodierenden Nukleinsäuresequenzen in einem Vektor vereint.

In einer besonders vorteilhaften Variante der vorliegenden Erfindung ist auch ein Vektor umfasst, der eine NAD⁺-abhängige Dehydrogenase kodiert durch eine Nukleinsäuresequenz mit einer Identität von wenigstens 70% gemäß SEQ ID NO. 3 und eine NADPH-abhängige Dehydrogenase kodiert durch eine Nukleinsäuresequenz mit einer Identität von wenigstens 70% gemäß SEQ ID NO. 4, und eine Nukleinsäuresequenz kodierend für eine myo-Inositol-1-Phosphat-Synthase (Ino1) SEQ ID NO: 12 aufweist.

In einer besonders vorteilhaften Variante der vorliegenden Erfindung ist auch ein Vektor umfasst, der eine NAD⁺-abhängige Dehydrogenase kodiert durch eine Nukleinsäuresequenz gemäß SEQ ID NO. 5, und eine NADPH-abhängige Dehydrogenase kodiert durch eine Nukleinsäuresequenz gemäß SEQ ID NO. 6, und eine Nukleinsäuresequenz kodierend für eine myo-Inositol-1-Phosphat-Synthase (Ino1) SEQ ID NO: 12 aufweist.

Vorteilhafte Varianten der vorliegenden Erfindung basieren dabei auf Derivaten ausgehend von dem Plasmid pMKEx2, das nachfolgend konkret beschrieben ist. Als besonders vorteilhafte Ausgestaltungen der erfindungsgemäßen Vektoren sind die Plasmide pOEPa-b bzw. pInoDCI zu nennen. In Sinne der vorliegenden Erfindung werden als Bezeichnungen für die bevorzugten erfindungsgemäßen Plasmide z.B. pOEPa-b und pMKEx2-OEPa-b synonym verwendet. D.h., pOEPa-b ist eine Abkürzung für pMKEx2-OEPa-b. pMKEx2 ist dabei die Bezeichnung des Ausgangsplasmids, in welches die Gene erfindungsgemäß kloniert wurden. In Sinne der vorliegenden Erfindung werden als Bezeichnungen für die bevorzugten erfindungsgemäßen Plasmide z.B. pInoDCI und pMKEx2-InoDCI synonym verwendet. D.h., pInoDCI ist eine Abkürzung für pMKEx2-InoDCI. pMKEx2 ist dabei die Bezeichnung des Ausgangsplasmids, in welches die Gene erfindungsgemäß kloniert wurden.

Gegenstand der vorliegenden Erfindung ist auch ein Vektor zur poly-cistronen Genexpression in Mikroorganismen ausgewählt aus der Gruppe enthaltend coryneforme Bakterien und Enterobacteriaceae, der wenigstens zwei Expressionskassetten aufweist, jeweils enthaltend wenigstens einen Promotor, einen LacO-Operator, eine multiple-cloning-site (MCS) sowie einen Transkriptionsterminator und eine Ribosomenbindungsstelle, wobei die Expressionskassetten für eine gleichgerichtete oder gegengerichtete Expression der kodierenden Nukleinsäuresequenzen angeordnet sind. Darüber hinaus weist dieser Vektor einen Replikationsursprung (Origin of Replication) für coryneforme Bakterien, bevorzugt Cornybacterium (oric._{g}), und Enterobakterien, bevorzugt Escherichia (ori_{E.c.}), auf.

In vorteilhafter Weise zeichnet sich diese Variante des Vektors dadurch aus, dass zwei komplette sogenannte Multiple Cloning Sites vorhanden sind, die jeweils unterschiedliche Restriktionsstellen besitzen, damit unabhängig voneinander in jede Expressionskassette eine gewünschte kodierende Nukleinsäuresequenz (Cistron) kloniert werden kann.

Im Sinne der vorliegenden Erfindung werden die Begriffe "Nukleinsäuresequenz" und "DNA-Sequenz" synonym verwendet. Ebenso werde vorliegend werden "kodierende Nukleinsäuresequenz", "Gen" und "Cistron" synonym verwendet.

In einer Variante des erfindungsgemäßen Vektors können die Promotoren der Expressionskassetten so angeordnet sein, dass die Inititation der Transkription durch die RNA-Polymerase für alle kodierenden Nukleinsäuren in der Multiple Cloning Site in die gleiche Transkriptionsrichtung erfolgt. In einer weiteren Variante der Erfindung können die Promotoren der Expressionskassetten in einer entgegengesetzten Transkriptionsrichtung ausgerichtet sein. In einer vorteilhaften Variante der Erfindung weist der erfindungsgemäße Vektor zur poly-cistronen Expression wenigstens zwei Expressionskassetten auf, die für eine gegengerichtete Expression angeordnet sind. In vorteilhafter Weise kann so die Effizienz der Expression der kodierenden Nukleinsäuren der vorliegenden Erfindung optimiert bzw. gesteigert werden, was sich ebenfalls positiv auf die Produktion des gewünschten Produktes D-chiro-Inositol auswirkt.

In einer weiteren Variante der vorliegenden Erfindung wurde ein Vektor bereitgestellt, bei dem die Promotoren der Expressionskassetten mit einem (dem Fachmann bekannten) genügend großen Abstand zueinander, beispielsweise zwischen 50 bis 1000 Nukleotiden, in den Vektor kloniert wurden, sodass die Initiation der Transkription durch die RNA-Polymerase gleichzeitig an beiden Promotoren sich nicht gegenseitig beeinträchtigt. Dabei ist es unabhängig davon, ob eine gleichgerichtete oder gegengerichtete Expression der Cistrone erfolgt.

In einer vorteilhaften Variante der vorliegenden Erfindung sind die Promotoren der Expressionskassetten mit ausreichendem Abstand und in gegengerichteter Transkriptionsrichtung angeordnet.

Ein Vorteil der erfindungsgemäßen Varianten des Vektors zur poly-cistronen Expression von kodierenden Nukleinsäuren im Sinne der vorliegenden Erfindung ist, dass die wenigstens zwei Promotoren auf einem Vektor die gleichzeitige und gleichstarke Expression von mehreren Genen erlaubt, bei gleichzeitigem Einsatz nur eines einzigen Antibiotika-Selektionsmarkers. Es ist bekannt, dass Mikroorganismen enthaltend zwei oder mehr Vektoren oder mehr als 2 kodierenden Nukleinsäuresequenzen (Cistrone) pro Vektor oft nicht sehr stabil sind. Problematisch ist dabei auch, dass hintereinander angeordnete kodierende Nukleinsäuresequenzen in einer einzelnen Expressionskassette schlechter exprimiert werden, je weiter sie vom Promotorelement entfernt liegen. Aufgrund dessen löst der erfindungsgemäße Vektor zur poly-cistronen Expression in coryneformen Bakterien und Enterobakterien und seine beschriebenen Varianten der vorliegenden Erfindung einige der Erfindung zugrundeliegenden Probleme aus dem Stand der Technik in vorteilhafter Weise. Unter dem Einsatz nur eines Antibiotika-Selektionsmarkers sind die Varianten der vorliegenden Erfindung in Mikroorganismen über viele Replikationszyklen bei der biotechnologischen Herstellung gewünschter Produkte sehr stabil. Gleichzeitig ermöglicht die vorliegende Erfindung mit diesen Vektoren alle Anforderungen an einen preiswerteren und damit wirtschaftlich interessanteren biotechnologischen Herstellungsprozess im Gegensatz zum herkömmlichen Einsatz mehrerer Antibiotika in bekannten Verfahren aus dem Stand der Technik. Gleichzeitig minimiert die vorliegende Erfindung mit ihren Vektoren der zuvor genannten Art in vorteilhafter Weise ein nicht zu vernachlässigendes Risiko für die Entstehung von resistenten Keimen in der Umwelt, z.B. über stark Antibiotika-belastete Abwässer.

Die Varianten des erfindungsmäßen Vektors zur poly-cistronen Expression in coryneformen Bakterien und Enterobacteriaceae reduziert somit in vorteilhafter Weise die Kosten bei deren Einsatz in der Produktion von D-chiro-Inositol. Zudem wird der erfindungsgemäß genetisch modifizierte Mikroorganismus durch die Kultivierung mit nur einem Antibiotika-Selektionsmarker deutlich weniger in seiner Physiologie beeinflusst, was sich ebenfalls auf eine verbesserte Expression der erfindungsgemäßen Nukleinsäuren auswirkt, verbunden mit einer besseren Produktivität mit Blick auf das gewünschte bei Produkt D-chiro-Inositol.

Ferner zeichnet sich der erfindungsgemäße poly-cistrone Vektor in vorteilhafter Weise dadurch aus, dass beide Promoteren durch den gleichen Operator (z. B. lacO) reguliert werden, so dass nur ein Induktor (z. B. IPTG) erforderlich ist, um die Vektor-kodierte Genexpression zu starten. Dies stellt einen weiteren Vorteil der vorliegenden Erfindung dar, der sich reduzierend auf die Kosten zur Herstellung, die Umweltbelastung und somit eine gesteigerte Ökonomie der Produktion von D-chiro-Inositol auswirkt. Der erfindungsgemäße Einsatz eines induzierbaren Promotors hat ferner den Vorteil, dass die Expression der für die gewünschten Metabolite erforderlichen kodierenden Nukleinsäuresequenzen gezielt gesteuert, d.h. angeschaltet werden können, beispielsweise in Abhängigkeit von den Wachstums- bzw. Kultivierungsbedingungen der erfindungsgemäßen genetisch modifizierten Mikroorganismen.

Gegenstand der vorliegenden Erfindung ist auch ein poly-cistroner Vektor der zuvor beschriebenen Art, enthaltend starke Promotoren, vorteilhaft wenigstens einen trp-Promotor oder einen lac-Promotor oder einen T7-Promotor oder Kombinationen davon. Erfindungsgemäß umfasst sind somit Kombinationen der Promotoren ausgewählt aus der Gruppe enthaltend trp-lac, trp-T7, trp-trp, lac-trp, lac-T7, lac-lac, T7-trp, T7-lac, T7-T7. In einer vorteilhaften Variante der vorliegenden Erfindung enthält der erfindungsgemäße Vektor zwei T7-Promotoren.

In einer besonders vorteilhaften Variante der vorliegenden Erfindung ist auch ein poly-cistroner Vektor umfasst, der eine NAD⁺-abhängige Dehydrogenase kodiert durch eine Nukleinsäuresequenz mit einer Identität von wenigstens 70% gemäß SEQ ID NO. 3 und eine NADPH-abhängige Dehydrogenase kodiert durch eine Nukleinsäuresequenz mit einer Identität von wenigstens 70% gemäß SEQ ID NO. 4, und eine Nukleinsäuresequenz kodierend für eine putative myo-Inositol-1-Phosphat-Phosphatase (Inositol-Monophosphatase) von wenigstens 70% Identität gemäß SEQ ID NO: 12. aufweist.

In einer weiteren besonders vorteilhaften Variante der vorliegenden Erfindung ist auch ein poly-cistroner Vektor umfasst, der eine NAD⁺-abhängige Dehydrogenase kodiert durch eine Nukleinsäuresequenz gemäß SEQ ID NO. 5, und eine NADPH-abhängige Dehydrogenase kodiert durch eine Nukleinsäuresequenz gemäß SEQ ID NO. 6, und eine Nukleinsäuresequenz kodierend für eine putative myo-Inositol-1-Phosphat-Phosphatase (Inositol-Monophosphatase) gemäß SEQ ID NO: 12 aufweist.

Vorteilhafte Varianten der vorliegenden Erfindung basieren dabei auf Derivaten ausgehend von den Plasmiden pMKEx2 bzw. pMKEx2-BiT7, die nachfolgend konkret beschrieben sind. Als besonders vorteilhafte Ausgestaltung der erfindungsgemäßen Vektoren ist das Plasmid pBiT7-InoDCI zu nennen. In Sinne der vorliegenden Erfindung wird als Bezeichnung für das bevorzugte erfindungsgemäße Plasmid z.B. pBiT7-InoDCI und pMKEx2-pBiT7-InoDCI synonym verwendet. D.h., pBiT7-InoDCI ist eine Abkürzung für pMKEx2-pBiT7-InoDCI. pMKEx2 bzw. pMKEx2-BiT7 sind dabei die Bezeichnungen der Ausgangsplasmide, in welche die Gene erfindungsgemäß kloniert wurden.

In einer Variante der vorliegenden Erfindung ist auch die Verwendung der zuvor beschriebenen erfindungsgemäßen Nukleinsäuresequenzen und die Verwendung der erfindungsgemäßen Vektoren enthaltend diese Nukleinsäuresequenzen der vorliegenden Erfindung zur Herstellung von lebenden Organismen, bevorzugt Mikroorganismen, besonders bevorzugt Mikroorganismen ausgewählt aus der Gruppe enthaltend coryneforme Bakterien, bevorzugt Corynebacteriaceae oder Brevibacteriaceae, Bacillaceae, Enterobacteriaceae, Zymomonadaceae, Methylobacteriaceae, Burkholderiaceae, Vibrionaceae, Clostridiaceae, Pseudomonadaceae und Acetobacteraceae für die Herstellung von D-chiro-Inositol umfasst. Umfasst in der vorliegenden Erfindung sind in diesem Sinne bevorzugt Mikroorganismen der Gattung coryneformer Bakterien wie Corynebacterium oder Brevibacterium, und der Gattung Escherichia. Besonders bevorzugt sind in diesem Sinne Corynebacterium glutamicum, ganz besonders bevorzugt Corynebacterium glutamicum MB001(DE3)

Die erfindungsgemäßen Vektoren der zuvor beschriebenen Art eignen sich dabei gleichermaßen in vorteilhafter Weise zur Expression in einem der zuvor beschriebenen genetisch modifizierten Mikroorganismen der vorliegenden Erfindung.

Alle genetisch modifizierten Mikroorganismen der vorliegenden Erfindung, enthaltend einen erfindungsgemäßen Vektor oder dessen beschriebene Varianten sind ebenfalls Gegenstand der vorliegenden Erfindung.

Eine vorteilhafte Variante der vorliegenden Erfindung umfasst einen erfindungsgemäß genetisch modifizierten Mikroorganismus, ausgehend von den Gattungen Corynebacterium, Bevibacterium oder Escherichia, enthaltend Varianten der erfindungsgemäßen Vektoren. Weitere vorteilhafte Varianten der vorliegenden Erfindung umfassen einen erfindungsgemäß genetisch modifizierten Mikroorganismus ausgehend von Corynebacterium glutamicum MB001(DE3) oder Escherichia coli BL21(DE3), welcher Varianten der erfindungsgemäßen Vektoren aufweist.

In einer weiteren vorteilhaften Variante der vorliegenden Erfindung handelt es sich um einen genetisch veränderten Mikroorganismus der Gattung Corynebacterium glutamicum, enthaltend Vektoren der vorliegenden Erfindung, welche ihrerseits Nukleinsäuresequenzen kodierend für eine NAD⁺-abhängige Dehydrogenase-Aktivität zur Umsetzung von *myo-*Inositol zu 1-Keto-D-*chiro*-Inositol, und für eine NADPH-abhängige Dehydrogenase-Aktivität zur Umsetzung von 1-Keto-D-*chiro*-Inositol zur D-chiro-Inositol, und für einen myo-Inositol/Proton-Symporter IolT1 und/oder myo-Inositol/Proton-Symporter IolT2, und für eine myo-Inositol-1-Phosphat-Synthase, sowie optional für eine T7-RNA-Polymerase, sofern letztere nicht bereits chromosomal-kodiert vorhanden ist. Besonders vorteilhaft handelt es sich um Cornyebacterium glutamicum MB001 (DE3), der nachfolgend weiter beschrieben ist und beispielsweise die T7-RNA-polymerase unter Kontrolle eines starken Promotors trägt.

Gleichermaßen vorteilhaft ist eine Variante der vorliegenden Erfindung umfassend einen genetisch veränderten Mikroorganismus ausgehend von Escherichia coli, enthaltend Vektoren der vorliegenden Erfindung, welche ihrerseits die zuvor genannten Nukleinsäuresequenzen kodierend für eine NAD⁺-abhängige Dehydrogenase-Aktivität zur Umsetzung von *myo*-Inositol zu 1-Keto-D-*chiro*-Inositol, und eine NADPH-abhängige Dehydrogenase-Aktivität zur Umsetzung von 1-Keto-D-*chiro-*Inositol zur D-*chiro*-Inositol, und für einen myo-Inositol-Transporter ausgewählt aus der Gruppe enthaltend IolT1, IolT2, IolT, Iolf und YfiG, und für eine myo-Inositol-1-Phosphat-Synthase, und für eine putative myo-Inositol-1-Phosphat-Phosphatase (Inositol-Monophosphatase), und für eine T7-RNA-Polymerase enthalten, sofern letztere nicht bereits chromosomal-kodiert vorhanden ist. Besonders vorteilhaft handelt es sich um Escherichia coli BL(DE3).

Gegenstand der vorliegenden Erfindung ist auch die Verwendung eines genetisch modifizierten Mikroorganismus der erfindungsgemäßen Art und/oder von Vektoren gemäß der vorliegenden Erfindung für die biotechnologische Herstellung von D*-chiro-*Inositol.

Von der vorliegenden Erfindung umfasst ist auch die Verwendung eines Proteins mit einer NAD⁺-abhängige Dehydrogenase-Aktivität zur Umsetzung von *myo*-Inositol zu 1-Keto-D-*chiro*-Inositol gemeinsam mit einem weiteren Protein mit einer NADPH-abhängige Dehydrogenase-Aktivität zur Umsetzung von 1-Keto-D-*chiro*-Inositol zur D-*chiro*-Inositol für die in-vitro-Herstellung von D-chiro-Inositol.

Erfindungsgemäß umfasst ist auch die Verwendung von Proteinen, die eine Aminosäuresequenz mit einer Identität von wenigstens 80% zur Sequenz gemäß SEQ ID NO. 1 und eine Aminosäuresequenz mit einer Identität von wenigstens 80% zur Aminosäuresequenz gemäß SEQ ID NO. 2 aufweisen für die in-vitro-Herstellung von D-chiro-Inositol. Erfindungsgemäß umfasst ist auch die Verwendung von Proteinen, die eine Aminosäuresequenz gemäß SEQ ID NO. 1 und eine Aminosäuresequenz gemäß SEQ ID NO. 2 aufweisen für die in-vitro-Herstellung von D-chiro-Inositol.

Im Sinne der vorliegenden Erfindung ist unter "in-vitro-Herstellung" ein organischer Vorgang zu verstehen, der außerhalb eines lebenden Organismus abläuft. Die Bezeichnung "in-vitro" wird im Sinne der Erfindung mit "enzymatisch" und "enzymatischer Herstellung" synonym verwendet. Erfindungsgemäße ist für den in-vitro-Herstellungsprozess die Bereitstellung angereicherter und optional aufgereinigter Enzyme in isolierter Form zu verstehen.

Im Sinne der vorliegenden Erfindung ist unter "biotechnologischer Herstellung" ein Vorgang zu verstehen, der in einem lebenden Organismus abläuft. Die Bezeichnung "biotechnologische Herstellung" wird im Sinne der Erfindung mit "durch Expression von kodierenden Nukleinsäuresequenzen in einem lebenden Organismus/Mikroorganismus" oder "mikrobiell" synonym verwendet.

Die Herstellung von D-chiro-Inositol in lebenden Organismen, und hier die mikrobielle Herstellung, ist erfindungsgemäß bevorzugt, aber nicht limitierend für die vorliegende Erfindung.

Erfindungsgemäß bevorzugt ist die Herstellung von D-chiro-Inositol in lebenden Organismen, besonders bevorzugt eine mikrobielle Herstellung in genetisch modifizierten Mikroorganismen ausgewählt aus der Gruppe enthaltend coryneforme Bakterien, bevorzugt Corynebacteriaceae oder Brevibacteriaceae, sowie Bacillaceae, Enterobacteriaceae, Zymomonadaceae, Methylobacteriaceae, Burkholderiaceae, Vibrionaceae, Clostridiaceae, Pseudomonadaceae und Acetobacteraceae. Erfindungsgemäß weiter bevorzugt ist eine mikrobielle Herstellung von D-chiro-Inositol in genetisch modifizierten Mikroorganismen der Gattung Corynebacterium, Brevibacterium und Enterobacterium. Erfindungsgemäß besonders vorteilhaft ist eine mikrobielle Herstellung von D-chiro-Inositol in einem genetisch modifizierten Corynebacterium. Ganz besonders vorteilhaft ist eine mikrobielle Herstellung von D-chiro-Inositol in Corynebacterium glutamicum, insbesondere Corynebacterium glutamicum MB001 (DE3).

Außerdem von der vorliegenden Erfindung umfasst ist die Verwendung von Nukleinsäuresequenzen für die biotechnologische oder in-vitro-Herstellung von D*-chiro-*Inositol, ausgewählt aus der Gruppe enthaltend
a) Nukleinsäuresequenzen mit mindestens 70 % Sequenzidentität zu den Gensequenzen gemäß SEQ. ID. NO. 3 bzw. SEQ. ID.NO.4, oder
b) Nukleinsäuresequenzen, die unter stringenten Bedingungen mit komplementären Sequenzen zu den Nukleinsäuresequenzen gemäß SEQ ID NO. 3 bzw. SEQ ID NO. 4 oder mit Fragmenten davon hybridisiert, oder
c) Nukleinsäuresequenzen gemäß SEQ ID NO. 3 bzw. SEQ ID. NO. 4, die sich jedoch von diesen durch die Degeneriertheit des genetischen Codes oder durch funktionsneutrale Mutationen unterscheiden, oder
d) Nukleinsäuresequenzen gemäß SEQ ID NO.5 bzw. gemäß SEQ ID NO. 6.

Dabei ist im Sinne der Erfindung gemeint, dass die zuvor beschriebenen Nukleinsäuresequenzen in einem Organismus exprimiert werden, was zum einen bedeutet, dass sie in einem der erfindungsgemäßen Vektoren enthalten sind und nach Übertragung in einen erfindungsgemäßen genetisch modifizierten Mikroorganismus exprimiert werden, wobei die Expression extrachromosomal-kodiert erfolgt. Zum anderen kann es im Sinne der Erfindung bedeuten, dass die Expression der zuvor beschriebenen Nukleinsäuren übergangsweise auf einen Vektor in einen Organismus übertragen und dann durch doppelte Rekombinationsreaktionen in das Chromosom des Organismus integriert werden, so dass die Expression der zuvor beschriebenen Nukleinsäuresequenzen chromosomal-kodiert erfolgt. Die allgemeinen molekularbiologischen Methoden zur Erzeugung der entsprechenden kodierenden Nukleinsäuresequenzen, Vektoren oder Mikroorganismen sind dem Fachmann bekannt.

In einer weiteren Variante der vorliegenden Erfindung ist umfasst, die Verwendung von Nukleinsäuresequenzen der zuvor beschriebenen Art zur Herstellung von erfindungsgemäßen Vektoren und/oder zur Herstellung von erfindungsgemäß genetisch modifizierten Mikroorganismen zum Einsatz bei der biotechnologischen Herstellung von D-*chiro*-Inositol.

Gegenstand der vorliegenden Erfindung ist auch die Verwendung von kodierenden Nukleinsäuresequenzen der zuvor beschriebenen Art zur Herstellung von Proteinen mit einer NAD⁺-abhängige Dehydrogenase-Aktivität zur Umsetzung von *myo-*Inositol zu 1-Keto-D-*chiro*-Inositol, und mit einer NADPH-abhängige Dehydrogenase-Aktivität zur Umsetzung von 1-Keto-D-*chiro*-Inositol zur D-*chiro*-Inositol, für den nachfolgenden Einsatz der hergestellten, optional angereicherten und weiter optional aufgereinigten Enzyme in einem in-vitro-Verfahren zur Herstellung von D-*chiro*-Inositol. Dabei kann im Sinne der Erfindung die Herstellung der Proteine durch die Verwendung der beschriebenen kodierenden Nukleinsäuresequenzen auch in anderen Mikroorganismen und Vektoren erfolgen als sie für die vorliegende Erfindung beschrieben bzw. eingesetzt werden. Dem Fachmann sind eine Vielzahl an Expressionsvektoren und geeigneten Mikroorganismen bekannt. Die enzymatisch aktiven Proteine können dann in einem in-vitro-Verfahren zur Herstellung von D-chiro-Inositol eingesetzt werden.

Gegenstand der vorliegenden Erfindung ist außerdem ein Verfahren zur biotechnologischen Herstellung von D-*chiro*-Inositol, umfassend
a) die Bereitstellung eines erfindungsgemäß genetisch modifizierten Mikroorganismus der zuvor beschriebenen Art,
b) die Kultivierung des genetisch modifizierten Mikroorganismus aus a) in einem Medium, welches ein geeignetes Ausgangssubstrat für die Bildung von D-*chiro*-Inositol enthält, ausgewählt aus *myo-*Inositol, Glukose oder Glukosebausteine enthaltenden Zuckern,
c) die Isolierung, und optional die Aufreinigung, des gebildeten D-*chiro*-Inositols.

Erfindungsgemäß umfasst ist auch ein Verfahren der zuvor genannten Art, umfassend eine enzymatische Umsetzung ausgehend von Glukosebausteinen enthaltenden Zuckern, wie Saccharose, Glukose und Maltose, zu *myo*-Inositol, die während Schritt b) stattfindet.

Unter "Medium" ist erfindungsgemäß gleichbedeutend zu verstehen "Lösung", "Kulturmedium", "Kulturbrühe" oder "Kulturlösung". Unter "Kultivierung" ist erfindungsgemäß gleichbedeutend zu verstehen "Umsetzung", "Verstoffwechslung" oder "Metabolisierung". Beispielsweise kann bei der Kultivierung eine Umsetzung einer C-Quelle in ein Produkt erfolgen. Dabei kann das Produkt Biomasse oder ein Metabolit oder Sekundärmetabolit oder ein beliebiges anderes Produkt sein. Das Produkt kann in einer Variante auch durch Expression von homologen und/oder heterologen Genen erfolgen.

Im Sinne der vorliegenden Erfindung ist unter "Ausgangssubstrat" zu verstehen, myo-Inositol, Glukosebausteine enthaltende Zucker, wie Saccharose, Glukose und Maltose. Weitere denkbare Substrate neben *myo-*Inositol und Glukose sind somit Zucker, die Glukosemoleküle enthalten und die die erfindungsgemäß genetische modifizierten Mikroorganismen ohne weitere genetische Modifikationen zu Glukose-6-phosphat umsetzen können. Dies sind zum Bsp. Saccharose und Maltose.

Vorteilhafter Weise können coryneforme Bakterien mit Hilfe ihres Gens für die myo-Inositol-1-phosphat-Synthase (ino1) Glukose-6-Phosphat zu myo-Inositol-1-phosphat umwandelt. Im Genom coryneformer Bakterien enthaltene putative Inositol-Monophosphatasen können durch Dephosphorylierung myo-Inositol-1-phosphat zu myo-Inositol verstoffwechseln und auf diese Weise D-chiro-Inositol aus Glukose produzieren.

Im Sinne der vorliegenden Erfindung ist "biotechnologisch" gleichbedeutend zu verstehen mit "mikrobiell", oder "durch Expression von kodierenden Nukleinsäuresequenzen in einem lebenden Organismus/Mikroorganismus" oder "fermentativ". Unter "Aufbereitung" ist erfindungsgemäß gleichbedeutend zu verstehen "Abtrennung", "Aufkonzentrierung" oder "Aufreinigung".

Das zu verwendende Kulturmedium sollte in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Neben myo-Inositol als Ausgangssubstrat der D-chiro-Inositol-Bildung können als Kohlenstoffquelle Zucker und Kohlehydrate wie z.B. Glucose, Saccharose, Fructose, Maltose, Melasse, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische, Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium-haltigen Salze verwendet werden. Das Kulturmedium sollte weiterhin Salze von Metallen enthalten wie z.B. Magnesiumsulfat oder Eisensulfat, die für das Wachstum notwendig sind. Schließlich können essentielle Wuchsstoffe, wie Aminosäuren und Vitamine zusätzlich zu den oben genannten Stoffen eingesetzt werden. Die genannten Einsatzstoffe können zur Kultur in Form eines einmaligen Ansatzes hinzugegeben oder in geeigneter Weise während der Kultivierung zugefüttert werden. Zur pH-Kontrolle der Kultur werden basische Verbindungen wie Natriumhydroxid, Kaliumhydroxid, Ammoniak oder saure Verbindungen wie Salzsäure, Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe, z.B. Antibiotika, hinzugefügt werden. Um aerobe Bedingungen aufrechtzuerhalten werden Sauerstoff oder Sauerstoff-haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum an D-chiro-Inositol gebildet hat. Dieses Ziel wird normalerweise innerhalb von 10 Stunden bis 72 Stunden erreicht.

Die Kultivierung der erfindungsgemäßen Mikroorganismen kann mittels bekannter Kultivierungsmethoden oder darauf basierenden, modifizierten Methoden erfolgen. Die Kultivierung kann bevorzugt in synthetischem Mineralsalz-Medium oder in Komplex-Medium erfolgen.

Solche Mineralsalz-Medien können eine oder mehrere der nachfolgenden Komponenten enthalten:
i) eine Kohlenstoff-Quelle, wie z.B.
   (1) Kohlenhydrate wie z. B. Glucose, Fructose, Saccharose, Maltose oder Melasse,
   (2) organische Säuren wie z. B. Essigsäure,
   (3) Alkohole wie z. B. Ethanol
ii) eine Stickstoff-Quelle, wie z.B.
   Ammoniak und Ammoniumsalze, Harnstoff, Pepton, Fleisch- oder Hefeextrakte
iii) eine Schwefel-Quelle, wie z.B.Magnesiumsulfat
iv) eine Phosphat⁻Quelle, wie z.B.
   (1) Kaliumdihydrogenphosphat oder Dikaliumhydrogenphosphat
v) anorganische Salze, wie z.B.
   (1) Kaliumdihydrogenphosphat, Dikaliumhydrogenphosphat, Magnesiumphosphat,
   (2) Natriumchlorid, Calciumchlorid
   (3) Mangansulfat, Magnesiumsulfat, Kupfersulfat, oder
   (4) Calciumcarbonat
vi) ggf. Puffer, Säuren oder Basen, wie z.B.
   (1) Puffer: Phosphat-, TRIS-, HEPES-, MES- oder MOPS-Puffer
   (2) Säuren: Salzsäure
   (3) Basen: Natriumhydroxid
vii) Kofaktoren, wie z. B. Metallionen-Quellen, wie z.B.:
   (1) Eisensulfat
   (2) Mangansulfat
   (3) Zinksulfat
   (4) Kupfersulfat
   (5) Nickelsulfat
viii) optional ein oder mehrere Antibiotika
ix) optional Biotin.

Komplex-Medien können eine oder mehrere der nachfolgenden Komponenten enthalten:
i) eine Kohlenstoff-Quelle, wie z. B.
   (1) Kohlenhydrate wie z. B. Glucose, Fructose, Saccharose,
   (2) organische Säuren wie Essigsäure,
   (3) Alkohole wie z. B. Ethanol
ii) eine Stickstoff-Quelle, wie z.B.
   (1) Ammoniak und Ammoniumsalze, Harnstoff, Pepton, Fleisch- oder Hefeextrakte, Glutamin, Asparagin
   (2) eine Schwefel-Quelle, wie z. B. Magnesiumsulfat
iii) eine Phosphat⁻Quelle, wie z. B.
   (1) Kaliumdihydrogenphosphat, Dikaliumhydrogenphosphat
iv) anorganische Salze, wie z. B.
   (1) Kaliumdihydrogenphosphat, Dikaliumhydrogenphosphat, Magnesiumphosphat,
   (2) Natriumchlorid, Calciumchlorid
   (3) Mangansulfat, Magnesiumsulfat, Kupfersulfat,
   (4) Calciumcarbonat
v) Kofaktoren, wie z. B. Metallionen-Quellen, wie z. B.:
   (1) Eisensulfat
   (2) Mangansulfat
   (3) Zinksulfat
   (4) Kupfersulfat
   (5) Nickelsulfat
vi) ggf. Puffer, Säuren oder Basen, wie z.B.
   (1) Puffer: Phosphat-, TRIS-, HEPES-, MES- oder MOPS-Puffer
   (2) Säuren: Salzsäure
   (3) Basen: Natriumhydroxid
vii) ggf. ein oder mehrere Antibiotika.

Die vorliegende Erfindung betrifft Verfahren bei dem die Kultivierung diskontinuierlich oder kontinuierlich, bevorzugt im batch-, fed-batch- oder repeated-fed-batch-Modus erfolgt. Eine vorteilhafte Variante ist die Kultivierung im batch-System unter Zugabe von Glukose und myo-Inositol. Eine weitere Variante eines erfindungsgemäßen Verfahrens erfolgt im batch-Modus unter Einsatz von Glukose alleine. Eine weitere Variante eines erfindungsgemäßen Verfahrens erfolgt im fed-batch-Modus. Besonders bevorzugt ist dabei die anfängliche Zugabe von Glukose und myo-Inositol, die im Weiteren durch die "Zufütterung" von myo-Inositol oder von Glukose oder myo-Inositol und Glukose erfolgt.

Erfindungsgemäß bevorzugt ist ein Verfahren zur biotechnologischen Herstellung von D-chiro-Inositol durch die Bereitstellung genetisch modifizierter Mikroorganismen ausgewählt aus der Gruppe enthaltend coryneforme Bakterien, bevorzugt Corynebacteriaceae oder Brevibacteriaceae, Bacillaceae, Enterobacteriaceae, Zymomonadaceae, Methylobacteriaceae, Burkholderiaceae, Vibrionaceae, Clostridiaceae, Pseudomonadaceae und Acetobacteraceae. In einer weiteren Variante des erfindungsgemäßen Verfahrens werden genetisch modifizierte Mikroorganismen bereitgestellt, ausgewählt aus der Gruppe enthaltend Corynebacterium, Brevibacterium, Bacillus, Lactobacillus, Lactococcus, Escherichia, Zymomonas, Methylobacterium, Ralstonia, Vibrio, Clostridium, Pseudomonas und Gluconobacter. Eine weitere vorteilhafte Verfahrensvariante umfasst die Bereitstellung eines genetisch modifizierten Mikroorganismus gemäß der vorliegenden Erfindung, ausgewählt aus der Gruppe enthaltend Corynebacterium glutamicum, Corynebacterium acetoglutamicum, Brevibacterium flavum, Brevibacterium lactofermentum und Brevibacterium divaricatum. In einer weiteren vorteilhaften Verfahrensvariante wird erfindungsgemäß ein genetisch modifizierter Mikroorganismus bereitgestellt, ausgewählt aus der Gruppe enthaltend Corynebacterium glutamicum ATCC13032, Corynebacterium glutamicum MB001(DE3), Corynebacterium acetoglutamicum ATCC15806, Corynebacterium acetoacidophilum ATCC13870, Brevibacterium flavum ATCC14067, Brevibacterium lactofermentum ATCC13869 und Brevibacterium divaricatum ATCC14020.

Weitere Vorteile der Erfindung ergeben sich aus den Unteransprüchen und aus der nachfolgenden Beschreibung von Ausführungsbeispielen der Erfindung. Nachfolgend wird der Gegenstand der Erfindung anhand von Tabellen und Figuren näher erläutert, ohne dass der Gegenstand der Erfindung dadurch beschränkt wird.

### Tabellen, Figuren und Abkürzungen

### Abkürzungen

- DCI: D-*chiro*-Inositol
- MI: *myo*-Inositol
- 2KMI: 2-Keto-*myo*-Inositol
- 1KDCI: 1-Keto-D-*chiro*-Inositol
- Ino1: myo-Inositol-1-Phosphat-Synthase (Ino1)
- OEPa: NAD⁺-abhängige Dehydrogenase, welche erfindungsgemäß die Umsetzung von *myo-*Inositol zu 1-Keto-D-*chiro*-Inositol katalysiert (MI-Dehydrogenase). Die Nukleinsäuresequenz kodierend für diese NAD⁺-abhängige Dehydrogenase (OEPa) wurde basierend auf der Dehydrogenase_a aus Medicago truncatula (MtOEPa) an die Codon-Usage coryneformer Bakterien angepasst. In Sinne der vorliegenden Erfindung wird sowohl ausgehend von dieser Codon-optimierten Nukleinsäuresequenz als auch des pflanzlichen Gens die erfindungsgemäße Enzymaktivität gleichermaßen in den erfindungsgemäß genetisch modifizierten Mikroorganismen, exprimiert und ist somit nicht limitierend für die vorliegende Erfindung. Die Bezeichnung MtOEPa, OEPa, NAD⁺-abhängige Dehydrogenase und MI-Dehydrogenase sowohl für die erfindungsgemäßen Nukleinsäuresequenzen und der durch sie kodierten NAD⁺abhängige Dehydrogenase, welche die Umsetzung von myo-Inositol zu 1-Keto-D-*chiro*-Inositol katalysiert (MI-Dehydrogenase), synonym verwendet. Die Bezeichnungen werden insbesondere in der Beschreibung, den Beispielen, den Tabellen sowie in den Plasmidkarten der Vektoren synonym verwendet.
- OEPb: NADPH-abhängige Dehydrogenase, welche die erfindungsgemäße Umsetzung von 1-Keto-D-*chiro*-Inositol zur D-*chiro*-Inositol katalysiert (1KDCI-Dehydrogenase). Die Nukleinsäuresequenz kodierend für diese NADPH-abhängige Dehydrogenase (OEPb) wurde basierend auf der Dehydrogenase_b aus Medicago truncatula (MtOEPb) an die Codon-Usage coryneformer Bakterien angepasst. In Sinne der vorliegenden Erfindung wird sowohl ausgehend von dieser Codon-optimierten Nukleinsäuresequenz als auch des pflanzlichen Gens die erfindungsgemäße Enzymaktivität gleichermaßen in den erfindungsgemäß genetisch modifizierten Mikroorganismen exprimiert und ist somit nicht limitierend für die vorliegende Erfindung. Die Bezeichnung MtOEPb, OEPb, NADPH-abhängige Dehydrogenase und 1KDCI-Dehydrogenase sowohl für die erfindungsgemäßen Nukleinsäuresequenzen und der durch sie kodierten NADPH-abhängige Dehydrogenase, welche die Umsetzung von 1-Keto-D-*chiro*-Inositol zur D-*chiro*-Inositol katalysiert (1KDCI-Dehydrogenase), synonym verwendet. Die Bezeichnungen werden insbesondere in der Beschreibung, den Beispielen, den Tabellen sowie in den Plasmidkarten der Vektoren synonym verwendet.

**Tabelle 1** zeigt eine Übersicht an Bakterienstämmen der vorliegenden Erfindung.
**Tabelle 2** zeigt eine Übersicht an Plasmiden der vorliegenden Erfindung
**Tabelle 3** zeigt eine Übersicht der SEQ ID NO.`s der vorliegenden Erfindung.

**Fig. 1** zeigt das Plasmid pK19mobsacBΔiol1. Enthalten sind 1000bp homologen Flanken aufwärts und abwärts von cg0196-0212, mit Hilfe derer der kodierende Bereich von cg0196-0212durch doppelt homologe Rekombination im Genom des coryneformen Bakteriums deletiert wurde.

**Fig 2****:** zeigt das Plasmid pK19mobsacBΔiol2. Enthalten sind 1000bp homologen Flanken aufwärts und abwärts von cg3389-3392, mit Hilfe derer der kodierende Bereich von cg3389-3392 durch doppelt homologe Rekombination im Genom des coryneformen Bakteriums deletiert wurde.

**Fig: 3** zeigt das Plasmid pK19mobSacBΔcg2312-13. Enthalten sind 1000bp Regionen der 5'-Region und 3' Region des Genlocus cg2312-cg2313, mit Hilfe derer der kodierende Bereich von cg2312-cg2313 durch doppelt homologe Rekombination im Genom des coryneformen Bakteriums deletiert wurde.

**Fig. 4** zeigt das Expressions-Plasmid pOEPa-b auf der Basis des Plasmids pMKEx2. Enthalten sind die NAD⁺- und die NADPH-abhängige Dehydrogenase (OEPa bzw. OEPb) unter der Kontrolle des induzierbaren T7-Promoters (durch lac Operator reprimiert) mit Ribosomenbindungsstelle und des dazugehörigen T7-Terminators. Dieses Plasmid ermöglicht die Produktion von D-chiro-Inositol ausgehend von *myo-*Inositol in *Corynebacterium glutamicum ΔIOL.*

In Sinne der vorliegenden Erfindung werden als Bezeichnungen für die bevorzugten erfindungsgemäßen Plasmide z.B. pOEPa-b und pMKEx2-OEPa-b synonym verwendet. D.h., pOEPa-b ist eine Abkürzung für pMKEx2-OEPa-b. pMKEx2 ist dabei die Bezeichnung des Ausgangsplasmids, in welches die Gene erfindungsgemäß kloniert wurden.

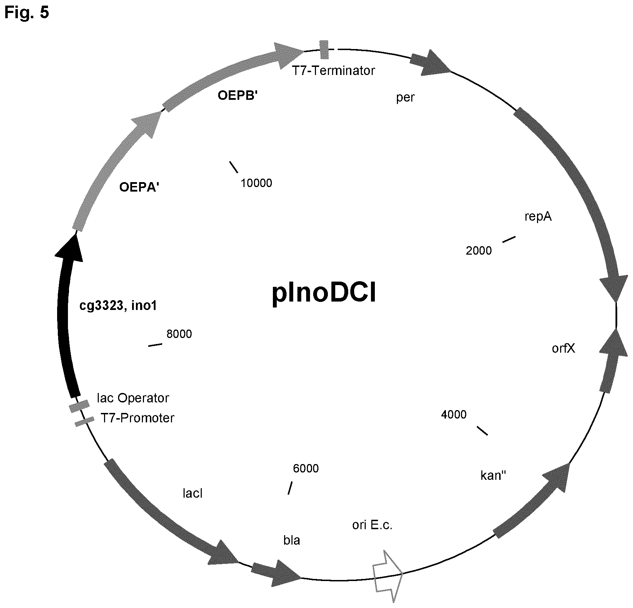

**Fig. 5** zeigt das Expressions-Plasmid pInoDCI auf der Basis des Plasmids pMKEx2. Enthalten sind sowohl die kodierende Nukleinsäuresequenz für *myo*-Inositol-1-phosphat-Synthase (ino1) unter der Kontrolle des induzierbaren T7-Promoters (durch lac Operator reprimiert) mit Ribosomenbindungsstelle und dazugehörigem T7-Terminator sowie der NAD⁺- und die NADPH-abhängige Dehydrogenase (OEPa bzw. OEPb) unter der Kontrolle des induzierbaren T7-Promoters (durch lac Operator reprimiert) mit Ribosomenbindungsstelle und des dazugehörigen T7-Terminators. Dieses Plasmid ermöglicht die Produktion von D-chiro-Inositol ausgehend von Glukose in *Corynebacterium glutamicum ΔIOL.*

In Sinne der vorliegenden Erfindung werden als Bezeichnungen für die bevorzugten erfindungsgemäßen Plasmide z.B. pInoDCI und pMKEx2-InoDCI synonym verwendet. D.h., plnoDCI ist eine Abkürzung für pMKEx2-InoDCI. pMKEx2 ist dabei die Bezeichnung des Ausgangsplasmids, in welches die Gene erfindungsgemäß kloniert wurden.

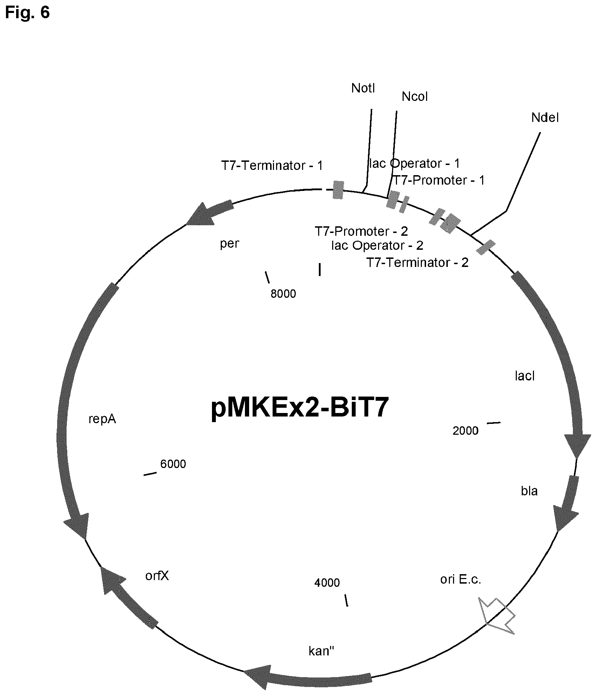

**Fig. 6** zeigt das Expressions-Plasmid pMKEx2-BiT7 auf der Basis des Plasmids pMKEx2. Dieses Plasmid enthält zwei Expressionskassetten (Ribosomenbindungsstelle, T7-Promoter, lacO-Operator, multiple-cloning-site (MCS), T7-Terminator), wodurch die poly-cistrone Expression synthetischer Operone möglich ist. Die Erkennungssequenzen der Restriktionsenzyme Notl, Ncol und Ndel in einer multiplencloning-site (MCS) ermöglichen das Klonieren gewünschter kodierender Nukleinsäuresequenzen (Gene, Cistrone).

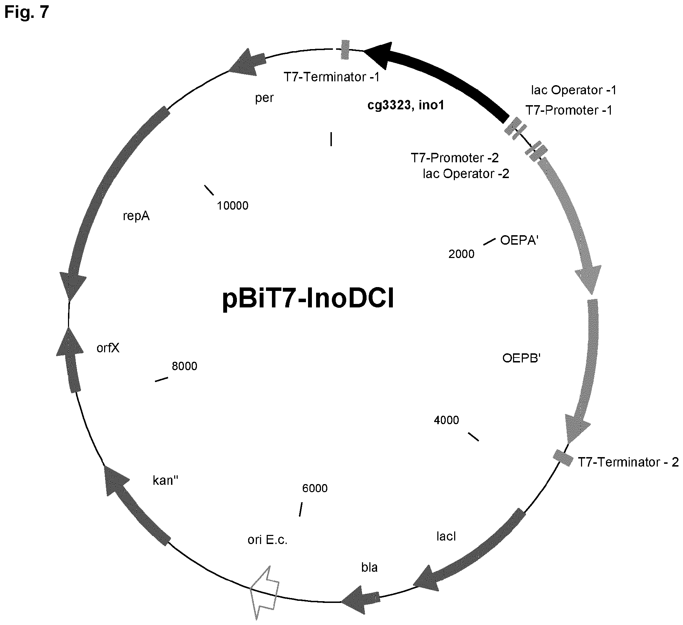

**Fig. 7** zeigt das Expressions-Plasmid pBiT7-InoDCI auf der Basis des Plasmids pMKEx2 und ist ein Derivat von pMKEx2-BiT7. Enthalten sind sowohl das Gen, welches die *myo*-Inositol-1-phosphat-Synthase (ino1) kodiert unter der Kontrolle eines ersten T7-Promotors (-1-), als auch die beiden erfindungsgemäßen Dehydrogenase-Gene OEPa und OEPb unter der Kontrolle eines zweiten T7-Promoters (-2-). Dies ermöglicht die bicistronische Expression beider Expressionskassetten. Dieses Plasmid ermöglicht die Produktion von D-*chiro*-Inositol ausgehend von Glukose in *Corynebacterium glutamicum ΔIOL.* In Sinne der vorliegenden Erfindung wird als Bezeichnung für das bevorzugte erfindungsgemäße Plasmid z.B. pBiT7-InoDCI und pMKEx2-pBiT7-InoDCI synonym verwendet. D.h., pBiT7-InoDCI ist eine Abkürzung für pMKEx2-pBiT7-InoDCI. pMKEx2 bzw. pMKEx2-BiT7 sind dabei die Bezeichnungen der Ausgangsplasmide, in welche die Gene erfindungsgemäß kloniert wurden.

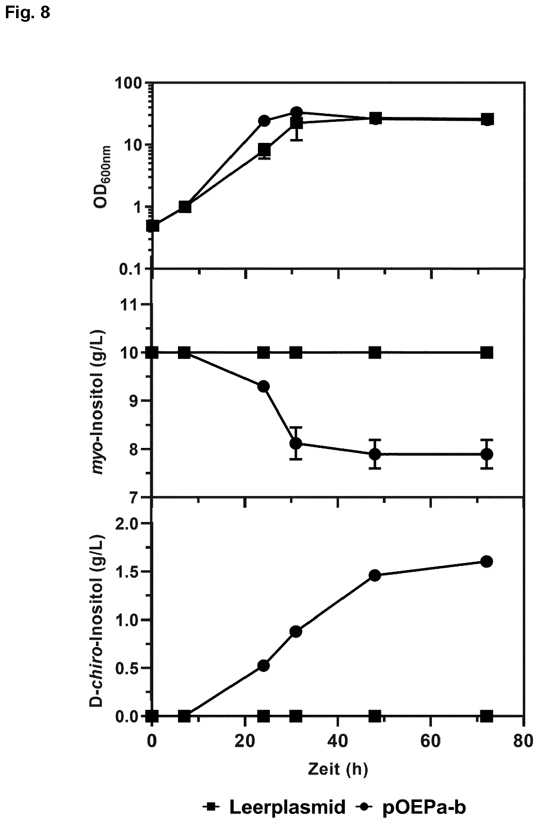

**Fig. 8** zeigt die Herstellung von D-chiro-Inositol (DCI) aus myo-Inositol (MI) in C. *glutamicum* MB001(DE3)Δ*IOL* durch Expression der Codon-optimierten NAD⁺-abhängigen bzw. NADPH-abhängigen Dehydrogenasen kloniert auf dem Plasmid pEOPa-b. Die angegebenen Stämme wurden in Minimalsalz-Medium kultiviert, das 20 g/L Glukose und 10 g/L myo-Inositol enthält. Die Genexpression wurde durch Zugabe von 500 µM IPTG zu Beginn der Kultivierung induziert. Angegeben sind Mittelwerte von biologischen Triplikaten und Standardabweichungen. Als Kontrolle wurde als sogenanntes Leerplasmid das Plasmid pMKEx2 eingesetzt. Oben dargestellt ist das Zellwachstum über der Zeit gemessen bei einer optischen Dichte OD₆₀₀. In der Mitte ist der Abbau des Ausgangssubstrats myo-Inositol (MI) über der Zeit dargestellt. Unten ist die Bildung des gewünschten Produktes D-chiro-Inositol (DCI) über der Zeit dargestellt. Innerhalb von 48 Stunden konnte bis zu 1,6 g/L DCI pro 20 g/L Glukose akkumuliert werden.

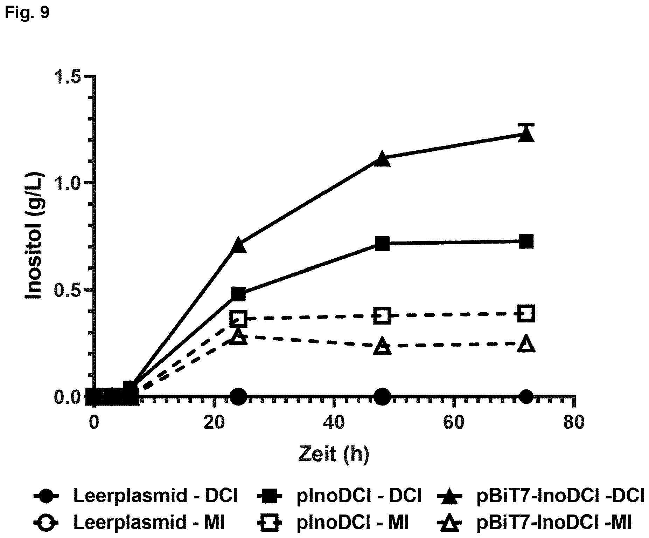

**Fig. 9** zeigt die Produktion von D-chiro-Inositol (DCI) und myo-Inositol (MI) aus Glucose in C. *glutamicum* MB001(DE3)ΔIOL durch Expression der Codon-optimierten NAD⁺-abhängigen bzw. NADPH-abhängigen Dehydrogenasen plus der myo-Inositol-1-Phosphat-Synthase (Ino1) kloniert auf den Plasmiden pInoDCI und pBiT7-InoDCI. Die Stämme wurden in Minimalsalz-Medium mit 20 g/L Glukose 72 Stunden lang bei 30°C kultiviert. Die Genexpression wurde durch Zugabe von 500 µM IPTG nach 3 Stunden nach Beginn der Kultivierung induziert. Angegeben sind Mittelwerte von biologischen Triplikaten und Standardabweichungen. Als Kontrolle für die myo-InositolBildung wurde *C*. *glutamicum* MB001(DE3)ΔIOL enthaltend das Leerplasmid auf der Basis von pMKEx2 (Leerplasmid-MI) eingesetzt. Als Kontrolle für die D-chiro-Inositol-Bildung wurde *C*. *glutamicum* MB001(DE3)ΔIOL enthaltend das Leerplasmid auf der Basis von pMKEx2-BiT7 (Leerplasmid-DCI) eingesetzt. Leerplasmid-DCI bzw. Leerplasmid-MI zeigen den gleichen Kurvenverlauf und sind überlagert dargestellt, so dass in der Darstellung die Symbole für das Leerplasmid-MI (offener Kreis) nicht sichtbar ist.

Dargestellt ist die Gesamt-Inositol-Bildung über der Zeit mit einer myo-Inositol-Bildung (pInoDCI-MI) und D-chiro-Inositol-Bildung (pInoDCI-DCI) in *C*. *glutamicum* MB001 (DE3)ΔIOL_pInoDCI. Dabei werden ca. 0,3 g/L myo-Inositol und ca. 0,7 g/L D-chiro-Inositol gebildet.

Dargestellt ist ferner die Gesamt-Inositol-Bildung über der Zeit mit einer myo-Inositol-Bildung (pBiT7-InoDCI-MI) und D-chiro-Inositol-Bildung (pBiT7-InoDCI-DCI) in C. *glutamicum* MB001(DE3)ΔIOL_pBiT7-InoDCI. Dabei werden ca. 0,2 g/L myo-Inositol und ca. 1,2 g/L D-chiro-Inositol pro 20 g/L Glukose gebildet.

Die Expression der Codon-optimierten NAD⁺-abhängigen bzw. NADPH-abhängigen Dehydrogenasen plus der myo-Inositol-1-Phosphat-Synthase (Ino1) kloniert auf den bi-cistronen Plasmiden pBiT7-InoDCI führt zu einer 70% höheren DCI-Produktion gegenüber der Expression ausgehend von dem Plasmiden pInoDCI. Dies zeigt eindeutig die Vorteilhaftigkeit des erfindungsgemäß poly-cistronen Vektors pMKEx2-BiT7.

Die vorliegende Erfindung wird durch die folgenden Beispiele näher erläutert, die jedoch nicht limitierend sind:

### Herstellung von Corynebacterium glutamicum MB001(DE3)Δiol1 und MB001 (DE3)Δiol1Δiol2

Ausgehend von Corynebacterium glutamicum MB001(DE3) (Kortmann, Maike, et al. "A chromosomally encoded T 7 RNA polymerase-dependent gene expression system for Corynebacterium glutamicum: construction and comparative evaluation at the single-cell level", Microbial biotechnology 8.2 (2015): 253-265.) wurde die Deletion des großen Inositol-Gen-Clusters iol1 (cg0196 - cg0212) und des kleinen Inositol-Gen-Clusters iol2 (cg3389-cg3392), welche die Gene für den Inositolabbauweg enthalten vorgenommen. Beide Stämme wurden nach Niebisch und Bott (2001) (https://doi.org/10.1007/s002030100262) mit dem Vektor pK19mobsacB über doppelt homologe Rekombination (Schäfer et al., 1994) (https://doi.org/10.1016/0378-1119(94)90324-7) konstruiert.

Hierfür wurden zuerst die entsprechenden Deletionsplasmide pK19mobsacBΔiol1 und pK19mobsacBΔiol2 konstruiert:

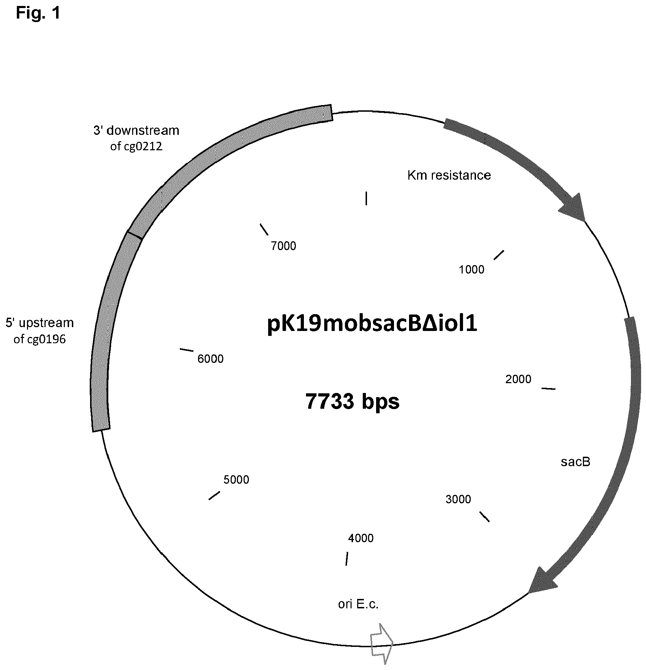

### Konstruktion von pK19mobsacBΔiol1 (Fig. 1):

Zunächst wurden zwei 1000bp großen Homologieflanken, die die 5'-Region des Gens cg0196 und die 3`-Reion des Gens cg0212 enthalten mittels PCR (Q5^{®} High-Fidelity 2X Master Mix (New England BioLabs)) und der nachfolgend beschriebenen Primer mittels PCR amplifziert:
Flanke 1 wurde mit den Primern P001_Δiol1_FW1 × P002_Δiol1_RV1 (Ann. Temp. 53°C, Zyklen: 30, Elong. Zeit. 0:30 min) und Flanke 2 wurde mit den Primern P003_Δiol1_FW2 × P004_Δiol1_RV2 (Ann. Temp. 52°C, Zyklen 30, Elong. Zeit. 0:30 min) von genomischer DNA von C. glutamicum amplifiziert.

Beide Fragmente wurden im Verhältnis 1:1 mittels Gibson Assembly mit pK19mobSacB Plasmid-DNA, welche vorher mit dem Resitrktionsenzym EcoRI restringiert wurde zum finalen Plasmid pK19mobsacBΔiol1 assembliert (Gibson et al., 2009) (https://doi.org/10.1038/NMETH.1318).

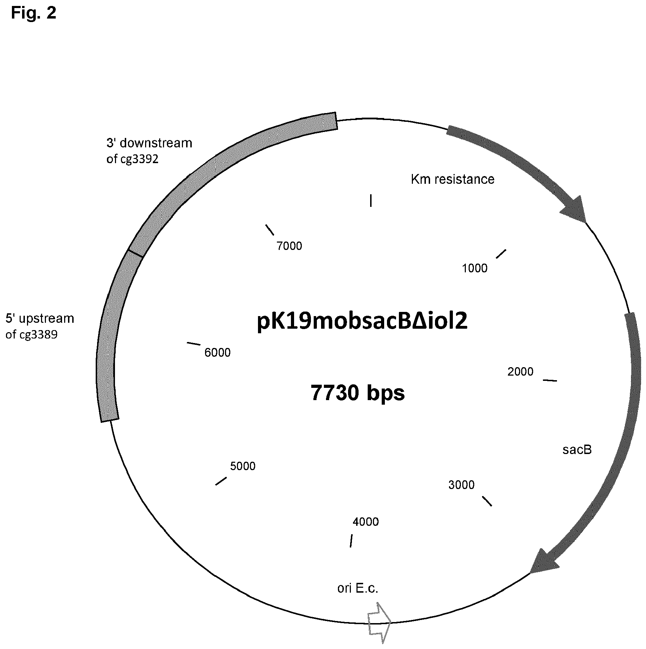

### Konstruktion von pK19mobsacBΔiol2 (Fig. 2):

Zunächst wurden zwei 1000bp großen Homologieflanken, die die 5'-Region des Gens cg3389 und die 3`-Reion des Gens cg3392 enthalten mittels PCR (Q5^{®} High-Fidelity 2X Master Mix (New England BioLabs)) und der nachfolgenden Primer mittels PCR amplifziert:
Flanke 1 wurde mit den Primern P007_ΔoxiC-E_FW1 × P008_ΔoxiC-E_RV1 (Ann. Temp. 51°C, Zyklen: 30, Elong. Zeit. 0:30 min) und Flanke 2 wurde mit den Primern P009_ΔoxiC-E_FW2 × P010_ΔoxiC-E_RV2 (Ann. Temp. 54°C, Zyklen 30, Elong. Zeit. 0:30 min) von genomischer DNA von C. glutamicum amplifiziert.

Beide Fragmente wurden im Verhältnis 1:1 mittels Gibson Assembly mit pK19mobSacB Plasmid-DNA, welche vorher mit dem Resitrktionsenzym EcoRI restringiert wurde zum finalen Plasmid pK19mobSacBΔiol1 assembliert (Gibson et al., 2009) (https://doi.org)/10.1038/NMETH.1318).

### Transformation beider Plasmide in E.coli DH5α

Die so erhaltenen Plasmide pK19mobsacBΔiol1 und pK19mobsacBΔiol2 wurden mittels Hitzeschock bei 42°C für 90 Sekunden in E. coli DH5α transformiert. Wegen des auf den Plasmiden vorliegenden Kanamycinresistenzgens konnten nur solche Klone wachsen, die ein Plasmid aufgenommen hatten. Diese wurden mittels Kolonie-PCR (DreamTaq Green PCR Master Mix (Thermofisher Scientific) Annealing Temp: 53°C, Zyklen: 30, Elongation: 2:00 min, Primer: P011_pK19_1 × P012_pK19_2) und anschließender Gelelektrophorese auf ihre Korrektheit überprüft, und deren DNA-Sequenz wurde bestimmt, durch Sanger-Sequenzierung über Kapillarelektrophorese durchgeführt über den DNA-Sequenzierung-Service von Eurofins Genomics.

### Primer für die Konstruktion von pK19mobsacBΔiol1 und pK19mobsacBΔiol2

| | |
|---|---|
| P001_Δiol1_FW1 | GAGGATCCCCGGGTACCGAGCTCGTTCCGCCAACTCAACC |
| P002_Δiol1_RV1 | TGAAACCACTCTGGTGGCCAGGTAAG |
| P003_Δiol1_FW2 | CTTACCTGGCCACCAGAGTGGTTTCAGAAGAGTCCCTGGTTTC |
| P004_Δiol1_RV2 | CGTTGTAAAACGACGGCCAGTGAATTTCCTTGGTCACCAGATC |
| P007_ΔoxiC-E_FW1 | GAGGATCCCCGGGTACCGAGCTCGGGAACCCAATTCACCTTCG |
| P008_ΔoxiC-E_RV1 | GCCTAGCAGGCCAACAAC |
| P009_ΔoxiC-E_FW2 | AAATTGTTGTTGGCCTGCTAGGCTGCGCTGGTTCCATCTGC |
| P010_ΔoxiC-E_RV2 | CGTTGTAAAACGACGGCCAGTGAATTGGACCAACCAGAAACTTC |
| P011_pK19_1 | AGCGGATAACAATTTCACACAGGA |
| P012_pK19_2 | CGCCAGGGTTTTCCCAGTCAC |

### Transformation von C. glutamicum MB001(DE3) mit pK19mobsacBΔiol1 zur Herstellung von C. glutamicum MB001 (DE3)Δiol1:

Elektrokompetente C. glutamicum MB001 (DE3)-Zellen wurden wie folgt hergestellt: 50 mL BHIS ((Brain-Heart-Infusion (Sigma Aldrich) supplementiert mit 91 g/L Sorbitol)) in einem 500 ml Schüttelkolben wurden mit 1 mL Vorkultur, die über Nacht in 10 ml BHIS gewachsen war, bei 30°C, 170 Upm, beimpft. Die Kultur wurde bei 120 rpm und 30 °C geschüttelt, bis eine OD600 von 0,6 erreicht war. Es wurden 15 µL Ampicillin (5 mg/mL) und 2 mL Isoniazid (100 mg/mL) zugegeben, und die Kultur wurde eine weitere Stunde bei 30 °C und 120 U/min geschüttelt. Die Zellen wurden durch 10-minütiges Zentrifugieren bei 4 °C und 4000 U/min geerntet. Das Zellpellet wurde dreimal durch Resuspendierung in 30 mL EPB1-Puffer [20 mM Hepes pH 7,2, 5 % (v/v) Glycerin] gewaschen und anschließend 10 Minuten bei 4 °C und 4000 U/min zentrifugiert. Das Zellpellet wurde dann in 1 mL EPB2-Puffer [5 mM Hepes pH 7,2, 15% (v/v) Glycerin] resuspendiert.

Nach Isolierung des Plasmids pK19mobsacBΔiol1 wurde ein 100 µL Aliquot elektrokompetenter C. glutamicum Zellen auf Eis aufgetaut, dieses mit 1 µg des Plasmids vermischt und in eine auf Eis vorgekühlte 0,2 cm Elektroporationsküvette überführt. Die Mischung wurde mit 800 µL, 4 °C kaltem, 10 %igem Glycerin (v/v) überschichtet und im Elektroporator (2500 V, 25 µF, 200 Ω, 2 mm) elektroporiert. Nach der Elektroporation wurde die Mischung in 0,5 mL auf 46 °C vorgewärmtes BHIS (Brain-Heart-Infusion (Sigma Aldrich) supplementiert mit 91 g/L Sorbitol) Medium überführt und für 6 min bei 46 °C inkubiert. Anschließend wurden die Zellen für zwei Stunden bei 30 °C mit 170 Upm inkubiert und die Suspension auf BHI-Kan25-Agar ausplattiert.

### Deletionsanalyse des iol1 Inositol-Gen-Clusters

Anschließend wurde die Funktion des sacB-Gens getestet, indem die Klone auf BHI-Kan25-Agar und auf BHI-Kan25-Agar mit 10%- Saccharose übertragen wurden. Zur Selektion auf erfolgreiche Exzision bei einem zweiten Rekombinationsereignis wurden die auf BHI-Kan25 kultivierten Zellen für ca. 6 h in 1 ml BHI-Medium (Sigma Aldrich) kultiviert und 20 µl der Kultur einer 1:10-Verdünnung auf BHI-Agar mit 10 % Saccharose ausplattiert. Die Klone wurden auf BHI-Kan25-Agar und BHI-Agar mit 10 % Saccharose übertragen. Von den Saccharose-resistenten und Kanamycin-sensitiven Klonen wurde eine Kolonie-PCR ((DreamTaq Green PCR Master Mix (Thermofisher Scientific) Annealing Temp: 50°C, Zyklen: 30, Elongation: 2:00 min P013_Col deliol_FW x P014_Col deliolR-E2_RV mit anschließender Gelelektrophorese durchgeführt, um die erfolgreiche Deletion zu bestätigen.

### Transformation von C. glutamicum MB001(DE3)Δiol1 mit pK19mobsacBΔiol2 zur Herstellung von C. glutamicum MB001(DE3)Δiol1Δiol2:

Elektrokompetente C. glutamicum MB001(DE3)Δiol1-Zellen wurden analog wie oben beschrieben hergestellt. Nach Isolierung des Plasmids pK19mobsacBΔiol2 wurde ein 100 µL Aliquot elektrokompetenter C. glutamicum Zellen auf Eis aufgetaut, dieses mit 1 µg des Plasmids vermischt und in eine auf Eis vorgekühlte 0,2 cm Elektroporationsküvette überführt. Das weitere Vorgehen erfolgte analog wie oben beschrieben.

### Deletionsanalyse des iol2 Inositol-Gen-Clusters

Anschließend wurde die Funktion des sacB-Gens getestet, indem die Klone auf BHI-Kan25-Agar und auf BHI-Kan25-Agar mit 10%- Saccharose übertragen wurden. Zur Selektion auf erfolgreiche Exzision bei einem zweiten Rekombinationsereignis wurden die auf BHI-Kan25 kultivierten Zellen für ca. 6 h in 1 ml BHI-Medium (Sigma Aldrich) kultiviert und 20 µl der Kultur einer 1:10-Verdünnung auf BHI-Agar mit 10 % Saccharose ausplattiert. Die Klone wurden auf BHI-Kan25-Agar und BHI-Agar mit 10 % Saccharose übertragen. Von den Saccharose-resistenten und Kanamycin-sensitiven Klonen wurde eine Kolonie-PCR ((DreamTaq Green PCR Master Mix (Thermofisher Scientific) Annealing Temp: 48°C, Zyklen: 30, Elongation: 2:00 min Primer P015_Col_deloxiC-E_FWx P016_Col_deloxiC-E_RV1 mit anschließender Gelelektrophorese durchgeführt, um die erfolgreiche Deletion zu bestätigen.

### Primer für die Analyse der Deletionen von iol1 und iol2

| | |
|---|---|
| P013_Col_deliol_FW | GGGATTTCGTTGCCATG |
| P014_Col_deliolR-E2_RV | GGTTGCGGCAATCTTCC |
| P015_Col_deloxiC-E_FW | ACACCATCCGGGACAC |
| P016_Col_deloxiC-E_RV1 | CGTTCAAGACGTCATC |

### Herstellung von Corynebacterium glutamicum MB001(DE3)ΔIOL

C. glutamicum MB001(DE3)ΔIOL wurde nach Niebisch und Bott (2001) (https://doi.org/10.1007/s002030100262) mit dem Vektor pK19mobsacB über doppelt homologe Rekombination (Schäfer et al., 1994) (https://doi.ora/10.1016/0378-1119(94)90324-7) konstruiert. Als Ausgangsstamm diente der bereits hergestellte C. glutamicum MB001(DE3)Δiol1Δiol2 Stamm (Ramp, Paul, et al. "Metabolic engineering of Corynebacterium glutamicum for production of scyllo-inositol, a drug candidate against Alzheimer's disease. Metabolic Engineering 67 (2021): 173-1852021).

### Konstruktion des Plasmids pK19Δcg2312-13

Dazu wurden in einem ersten Schritt zwei PCR-Produkte generiert, die Teile der 5'-Region des cg2312-cg2313-Genclusters (Primer: P01_pK19Δcg2312-13_FW1/P02_pK19Δcg2312-13_RV1) bzw. Teile der 3'-Region (Primer: P03_pK19Δcg2312-13_FW2/P04_pK19Δcg2312-13_RV2) enthielten. Für die spätere homologe Rekombination wurden jeweils 1000 Basenpaare der flankierenden Regionen amplifiziert (Annealing Temp: 65°C, Elongation: 0:30 min).

Im zweiten Schritt wurden die DNA-Fragmente (SEQ ID NO. 15) zusammen mit dem bereits über die Restriktionsendonuclease EcoRI geschnittenen pk19mobsac8-Vektor mittels Gibson Assembly fusioniert (Gibson *et al.,* 2009) (https://doi.org/10.1038/NMETH.1318).

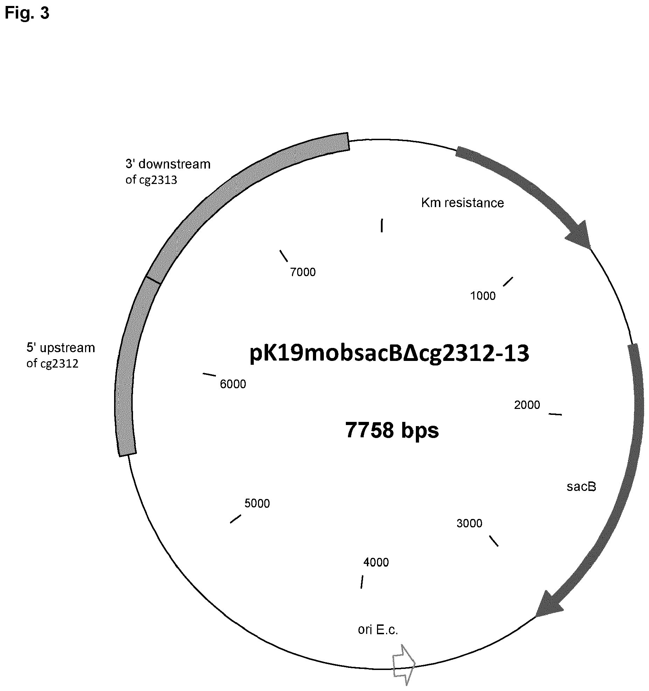

Das so erhaltene Plasmid pK19*mobsacB*_Δcg2313 (Fig. 3) wurde mittels Hitzeschock bei 42°C für 90 Sekunden in *E*. *coli* DH5α transformiert. Wegen des auf dem Plasmid vorliegenden Kanamycinresistenzgens konnten nur solche Klone wachsen, die das Plasmid aufgenommen hatten. Diese wurden mittels Kolonie-PCR und anschließender Gelelektrophorese auf ihre Korrektheit überprüft, und deren DNA-Sequenz wurde bestimmt.

### Primer zur Konstruktion von pK19mobSacBΔcg2312-13

| | |
|---|---|
| P01_pK19Δcg2312-13_FW1 | GAGGATCCCCGGGTACCGAGCTCGGAATCTG ACTCCGAGCAG |
| P02_pK19Δcg2312-13_RV1 | AAGTCAGCGTTCACGGTC |
| P03_pK19Δcg2312-13_FW2 | ATTTAGACCGTGAACGCTGACTTGAGGGCAAG TTTGACTGAC |
| P04_pK19Δcg2312-13_RV2 | CGTTGTAAAACGACGGCCAGTGAATTATGGTG GTCAAGCCGATG |

### Transformation von C. glutamicum

Nach Isolierung des Plasmids wurde ein 100 µL Aliquot elektrokompetenter *C*. *glutamicum* Zellen (Herstellung wie zuvor beschrieben) auf Eis aufgetaut, dieses mit 1 µg des Plasmids vermischt und in eine auf Eis vorgekühlte 0,2 cm Elektroporationsküvette überführt. Die Mischung wurde mit 800 µL, 4 °C kaltem, 10 %igem Glycerin (v/v) überschichtet und im Elektroporator (2500 V, 25 µF, 200 Ω, 2 mm) elektroporiert. Nach der Elektroporation wurde die Mischung in 0,5 mL auf 46 °C vorgewärmtes BHIS Medium überführt und für 6 min bei 46 °C inkubiert. Anschließend wurden die Zellen für zwei Stunden bei 30 °C mit 170 Upm inkubiert und die Suspension auf BHI-Kan25-Agar ausplattiert.

### Deletionsanalyse

Anschließend wurde die Funktion des sacB-Gens getestet, indem die Klone auf BHI-Kan25-Agar und auf BHI-Kan25-Agar mit 10%- Saccharose übertragen wurden. Zur Selektion auf erfolgreiche Exzision bei einem zweiten Rekombinationsereignis wurden die auf BHI-Kan25 kultivierten Zellen für ca. 6 h in 1 ml BHI-Medium kultiviert und 20 µl der Kultur einer 1:10-Verdünnung auf BHI-Agar mit 10 % Saccharose ausplattiert. Die Klone wurden auf BHI-Kan25-Agar und BHI-Agar mit 10 % Saccharose übertragen. Von den Saccharose-resistenten und Kanamycin-sensitiven Klonen wurde eine Kolonie-PCR (Primer: P05_ΔCg2312-13_FW/P06_ΔCg2312-13_RV, Annealing Temp. 45 °C, Elongation. 2:00 min) mit anschließender Gelelektrophorese durchgeführt, um die erfolgreiche Deletion zu bestätigen.

### Primer für die Analyse der Deletion ΔCg2312-13

| | |
|---|---|
| P05_ΔCg2312-13_FW | GTTCCATCAAAGTCAATGC |
| P06_ΔCg2312-13_RV | GTGCGCCGTGTGATCAATG |

### Aufreinigung der Enzyme der NAD⁺- und NADPH-abhängigen Dehydrogenasen:

Für die Aufreinigung der Enzyme werden die entsprechenden Gensequenzen in ein Plasmid, unter die Kontrolle eines starken Promoters, z.B T7, vor oder nach der kodierende Sequenz eines Strep-Tags II kloniert, wodurch nach Transkription und Translation ein Fusionsprotein mit N- oder C-terminalen Strep-Tag II gebildet wird. Dies kann beispielsweise durch die Expression des entsprechenden Gens mit Vektor pET51b (Novagen) in E.coli BL21(DE3) erreicht werden. Transformierte E.coli Stämme werden in LB Medium bei 37°C kultiviert. Die Genexpression wird wenn notwendig per Hinzugabe des entsprechenden Inducermoleküls nach ca. 3h induziert. Im Beispiel mit pET51b würden 1mM IPTG Endkonzentration nach 3h hinzugegeben. Die induzierten Zellen werden für weitere 18h bei 37°C kultiviert. Die Zellen werden durch Zentrifugation (>3000 xg) pelletiert und in Lysis-Puffer resuspendiert (bsp: 100mM TRIS, 150mM NaCl, pH 7.5). Der Zellaufschfluss der in der Zellsuspension enthaltenen Zellen wird durch mehrere Passagen durch eine French press bei >15000 psi aufgeschlossen. Überschüssige Zelltrümmer, Membranfragmente etc. werden durch erneute Zentriguagtion bei (>3000 xg) und anschließender Ultrazentrifugation (>45000 xg) von der löslichen Proteinfraktion getrennt. Der entstandene Überstand wird auf eine Affinitätschromatogrtaphie für StrepTag II basierte Aufreinigung geladen. In diesem Beispiel: eine StrepTrap ^{®} HP (Cytiva, 1-5mL), welche mit Strepavidin beladen ist und den StrepTag II bindet. Die angewandte Flussrate und das Säulenvolumen werden nach Herstellangaben verfolgt und dem Prozess individuell angepasst.

Nach auftragen der Proteinsuspension wird die Säule mit Puffer (100mM TRIS, 150mM NaCL, pH 7.5) für 5-10 Säulenvolumen gewaschen. Die Elution des Zielproteins erfolgt durch Waschen der Säule mit Puffer, welcher 2.5mM Desthiobiotin enthält.

Die Reinheit des Zielproteins wird mittels SDS-PAGE nach allgemein bekannten Methoden ermittelt. Die Proteinkonzentration wird durch Anwendung eines photometrischen Assays mit Hilfe des Bradford Reagenz (Sigma Aldrich), Messung der Absorption der Protein-Reagenzlösung bei 595 nm und anschließender Berechnung anhand einer Standardgeraden bestimmt.

### Bestimmung der enzymatischen Aktivität der NAD⁺- und NADPH-abhängigen Dehydrogenasen

In einem Gesamtvolumen von 600 µl wurden die Enzymtests zur Aktivitätsbestimmung der erfindungsgemäßen NAD⁺- und NADPH-abhängigen Dehydrogenasen OEPa und OEPb durchgeführt. Die Enzymtests wurden durch Zugabe von 50 µL Substratlösung mit verschiedenen myo-Inositol-Konzentrationen (0 - 50 mM, für OEPa) oder verschiedenen 1-keto-D-chiro-Inositol-Konzentrationen (0 - 50 mM, für OEPb) zu 550 µL Reaktionsmedium (100 mM Tris-HCl, 50 mM NAD⁺, 5 mM MgCl₂ und 1,0 mg OEPa-Protein, pH 7,5) bzw. (100 mM Tris-HCl, 50 mM NADPH, 5 mM MgCl₂ und 1,0 mg OEPb-Protein, pH 7,5) gestartet. Die enzymatische Aktivität wurde durch Messung der initialen NADH-Bildung für OEPa bzw. Abnahme von NADPH für OEPb bei 340 nm und 30 °C photometrisch bestimmt. Zur Bestimmung der maximalen Enzymaktivität (vmax) und der spezifischen Substrataffinität (Km) wurden die experimentellen Daten mittels nichtlinearer Regression an eine Michaelis-Menten-Kinetik gefittet.

### In-vitro Herstellung von D-chiro-Inositol

1 mg des wie zuvor beschrieben aufgeregtes Enzym der NAD+ abhängigen Dehydrogenase (OEPa) wurde mit 1 mg der aufgeregten, NADPH abhängigen Dehydrogenase (OEPb) in einem Reaktionsansatz eines Volumens von 2 mL mit 10 g/L *myo-*Inositol, 60mM NAD⁺ und 60mM NADPH in einem gepufferten System (K₂HPO₄/KH₂PO₄, 100mM, pH 7.5) eingesetzt und bei 37°C für 24h inkubiert. Nach 24h wurde D-*chiro*-Inositol mittels HPLC nach gängigem Vorgehen nachgewiesen. Die Ausbeute lag bei <10g/L.

### Medium und Kultivierungsbedingungen

Zur Kultivierung von *C. glutamicum-Stämmen* wurde komplexes Brain Heart Infusion-Medium (BHI, Difco Laboratories, Detroit, USA) und definiertes CGXII-Medium verwendet. Das CGXII-Medium enthielt pro Liter entionisiertem Wasser folgende Zusammensetzung: 1 g K₂HPO₄, 1 g KH₂PO₄, 5 g Harnstoff, 13.25 mg CaCl₂·2H₂O, 0.25 g MgSO₄·7H₂O, 10 mg FeSO₄·7H₂O, 10 mg MnSO₄·H₂O, 0.02 mg NiCl₂·6H₂O, 0.313 mg CuSO₄·5H₂O, 1 mg ZnSO₄·7H₂O, 0.2 mg Biotin. Für die Kultivierung von Stämmen mit Vektoren wurde Kanamycin und Isopropyl β-D-thiogalactoside (IPTG) zu finalen Konzentrationen von 25 µg mL⁻¹ bzw. 0,5 mmol L⁻¹ zugegeben. Alle Chemikalien wurden von Sigma-Aldrich (Steinheim, Deutschland) erworben. Zunächst wurden Vorkulturen auf BHI-Medium in Reagenzgläsern aus Einzelkolonien beimpft und für 8 h bei 30°C auf einem Rotationsschüttler bei 170 U min-' inkubiert. Aus dieser Kultur wurde dann eine zweite Vorkultur in 100 mL Schüttelkolben mit 10 mL definiertem CGXII Medium sowie 20 g L⁻¹ D-Glucose und 25 µg mL⁻¹ beimpft und für 15 h bei 30 °C auf einem Rotationsschüttler bei 130 U min-' inkubiert. Anschließend wurde die Hauptkultur auf eine OD₆₀₀ von 1 in 50 mL definiertem CGXII-Medium mit 20 g L⁻¹ D-Glucose und 10 g L⁻¹ D-myo-Inositol beimpft und für 72 h bei 30°C auf einem Rotationsschüttler bei 130 U min-' inkubiert. Aus der Hauptkultur wurden Proben zur Messung der Konzentrationen von Biomasse, D-myo-Inositol und D-chiro-Inositol entnommen.

### Codonoptimierung heterologer Gene in coryneformen Bakterienzellen

Die Etablierung synthetischer Biosynthesewege, wie der Synthese von Polyphenolen oder Polyketiden, aus Pflanzen in coryneformen Bakterienzellen erfordert eine heterologe Expression der erforderlichen pflanzlichen Gene. Es ist bekannt, dass verschiedene Spezies Varianten des universellen genetischen Codes mit unterschiedlicher Häufigkeit verwenden, was letztendlich auf unterschiedliche tRNA-Konzentrationen innerhalb der Zelle zurückzuführen ist. Man spricht in diesem Fall von Codonverwendung (engl. *Codon Usage*). Selten verwendete Codons können die Translation ausbremsen, während häufiger genutzte Codons die Translation beschleunigen können. Dies führt dazu, dass heterologe Gene mit Codonverwendung spezifisch für den Zielorganismus synthetisiert werden. Hierzu wird die Aminosäuresequenz des heterologen Proteins von Interesse in die DNA-Sequenz mit spezifischer Codonverwendung umgeschrieben. Für *C*. *glutamicum* ist eine Datenbank über die Codonverwendung verfügbar unter http://www.kazusa.or.jp/codon/cqibin/showcodon.cgi?species=196627&aa=1&style=N.

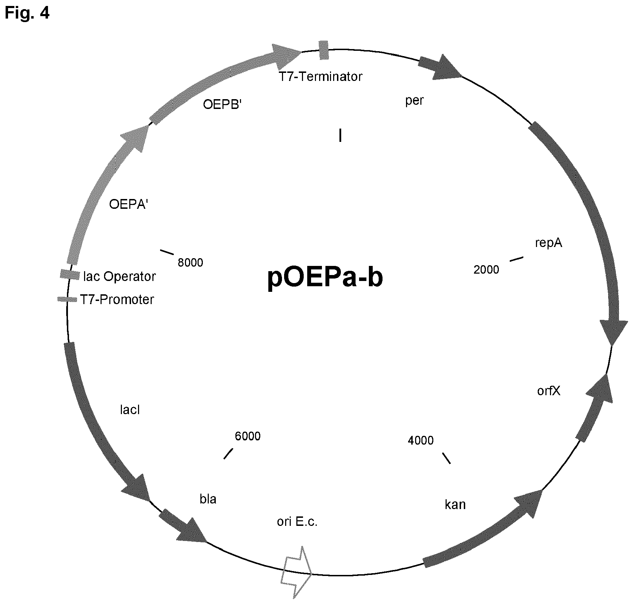

### Konstruktion des Plasmids pOEPa-b (Fig. 4)

DNA-Stränge, die jeweils für die Dehydrogenasen aus *Medicago truncatula* (MtOEPa und MtOEPb) kodieren (SEQ ID NO. 3 und 4), wurden für den Wirtsorganismus Co*rynebacterium glutamicum* Codon-optimiert synthetisiert (SEQ ID NO. 5 und SEQ ID NO. 6). Bei den Codon-optimierten OEPa (SEQ ID NO. 5) und OEPb-Genen (SEQ ID NO. 6) handelt es sich um die erfindungsgemäß besonders vorteilhaften kodierenden Nukleinsäuresequenzen. pEOPa-b wird in Sinne der Erfindung und auch nachfolgend synonym benutzt mit pMKEx2-OEPa-b.

Die beiden Gensequenzen kodierend für die erfindungsgemäße NAD⁺-abhängige und NADPH-abhängige Dehydrogenase-Gensequenzen wurden mittels Polymerase-Kettenreaktion (PCR) der synthetisierten DNA-Stränge (hergestellt im Auftrag der Erfinder von GeneArt Gensynthese und Serviceleistungen, Thermo Fisher Scientific, Deutschland) amplifiziert und in zwei verschiedenen Genanordnungen in das Plasmid pMKEx2 (Kortmann, M., et al., A chromosomally encoded T7 RNA polymerase-dependent gene expression system for Corynebacterium glutamicum: Construction and comparative evaluation at the single-cell level. Microb. Biotechnol. 8, 253-265, 2015) kloniert, um das rekombinante Plasmid pOEPa-b (Fig. 4) zu erhalten.

Um pMKEx2-OEPa-b (Fig. 4) zu konstruieren, wurde OEPa (SEQ ID NO. 5) mit den Oligonukleotiden P07_pOEPa_FW1 und P08_pOEPa-b_RV1, Anneal. Temp 65 °C, Elong. 0:30 min) und OEPb (SEQ ID NO. 6) mit den Oligonukleotiden P09_pOEPa-b_FW2 und P10_pOEPb_RV2, Anneal. Temp 64 °C, Elong. 0:30 min) amplifiziert und in das Plasmid pMKEx2, welche mit den Restriktionsenzymen Notl und Ncol geschnitten wurde, mittels Gibson-Assembly fusioniert. Dieses so erhaltene Plasmid wurde mittels Hitzeschock bei 42°C für 90 Sekunden in *E*. *coli* DH5α transformiert. Aufgrund des auf dem Plasmid vorliegenden Kanamycinresistenzgens konnten nur solche Klone wachsen, die das Plasmid aufgenommen hatten. Diese wurden mittels Kolonie-PCR und anschließender Gelelektrophorese auf ihre Korrektheit überprüft, und deren DNA-Sequenz bestimmt.

### Primer zur Konstruktion von pOEPa-b

| | |
|---|---|
| P07_pOEPa_FW | TTTAACTTTAAGAAGGAGATATACCATGTCCAAGACCGTGTGC |
| P08_pOEPa_RV | GCAGGTGCACAATGATACGATTACACCAGGCCACGGGAC |
| P09_pOEPb_FW | TTTAACTTTAAGAAGGAGATATACCATGGCAGGCAACAAGATCCC |
| P10_pOEPb_RV | TGGCACCAGAGCGAGCTCTGCGGCCTTACACGTCGCCATCCCAC |

### Produktion von D-chiro-Inositol aus myo-Inositol mittels genetisch modifiziertem C. glutamicum MB001(DE3)ΔIOL OEPa-b

Das wie vorstehend beschrieben erhaltene rekombinanten Plasmide pMKEx2-OEPa-b (Fig. 4) wurde in *C*. *glutamicum* MB001(DE3)Δ*IOL* eingeführt, der Inositole nicht abbauen oder verwerten kann.

Nach Isolierung des Plasmids wurde ein 100 µL Aliquot elektrokompetenter *C*. *glutamicum* Zellen auf Eis aufgetaut, dieses mit 1 µg des Plasmids vermischt und in eine auf Eis vorgekühlte 0,2 cm Elektroporationsküvette überführt. Die Mixtur wurde mit 800 µL, 4 °C kaltem, 10 %igem Glycerin (v/v) überschichtet und im Elektroporator (2500 V, 25 µF, 200 Ω, 2 mm elektroporiert. Nach der Elektroporation wurde die Mischung in 0,5 mL auf 46 °C vorgewärmtes BHIS Medium überführt und für 6 min bei 46 °C inkubiert. Anschließend wurden die Zellen für zwei Stunden bei 30 °C mit 170 Upm inkubiert und die Suspension auf BHI-Kan25-Agar ausplattiert. Einzelkolonien transformierter Zellen auf Agarplatten wurden verwendet, um eine erste Vorkultur in 5 mL BHI-Medium, supplementiert mit 25 µg/mL Kanamycin, zu starten und für 6 h bei 30°C und 170 Upm zu inkubieren. Mit 1 ml der ersten Vorkultur wurde eine zweite Vorkultur in einem 100-mL-Schüttelkolben mit 10 mL CGXII-Medium + 2 % (w/v) Glukose und 25 µg/mL Kanamycin beimpft. Die zweite Vorkultur wurde 16 Stunden lang bei 30°C und 120 Upm inkubiert. Als Hauptkultur wurden 50 ml CGXII-Medium mit 2 % (w/v) Glukose, 25 µg/ml Kanamycin und 1 % (*w*/*v*) *myo-*Inositol mit einer anfänglichen OD600 (optische Dichte bei 600 nm) von 1 beimpft und 72 Stunden lang bei 30 °C und 130 Upm inkubiert. Die Expression der Gene OEPa (SEQ ID NO. 5) und OEPb (SEQ ID NO. 6) wurde durch Zugabe von 0,5 mM Isopropyl-ß-D-thiogalactosid (IPTG) zu Beginn der Kultivierung induziert. Als Negativkontrolle diente ein *C*. *glutamicum* MB001(DE3)Δ*IOL* -Stamm, der mit pMKEx2 transformiert worden war.

Für die Analyse von *myo*- und D-*chiro*-Inositol wurde 1 mL Kultur bei 17.000 G für 20 min zentrifugiert, der Überstand wurde filtriert (0,2 µm Spritzenfilter, Whatman^{™}, GE Healthcare, Freiburg, Deutschland) und bis zur weiteren Analyse bei -20 °C eingefroren. Die aufgetauten Proben wurden mit deionisiertem Wasser verdünnt und für die HPLC-Analyse verwendet. Eine 5-pl-Probe wurde mit einem Agilent LC-1 100 System (Agilent, Santa Clara, CA, USA) untersucht, das mit einer Carbo-Pb Guard Cartridge (Phenomenex, Aschaffenburg, Deutschland) und einer Rezex UPM-Monosaccharid 300 × 7,8 mm Säule (Phenomenex, Aschaffenburg, Deutschland) ausgestattet war. Die Trennung erfolgte bei 85 °C mit Wasser als Elutionsmittel bei einer Flussrate von 0,6 mL/min. Zucker und Zuckeralkohole wurden mit einem bei 35 °C betriebenen Brechungsindexdetektor nachgewiesen.

Die Herstellung von D-chiro-Inositol (DCI) aus myo-Inositol (MI) in *C*. *glutamicum* MB001(DE3)Δ*IOL* durch Expression der Codon-optimierten NAD⁺-abhängigen bzw. NADPH-abhängigen Dehydrogenasen kloniert auf dem Plasmid pEOPa-b (Fig. 4) ist in Fig. 8 gezeigt. Innerhalb von 48 Stunden konnte bis zu 1,6 g/L DCI pro 10 g/L myo-Inositol und 20 g/L Glukose akkumuliert werden. Dies entspricht einer Effizienz von 16 %. Durch geeignete biotechnologische Prozessführung lässt sich diese Produktbildung noch weiter optimieren. Eine vorteilhafte Variante ist beispielweise die Herstellung von DCI in einer für den Fachmann bekannten und etablierten fed-batch-Kultivierung, bei der eine erhöhte Anfangskonzentration von Glukose (~ 40 g/L) und anschließend Glukose in einer Konzentration von 1 - 4 g/L*h kontinuierlich zugefüttert wird.

### Konstruktion des Plasmids plnoDCI (Fig. 5)

Die Herstellung des erfindungsgemäßen, rekombinanten Plasmids pInoDCI (Fig. 5) wurde wie folgt durchgeführt:
Die ino1-Gensequenz (SEQ ID NO. 12) kodierend für die myo-Inositol-1-Phosphat-Synthase (Ino1) wurde wurden mittels Polymerase-Kettenreaktion (PCR) von isolierter, genomsicher DNA von C. *glutamicum* amplifiziert. Zur Isolation genomischer DNA wurden *C*. *glutamicum-Zellen* durch Suspension in 50 µL 2 %-igem DMSO und anschließender Inkubation für 5 Minuten bei 95 °C aufgebrochen. Die Zelltrümmer wurden für 1 min bei 11.000 Upm abzentrifugiert und 3 µL des Überstandes als Template zur Amplifikation von ino1 (SEQ ID NO. 12) verwendet. Die beiden Dehydrogenase-Gensequenzen wurden mittels Polymerase-Kettenreaktion (PCR) des erfindungsgemäßen Expressionsplasmids pOEPa-b (Fig. 4) amplifiziert.

Um pInoDCI (Fig. 5) zu konstruieren, wurde das Gen ino1 (SEQ ID NO. 12) mit den Oligonukleotiden P15_pInoDCI_FW1 und P16_pInoDCI_RV, Anneal. Temp 67 °C, Elong. 0:30 min) (siehe unten) von genomischer DNA und die synthetische Genkassette OEPa-b (SEQ ID NO. 5 und SEQ ID NO. 6) mit den Oligonukleotiden P17_plnoDCI_FW2 und P10_pOEPab_RV2, Anneal. Temp 70 °C, Elong. 1:00 min) (siehe unten) von dem bereits oben beschriebenen Expressionsplasmid pOEPa-b amplifiziert und mit dem Plasmid pMKEx2, welches mit den Restriktionsenzymen Notl und Ncol geschnitten wurde, mittels Gibson-Assembly fusioniert. Dieses so erhaltene Plasmid wurde mittels Hitzeschock bei 42°C für 90 Sekunden in *E*. *coli* DH5α transformiert. Aufgrund des auf dem Plasmid vorliegenden Kanamycinresistenzgens konnten nur solche Klone wachsen, die das Plasmid aufgenommen hatten. Diese wurden mittels Kolonie-PCR und anschließender Gelelektrophorese auf seine Korrektheit überprüft, und dessen DNA-Sequenz bestimmt. pInoDCI wird in Sinne der Erfindung und auch nachfolgend synonym benutzt mit pMKEx2-InoDCI.

### Primer zur Konstruktion von pInoDCI

| | |
|---|---|
| P15_pInoDCI_FW1 | CTTTAAGAAGGAGATATACCATGAGCACGTCCACCATCAG |
| P16_pInoDCI_RV | ACATCGTTGAGTGGTCACCGTTACGCCTCGATGATGAATG |
| P17_plnoDCI_FW2 | CGGTGACCACTCAACGATGTGAAGGAGATATACCATGTCCAAGACCG |
| P10_pOEPab_RV2 | TGGCACCAGAGCGAGCTCTGCGGCCTTACACGTCGCCATCCCAC |

Die Expression der Nukleinsäuresequenz kodierend ino1 und der erfindungsgemäßen Nukleinsäuresequenzen kodierend für eine NAD⁺-abhängige Dehydrogenase und eine NADPH-abhängige Dehydrogenase erfolgt in pInoDCI unter der Kontrolle des gemeinsamen T7-Promotors.

### Konstruktion des Plasmids pMKEx2-BiT7 (Fig. 6):

Um pMKEx2-BiT7 (Fig. 6) zu konstruieren, wurde die Multiple Cloning Site von pET-Duet^{™}-1 (Cat. No. 71146-3, Novagen) mit den mit den Oligonukleotiden P18_pMKEx2-BiT7_FW1 und P19_pMKEx2-BiT7_RV1, Anneal. Temp 69 °C, Elong. 0:30 min) und das Plasmid pMKEx2 (Kortmann, Maike, et al. "A chromosomally encoded T 7 RNA polymerase-dependent gene expression system for Corynebacterium glutamicum: construction and comparative evaluation at the single-cell level", Microbial biotechnology 8.2 (2015): 253-265) mit den Oligonukleotiden P20_pMKEx2-BiT7_FW3 und P21_pMKEx2-BiT7_RV2, Anneal. Temp 61 °C, Elong. 4:00 min) amplifiziert und mittels Gibson-Assembly fusioniert. Dieses so erhaltene Plasmid wurde mittels Hitzeschock bei 42°C für 90 Sekunden in *E*. *coli* DH5α transformiert. Aufgrund des auf dem Plasmid vorliegenden Kanamycinresistenzgens konnten nur solche Klone wachsen, die das Plasmid aufgenommen hatten. Diese wurden mittels Kolonie-PCR und anschließender Gelelektrophorese auf ihre Korrektheit überprüft, und deren DNA-Sequenz bestimmt. pBiT7 wird in Sinne der Erfindung und auch nachfolgend synonym benutzt mit pMKEx2-BiT7.

### Primer für die Konstruktion von pBiT7:

| | |
|---|---|
| P18_pM KEx2-BiT7_FW1 | TCGGGCTCATGAGCGCTTGTTTCGGTAATCGTATT GTACACGGCCG |
| P19_pMKEx2-BiT7_RV1 | CGGCCACGGGGCCTGCCACCATACATCCGGATAT AGTTCCTCC |
| P20_pM KEx2-BiT7_FW2 | CCGAAACAAGCGCTCATG |
| P21_pMKEx2-BiT7_RV2 | GTATGGTGGCAGGCCCCGTG |

### Konstruktion des Plasmids pBiT7-InoDCI (Fig. 7):

Um das entsprechende pBiT7-InoDCI Plasmid (Fig. 7) zu konstruieren, wurde ino1 mit den Oligonukleotiden P15_pInoDCI_FW1 und P22_pBiT7-InoDCI_RV1, Anneal. Temp 67 °C, Elong. 0:30 min amplifiziert und mit dem Plasmid pMKEx2, welche mit den Restriktionsenzymen Notl und Ncol geschnitten wurde, mittels Gibson-Assembly fusioniert. Das daraus resultierende Plasmid wurde mittels dem Restriktionsenzym Ndel geschnitten und via Gibson Assembly fusioniert mit OEPa-OEPb, welche von pOEPa-b (Fig. 4) mit den Oligonukleotiden P23_pBiT7-InoDCI_FW2 und P24_pBiT7-InoDCI_RV2, Anneal. Temp 66 °C, Elong. 1:00 min amplifiziert wurden. Das so erhaltene Plasmid wurde mittels Hitzeschock bei 42°C für 90 Sekunden in *E*. *coli* DH5α transformiert. Aufgrund des auf dem Plasmid vorliegenden Kanamycinresistenzgens konnten nur solche Klone wachsen, die das Plasmid aufgenommen hatten. Diese wurden mittels Kolonie-PCR und anschließender Gelelektrophorese auf ihre Korrektheit überprüft und deren DNA-Sequenz bestimmt. pBiT7-InoDCI wird in Sinne der Erfindung und auch nachfolgend synonym benutzt mit pMKEx2-BiT7-InoDCI.

### Primer für die Konstruktion von pBiT7-InoDCI

| | |
|---|---|
| P15_pInoDCI_FW1 | CTTTAAGAAGGAGATATACCATGAGCACGTCCACCA TCAG |
| P22_pBiT7-InoDCI_RV1 | GCACCAGAGCGAGCTCTGCGGCCTTACGCCTCGAT GATGAATG |
| P23_pBiT7-InoDCI_FW2 | GTTAAGTATAAGAAGGAGATATACAATGTCCAAGAC CGTGTGC |
| P24_pBiT7-InoDCI_RV2 | CGATATCCAATTGAGATCTGCCATATTACACGTCGCC ATCCCAC |

Für die poly-cistrone Expression der Nukleinsäuresequenz (ino1, SEQ ID NO. 12) kodierend für eine myo-Inositol-1-Phosphat-Synthase (Ino1) wurde das Gen ino1 unter die Kontrolle des ersten T7 Promoters (T7-1) kloniert und für die Expression der Nukleinsäuresequenz (SEQ ID NO. 5; OEPa) kodierend für eine erfindungsgemäße NAD⁺-abhängige und der Nukleinsäuresequenz (SEQ ID NO. 6, OEPb) kodierend für eine erfindungsgemäße NADPH-abhängige Dehydrogenase wurden beide Gene unter der Kontrolle eines T7-Promotors (T7-2) kloniert.

### Produktion von D-chiro-Inositol aus Glukose mittels genetisch modifiziertem C. glutamicum MB001(DE3)ΔIOL

Die vorstehend erhaltenen rekombinanten Plasmide pInoDCI (Fig. 5) und pBiT7-InoDCI (Fig. 7) wurden jeweils in *C*. *glutamicum* MB001(DE3)ΔIOL eingeführt, der Inositole nicht abbauen oder verwerten kann.

Nach Isolierung des Plasmids wurde ein 100 µL Aliquot elektrokompetenter *C. glutamicum* Zellen auf Eis aufgetaut, dieses mit 1 µg des Plasmids vermischt und in eine auf Eis vorgekühlte 0,2 cm Elektroporationsküvette überführt. Die Mischung wurde mit 800 µL, 4 °C kaltem, 10 %igem Glycerin (v/v) überschichtet und im Elektroporator (2500 V, 25 µP, 200 Ω, 2 mm elektroporiert. Nach der Elektroporation wurde die Mixtur in 0,5 mL auf 46 °C vorgewärmtes BHIS-Medium überführt und für 6 min bei 46 °C inkubiert. Anschließend wurden die Zellen für zwei Stunden bei 30 °C mit 170 Upm inkubiert und die Suspension auf BHI-Kan25-Agar ausplattiert.

Einzelkolonien transformierter Zellen auf Agarplatten wurden verwendet, um eine erste Vorkultur in 5 mL BHI-Medium supplementiert mit 25 µg/mL Kanamycin zu starten und für 6 h bei 30°C und 170 Upm zu inkubieren. Mit 1 ml der ersten Vorkultur wurde eine zweite Vorkultur in einem 100-mL-Schüttelkolben mit 10 mL CGXII-Medium + 2 % (w/v) Glukose und 25 µg/mL Kanamycin beimpft. Die zweite Vorkultur wurde 16 Stunden lang bei 30°C und 120 Upm inkubiert. Als Hauptkultur wurden 50 ml CGXII-Medium mit 2 % (w/v) Glukose und 25 µg/ml Kanamycin mit einer anfänglichen OD600 (optische Dichte bei 600 nm) von 0,5 beimpft und 72 Stunden lang bei 30 °C und 130 Upm inkubiert. Die Expression der Gene wurde durch Zugabe von 0,5 mM Isopropyl-ß-D-thiogalactosid (IPTG) drei Stunden nach Beginn der Kultivierung induziert. Als Negativkontrolle diente ein *C*. *glutamicum* MB001(DE3)Δ*IOL*-Stamm, der mit pMKEx2 transformiert worden war.

Für die Analyse von myo- und D-chiro-Inositol wurde 1 mL Kultur bei 17.000 g für 20 min zentrifugiert, der Überstand wurde filtriert (0,2 µm Spritzenfilter, Whatman^{™}, GE Healthcare, Freiburg, Deutschland) und bis zur weiteren Analyse bei -20 °C eingefroren. Die aufgetauten Proben wurden mit deionisiertem Wasser verdünnt und für die HPLC-Analyse verwendet. Eine 5-µl-Probe wurde mit einem Agilent LC-1100 System (Agilent, Santa Clara, CA, USA) untersucht, das mit einer Carbo-Pb Guard Catridge (Phenomenex, Aschaffenburg, Deutschland) und einer Rezex RPM-Monosaccharid 300 × 7,8 mm Säule (Phenomenex, Aschaffenburg, Deutschland) ausgestattet war. Die Trennung erfolgte bei 85 °C mit Wasser als Elutionsmittel bei einer Flussrate von 0,6 mL/min. Zucker und Zuckeralkohole wurden mit einem bei 35 °C betriebenen Brechungsindexdetektor nachgewiesen.

Die Bildung von D-chiro-Inositol (DCI) und myo-Inositol (MI) ausgehend von Glucose in *C*. *glutamicum* MB001(DE3)ΔIOL durch Expression der Codon-optimierten NAD⁺-abhängigen bzw. NADPH-abhängigen Dehydrogenasen plus der myo-Inositol-1-Phosphat-Synthase (!no1) kloniert auf den Plasmiden pInoDCI und pBiT7-InoDCI ist in Fig. 9 gezeigt.

*C*. *glutamicum* MB001(DE3)Δ*IOL_*plnoDCI produzierte ca. 0,7 g/L DCI und 0,3 g/L MI ausgehend von 20 g/L Glukose. Dies entspricht einer Effizienz von 3,5 % berechnet ausgehend von der eingesetzten Glukosekonzentration. Da in diesem Prozess Glukose ebenfalls als Kohlenstoffquelle zur Biomasseproduktion genutzt wird, liegt die tatsächliche Effizienz der Produktbildung ausgehend von der nicht zur Biomassebildung genutzen Glukosekonzentration höher.

C. glutamicum MB001(DE3)Δ*IOL_*pBiT7-lnoDCI produzierte ca. 1,2 g/L DCI und 0,2 g/L MI ausgehend von 20 g/L Glukose. Dies entspricht einer Effizienz von 6 % berechnet ausgehend von der eingesetzten Glukosekonzentration. Da auch in diesem Prozess Glukose ebenfalls als Kohlenstoffquelle zur Biomasseproduktion genutzt wird, liegt die tatsächliche Effizienz der Produktbildung ausgehend von der nicht zur Biomassebildung genutzen Glukosekonzentration höher.

Durch geeignete biotechnologische Prozessführung lassen sich diese Produktbildungen noch weiter optimieren. Eine vorteilhafte Variante ist beispielweise die Herstellung von DCI in einer für den Fachmann bekannten und etablierten fed-batch-Kultivierung, bei der eine erhöhte Anfangskonzentration von Glukose (~ 40 g/L) und anschließend Glukose in einer Konzentration von 1 - 4 g/L*h kontinuierlich zugefütter wird.

Die Aufteilung der Gene von ino1 und OEPa-b auf dem erfindungsgemäßen Vektor zur poly-cistronen Expression pMKEx2-BiT7 (Fig. 6), welcher die Expression von ino1 und der OEPa-b Gene jeweils unter der Kontrolle eines eigenen T7 Promoters ermöglicht, wie gezeigt in pBiT7-InoDCI (Fig. 7), führt zu einer 70% höheren DCI Produktion. Dies zeigt eindeutig die Vorteilhaftigkeit des erfindungsgemäß poly-cistronen Vektors pMKEx2-BiT7.

**Tabelle 1**

| **Stamm** | **Beschreibung** | **Referenz** |
|---|---|---|
| E. coli DH5α | F- Φ80dlacΔ(lacZ)M15 Δ(lacZYA-argF) U169 endA1 recA1 hsdR17 (r_{K}⁻, m_{K}⁺) deoR thi-1 phoA supE44 λ⁻ gyrA96 relA1 | Hanahan, D., Studies on transformation of Escherichia coli with plasmids. J. Mol. Biol. 166, 557-580, 1983 |
| E. coli BL21 (DE3) | F- ompT hsdSB(rB-mB-) gal dem (lclts857) ind1 Sam7 nin5 lacUV5-T7 Gen 1 | Studier, F. W. & Moffatt, B. A. (1986). Use of bacteriophage T7 RNA polymerase to direct selective high-level expression of cloned genes. J. Mol. Biol. 189, 113-130. |
| C. glutamicum MB001(DE3) | Derivat des Prophagen-freien C. glutamicum Stammes MB001 mit einem chromosomal kodierten E. coli lacl-Gen unter Kontrolle seines nativen Promotors, gefolgt von dem T7-RNA-Polymerase-Gen unter Kontrolle des lacUV5-Promotors | Kortmann, Maike, et al. "A chromosomally encoded T 7 RNA polymerase-dependent gene expression system for Corynebacterium glutamicum: construction and comparative evaluation at the single-cell level", Microbial biotechnology 8.2 (2015): 253-265 |
| C. glutamicum MB001(DE3)Δiol1Δiol2 | Derivat von C. glutamicum MB001(DE3), bei dem beide Inositol-Cluster: iol1 cg0196-cg0212 und iol2 cg3389-cg3392 deletiert sind. Dies umfasst die Deletion des IolR-Regulators (cg0196), resultierend in einer konstitutiven Expression des Inositol-Transporters IolT1 | Ramp P. et al. 2021, "Metabolic engineering of Corynebacterium glutamicum for production of scyllo-inositol, a drug candidate against Alzheimer's disease, Metabolic Engineering 67 (2021): 173-1852021 |
| C. glutamicum MB001(DE3)ΔIOL | Derivat von C. glutamicum MB001(DE3)Δiol1Δiol2, in dem zusätzlich die Inositol-Dehydrogenase cg2312-cg2313 deletiert ist, so dass der Abbau von Inositolen ausgeschaltet ist. | erfindungsgemäß |
| C. glutamicum MB001(DE3)ΔIOL_OEPa-b | Derivat von C. glutamicum MB001(DE3) ΔIOL, indem der Abbau von Inositolen durch die Deletion der Genloci cg0196-cg0212, cg2312-cg2313 und cg3389-cg3392 ausgeschaltet ist, und der zusätzlich die beiden Nukleinsäuresequenzen kodierend für die erfindungsgemäßen NAD⁺- und NADPH-abhängigen Dehydrogenasen trägt. | erfindungsgemäß |
| C. glutamicum MB001(DE3)ΔIOL_InoDCI | Derivat von C. glutamicum MB001(DE3)ΔIOL, indem der Abbau von Inositolen durch die Deletion der Genloci cg0196-cg0212, cg2312-cg2313 und cg3389-cg3392 ausgeschaltet ist, und der zusätzlich je eine Nukleinsäuresequenz kodierend für eine erfindungsgemäße NAD⁺-abhängige und eine erfindungsgemäße NADPH-abhängigen Dehydrogenase sowie eine Nukleinsäuresequenz kodierend für eine myo-Inositol-1-Phosphat-Synthase (!no1) unter der Kontrolle eines gemeinsamen T7-Promotors trägt. | erfindungsgemäß |
| C. glutamicum MB001(DE3)ΔIOL_BiT7Ino DCI | Derivat von C. glutamicum MB001(DE3)ΔIOL, indem der Abbau von Inositolen durch die Deletion der Genloci cg0196-cg0212, cg2312-cg2313 und cg3389-cg3392 ausgeschaltet ist, und der zusätzlich je eine Nukleinsäuresequenz kodierend für eine erfindungsgemäße NAD⁺-abhängige und für eine erfindungsgemäße NADPH-abhängigen Dehydrogenase unter der Kontrolle eines T7-Promotors (T7-2) trägt, und zusätzlich eine Nukleinsäuresequenz kodierend für eine myo-Inositol-1-Phosphat-Synthase (Ino1) unter der Kontrolle eines weiteren T7-Promotors (T7-1) trägt. | erfindungsgemäß |

**Tabelle 2**

| **Plasmid** | **Beschreibung** | **Referenz** |
|---|---|---|
| pK19mobsacB | Kan,^{R} ; Plasmid für den allelischen Austausch in C. glutamicum; (pK18 oriV_{E.c.}, sacB, lacZα) | Schäfer, A. et al., Small mobilizable multi-purpose cloning vectors derived from the Escherichia coli plasmids pK18 and pK19: selection of defined deletions in the chromosome of Corynebacterium glutamicum. Gene. 145, 69-73, 1994 |
| pK19mobSacBΔiol1 | pK19mobSacB-Derivat mit 1000bp homologen Flanken aufwärts und abwärts von cg0196-cg0212 | Ramp P. et al. 2021, "Metabolic engineering of Corynebacterium glutamicum for production of scyllo-inositol, a drug candidate against Alzheimer's disease, Metabolic Engineering 67 (2021): 173-1852021 |
| pK19mobSacBΔiol2 | pK19mobSacB-Derivat mit 1000bp homologen Flanken aufwärts und abwärts von cg3389-3392 | Ramp P. et al. 2021, "Metabolic engineering of Corynebacterium glutamicum for production of scyllo-inositol, a drug candidate against Alzheimer's disease, Metabolic Engineering 67 (2021): 173-1852021 |
| pK19mobsacB-Δcg2312-13 | pK19mobSacB-Derivat mit 1000bp homologen Flanken aufwärts und abwärts von cg2312-2313 | erfindungsgemäß |
| pMKEx2 | Kan^{R}; E. coli-C. glutamicum Shuttle-Vektor (lacl, P_{T7} , lacO1, pHM1519 ori c_{g} ; pACYC177 ori _{Ec} ) zur Expression von Zielgenen unter Kontrolle des T7-Promotors | Kortmann, Maike, et al. "A chromosomally encoded T 7 RNA polymerase-dependent gene expression system for Corynebacterium glutamicum: construction and comparative evaluation at the single-cell level", Microbial biotechnology 8.2 (2015): 253-265 |
| pOEPa-b | pMKEx2-Derivat für die Expression je einer Nukleinsäuresequenz kodierend für eine erfindungsgemäße NAD⁺-abhängige und für eine erfindungsgemäße NADPH-abhängigen Dehydrogenase unter der Kontrolle eines T7-Promotors. | erfindungsgemäß |
| pInoDCI | pMKEx2-Derivat für die Expression einer Nukleinsäuresequenz (ino1) kodierend für eine myo-Inositol-1-Phosphat-Synthase (!no1) sowie je eine Nukleinsäuresequenz kodierend für eine erfindungsgemäße NAD⁺-abhängige und für eine erfindungsgemäße NADPH-abhängigen Dehydrogenase unter der Kontrolle eines gemeinsamen T7-Promotors in einem synthetischen Operon | erfindungsgemäß |
| pMKEx2-BiT7 | pMKEx2-Derivat mit zwei Expressionskassetten enthaltend jeweils wenigstens einen T7-Promotor, einen lacO-Operator, eine multiple cloning-site (MCS) und einen T7-Terminator sowie eine Ribosomenbindungsstelle (RBS), wobei die Expression in entgegengesetzter Richtung erfolqt. | erfindungsgemäß |
| pBiT7-InoDCI | pMKEx-BiT7-Derivat mit einer Nukleinsäuresequenz (ino1) kodierend für eine myo-Inositol-1-Phosphat-Synthase (!no1) unter der Kontrolle eines ersten T7-Prompotors (T7-1) sowie je eine Nukleinsäuresequenz kodierend für eine erfindungsgemäße NAD⁺-abhängige und für eine erfindungsgemäße NADPH-abhängigen Dehydrogenase in einem synthetischen Operon unter der Kontrolle eines zweiten T7-Promotors (T7-2). | erfindungsgemäß |

**Tabelle 3**

| **Sequenz** | **Beschreibung** | **Referenz** |
|---|---|---|
| SEQ ID NO. 1 | Aminosäuresequenz aus *Medicago truncatula* für die Epimerase MtOEPa | Uniprot Accession Nr. G7J0Q8// Genbank Accession Nr. XM 003599277.2 |
| SEQ ID NO. 2 | Aminosäuresequenz aus *Medicago truncatula* für die Epimerase MtOEPb | Uniprot Accession Nr. A0A396HGB9 |
| SEQ ID NO.3 | DNA-Sequenz für den kodierenden Bereich für MtOEPa aus Medicago truncatula ausgehend von der mRNA Sequenz gemäß Genbank Accession No. GHBM01015624.1 | Genbank Accession No. GHBM01015624.1 |
| SEQ ID NO.4 | DNA-Sequenz kodierend für MtOEPb aus *Medicago truncatula* | Genbank Accession Nr. MK174261.1 |
| SEQ ID NO.5 | an die Codon-Usage coryneformer Bakterien angepasste DNA-Sequenz OEPa | erfindungsgemäß |
| SEQ ID NO.6 | an die Codon-Usage coryneformer Bakterien angepasste DNA-Sequenz OEPb | erfindungsgemäß |
| SEQ ID NO. 7 | Aminosäuresequenz für einen myo-Inositol/Proton-Symporter (IolT1) aus Corynebacterium glutamicum | Krings, Eva, et al. "Characterization of myo-inositol utilization by Corynebacterium glutamicum: the stimulon, identification of transporters, and influence on L-lysine formation." Journal of bacteriology 188.23 (2006): 8054-8061. |
| SEQ ID NO. 8 | Aminosäuresequenz für einen myo-Inositol/Proton-Symporter IolT2 aus Co-rynebacterium glutamicum | Krings, Eva, et al. "Characterization of myo-inositol utilization by Corynebacterium glutamicum: the stimulon, identification of transporters, and influence on L-lysine formation." Journal of bacteriology 188.23 (2006): 8054-8061. |
| SEQ ID NO. 9 | DNA-Sequenz des kodierenden Bereichs für (i-olT1) aus Corynebacteri-um glutamicum (cg0223) | Krings, Eva, et al. "Characterization of myo-inositol utilization by Corynebacterium glutamicum: the stimulon, identification of transporters, and influence on L-lysine formation." Journal of bacteriology 188.23 (2006): 8054-8061. |
| SEQ ID NO. 10 | DNA-Sequenz des kodierenden Bereichs für (i-olT2) aus Corynebacterium glutamicum (cg3387) | Krings, Eva, et al. "Characterization of myo-inositol utilization by Corynebacterium glutamicum: the stimulon, identification of transporters, and influence on L-lysine formation." Journal of bacteriology 188.23 (2006): 8054-8061. |
| SEQ ID NO. 11 | Aminosäuresequenz einer myo-Inositol-1-Phosphat-Synthase (Ino1) aus Co-rynebacterium glutamicum | Accession No. GenBank: BAC00390.1 |
| SEQ ID NO. 12 | DNA-Sequenz des kodierenden Bereichs für eine myo-Inositol-1-Phosphat-Synthase (ino1) aus Corynebacterium glutamicum (cq3323) | Accession No. Gene ID: 69623269 |
| SEQ ID NO. 13 | Aminosäuresequenz einer putativen myo-Inositol-1-Phosphat-Phosphatase (Inositol-Monophosphatase) aus Corynebacterium glutamicum | Accession No. BAB99488.1 |
| SEQ ID NO. 14 | DNA-Sequenz des kodierenden Bereichs für eine putative myo-Inositol-1-Phosphat-Phosphatase (Inositol-Monophosphatase) aus Corynebacterium glutamicum (cg2298) | Accession No. KEGG T00244: cq2298 (qenome.jp) |
| SEQ ID NO. 15 | DNA Fragment mit flankierenden Bereichen des Genlocus cg2312-13, welches in den Vektor pK19mobSacB kloniert wurde, resultierend in pK19mobSacBΔcg2312-13 zur Herstellung von Corynebacterium glutamicum | erfindungsgemäß |
| SEQ ID NO. 16 | P001_Δiol1_FW1 | erfindungsgemäß |
| SEQ ID NO. 17 | P002_Δiol1_RV1 | erfindungsgemäß |
| SEQ ID NO. 18 | P003_Δiol1_FW2 | erfindungsgemäß |
| SEQ ID NO. 19 | P004_Δiol1_RV2 | erfindungsgemäß |
| SEQ ID NO. 20 | P007_ΔoxiC-E_FW1 | erfindungsgemäß |
| SEQ ID NO. 21 | P008_ΔoxiC-E_RV1 | erfindungsgemäß |
| SEQ ID NO. 22 | P009_ΔoxiC-E_FW2 | erfindungsgemäß |
| SEQ ID NO. 23 | P010_ΔoxiC-E_RV2 | erfindungsgemäß |
| SEQ ID NO. 24 | P011_pK19_1 | erfindungsgemäß |
| SEQ ID NO. 25 | P012_pK19_2 | erfindungsgemäß |
| SEQ ID NO. 26 | P013_Col_deliol_FW | erfindungsgemäß |
| SEQ ID NO. 27 | P014_Col_deliolR-E2_RV | erfindungsgemäß |
| SEQ ID NO. 28 | P015_Col_deloxiC-E_FW | erfindungsgemäß |
| SEQ ID NO. 29 | P016_Col_deloxiC-E_RV1 | erfindungsgemäß |
| SEQ ID NO. 30 | P01_pK19Δcg2312-13_FW1 | erfindungsgemäß |
| SEQ ID NO. 31 | P02_pK19Δcg2312-13_RV1 | erfindungsgemäß |
| SEQ ID NO. 32 | P03_pK19Δcg2312-13_FW2 | erfindungsgemäß |
| SEQ ID NO. 33 | P04_pK19Δcg2312-13_RV2 | erfindungsgemäß |
| SEQ ID NO. 34 | P05_ΔCg2312-13_FW | erfindungsgemäß |
| SEQ ID NO. 35 | P06_ΔCg2312-13_RV | erfindungsgemäß |
| SEQ ID NO. 36 | P07_pOEPa_FW | erfindungsgemäß |
| SEQ ID NO. 37 | P08_pOEPa_RV | erfindungsgemäß |
| SEQ ID NO. 38 | P09_pOEPb_FW | erfindungsgemäß |
| SEQ ID NO. 39 | P10_pOEPb_RV | erfindungsgemäß |
| SEQ ID NO. 40 | P15_pInoDCI_FW1 | erfindungsgemäß |
| SEQ ID NO. 41 | P16_pInoDCI_RV | erfindungsgemäß |
| SEQ ID NO. 42 | P17_pInoDCI_FW2 | erfindungsgemäß |
| SEQ ID NO. 43 | P10_pOEPab_RV2 | erfindungsgemäß |
| SEQ ID NO. 44 | P18_pMKEx2-BiT7_FW1 | erfindungsgemäß |
| SEQ ID NO. 45 | P19_pMKEx2-BiT7_RV1 | erfindungsgemäß |
| SEQ ID NO. 46 | P20_pMKEx2-BiT7_FW2 | erfindungsgemäß |
| SEQ ID NO. 47 | P21_pMKEx2-BiT7_RV2 | erfindungsgemäß |
| SEQ ID NO. 48 | P15_pInoDCI_FW1 | erfindungsgemäß |
| SEQ ID NO. 49 | P22_pBiT7-InoDCI_RV1 | erfindungsgemäß |
| SEQ ID NO. 50 | P23_pBiT7-InoDCI_FW2 | erfindungsgemäß |
| SEQ ID NO. 51 | P24_pBiT7-InoDCI_RV2 | erfindungsgemäß |

## Patentansprüche

1. Genetisch modifizierter Mikroorganismus, **dadurch gekennzeichnet, dass** er eine NAD⁺-abhängige Dehydrogenase-Aktivität zur Umsetzung von *myo*-Inositol zu 1-Keto-D-*chiro*-Inositol und eine NADPH-abhängige Dehydrogenase-Aktivität zur Umsetzung von 1-Keto-D-*chiro*-Inositol zur D-*chiro*-Inositol exprimiert.

2. Genetisch modifizierter Mikroorganismus gemäß Anspruch 1, **dadurch gekennzeichnet, dass** er zusätzlich Inositol-Transporter aufweist und/oder eine Nukleinsäuresequenz kodierend für Inositol-Transporter exprimiert.

3. Genetisch modifizierter Mikroorganismus gemäß einem der Ansprüche 1 oder 2, **dadurch gekennzeichnet, dass** zusätzlich der Abbau von Inositolen ausgeschaltet ist, derart dass die Aktivität der am Abbau von Inositolen beteiligten Enzyme ausgeschaltet oder die für sie kodierenden Nukleinsäuren teilweise oder ganz aus dem Genom deletiert sind.

4. Genetisch modifizierter Mikroorganismus gemäß einem der Ansprüche 1 - 3, **dadurch gekennzeichnet, dass** die Nukleinsäuren cg0196-cg0212, cg2312-cg2313 und cg3389-cg3392 teilweise oder ganz aus dem Genom deletiert sind.

5. Genetisch modifizierter Mikroorganismus gemäß einem der Ansprüche 1 - 4, **dadurch gekennzeichnet, dass** die Aminosäuresequenz der NAD⁺-abhängigen Dehydrogenase eine Identität von wenigstens 80% zur Aminosäuresequenz gemäß SEQ ID NO. 1 und die Aminosäuresequenz der NADPH-abhängigen Dehydrogenase eine Identität von wenigstens 80% zur Aminosäuresequenz gemäß SEQ ID NO. 2 aufweist.

6. Genetisch modifizierter Mikroorganismus gemäß einem der Ansprüche 1 - 5, **dadurch gekennzeichnet, dass** er eine NAD⁺-abhängige Dehydrogenase kodiert durch eine Nukleinsäuresequenz mit einer Identität von wenigstens 70% gemäß SEQ ID NO. 3 und eine NADPH-abhängige Dehydrogenase kodiert durch eine Nukleinsäuresequenz mit einer Identität von wenigstens 70% gemäß SEQ ID NO. 4 aufweist.

7. Genetisch modifizierter Mikroorganismus gemäß einem der Ansprüche 1 - 6, **dadurch gekennzeichnet, dass** er eine Aminosäuresequenz der NAD⁺-abhängigen Dehydrogenase gemäß SEQ ID NO. 1 und eine Aminosäuresequenz der NADPH-abhängigen Dehydrogenase gemäß SEQ ID NO. 2 aufweist.

8. Genetisch modifizierter Mikroorganismus gemäß einem der Ansprüche 1 - 7, ausgewählt aus der Gruppe enthaltend coryneforme Bakterien, bevorzugt Corynebacteriaceae oder Brevibacteriaceae, Bacillaceae, Enterobacteriaceae, Zymomonadaceae, Methylobacteriaceae, Burkholderiaceae, Vibrionaceae, Clostridiaceae, Pseudomonadaceae und Acetobacteraceae.

9. Genetisch modifizierter Mikroorganismus gemäß einem der Ansprüche 1 - 8, ausgewählt aus der Gruppe enthaltend Corynebacterium glutamicum, Corynebacterium acetoglutamicum, Brevibacterium flavum, Brevibacterium lactofermentum und Brevibacterium divaricatum.

10. Genetisch modifizierter Mikroorganismus gemäß einem der Ansprüche 1 - 9, **dadurch gekennzeichnet, dass** er eine NAD⁺-abhängige Dehydrogenase kodiert durch eine Nukleinsäuresequenz gemäß SEQ ID NO. 5 und eine NADPH-abhängige Dehydrogenase kodiert durch eine Nukleinsäuresequenz gemäß SEQ ID NO. 6 exprimiert.

11. Genetisch modifizierter Mikroorganismus gemäß einem der Ansprüche 1 - 10, **dadurch gekennzeichnet, dass** er zusätzlich eine gesteigerte enzymatische Aktivität einer myo-Inositol-1-Phosphat-Synthase (Ino1) aufweist, so dass er ausgehend von Glukosebausteinen enthaltenden Zuckern, wie Saccharose, Glukose oder auch Maltose, diese über Glukose-6-Phosphat zu myo-Inositol-1-Phosphat umsetzt.

12. Genetisch modifizierter Mikroorganismus gemäß einem der Ansprüche 1 - 11, **dadurch gekennzeichnet, dass** er zusätzlich eine Aktivität einer putativen myo-Inositol-1-Phosphat Phosphatase (Inositol-Monophosphatase) aufweist, so dass er myo-Inositol-1-Phosphat zu freiem myo-Inositol umsetzt.

13. Genetisch modifizierter Mikroorganismus nach einem der Ansprüche 1 - 12, **dadurch gekennzeichnet, dass** er die Nukleinsäuresequenzen kodierend für eine NAD⁺-abhängige Dehydrogenase-Aktivität, für eine NADPH-abhängige Dehydrogenase-Aktivität, für Inositol-Transporter, für eine myo-Inositol-1-Phosphat-Synthase (Ino1) und für eine putative myo-Inositol-1-Phosphat Phosphatase (Inositol-Monophosphatase) chromosomal-kodiert und/oder extrachromosomal-kodiert, bevorzugt Vektor-kodiert, aufweist und exprimiert.

14. Vektor enthaltend eine Nukleinsäuresequenz kodierend für eine NAD⁺-abhängige Dehydrogenase mit einer Aktivität zur Umsetzung von *myo*-Inositol zu 1-Keto-D-*chiro*-Inositol und/oder eine Nukleinsäuresequenz kodierend für eine NADPH-abhängige Dehydrogenase mit einer Aktivität zur Umsetzung von 1-Keto-D-*chiro-*Inositol zur D-*chiro*-Inositol.

15. Vektor gemäß Anspruch 14, **dadurch gekennzeichnet, dass** er zusätzlich Inositol-Transporter aufweist und/oder eine Nukleinsäuresequenz kodierend für Inositol-Transporter exprimiert.

16. Vektor gemäß einem der Ansprüche 14 oder 15, **dadurch gekennzeichnet, dass** er eine NAD⁺-abhängige Dehydrogenase kodiert durch eine Nukleinsäuresequenz mit einer Identität von wenigstens 70% gemäß SEQ ID NO. 3 und/oder eine NADPH-abhängige Dehydrogenase kodiert durch eine Nukleinsäuresequenz mit einer Identität von wenigstens 70% gemäß SEQ ID NO. 4 aufweist.

17. Vektor gemäß einem der Ansprüche 14 - 16, **dadurch gekennzeichnet, dass** er eine NAD⁺-abhängige Dehydrogenase kodiert durch eine Nukleinsäuresequenz gemäß SEQ ID NO. 5 und/oder eine NADPH-abhängige Dehydrogenase kodiert durch eine Nukleinsäuresequenz gemäß SEQ ID NO. 6 aufweist.

18. Vektor zur poly-cistronen Genexpression in Mikroorganismen ausgewählt aus der Gruppe enthaltend coryneforme Bakterien und Enterobacteriaceae, **dadurch gekennzeichnet, dass** er wenigstens zwei Expressionskassetten aufweist, jeweils enthaltend wenigstens einen Promotor, einen LacO-Operator, eine multiplecloning-site (MCS) sowie einen Transkriptionsterminator und eine Ribosomenbindungsstelle, wobei die Expressionskassetten für eine gleichgerichtete oder gegengerichtete Expression kodierender Nukleinsäuresequenzen angeordnet sind.

19. Vektor gemäß Anspruch 18, enthaltend wenigstens einen trp-Promotor oder einen lac-Promotor oder einen T7-Promotor oder Kombinationen davon.

20. Vektor gemäß einem der Ansprüche 18 oder 19, enthaltend zwei T7-Promotoren.

21. Vektor gemäß einem der Ansprüche 14 - 20 zur Expression in einem genetisch modifizierten Mikroorganismus gemäß einem der Ansprüche 1 - 13.

22. Genetisch modifizierter Mikroorganismus gemäß einem der Ansprüche 1 - 13, enthaltend einen Vektor gemäß einem der Ansprüche 14 - 20.

23. Verwendung eines genetisch modifizierten Mikroorganismus gemäß einem der Ansprüche 1 - 13 und/oder der Vektoren gemäß einem der Ansprüche 14 - 20 und/oder eines Mikroorganismus gemäß Anspruch 22 für die biotechnologische Herstellung von D-*chiro*-Inositol.

24. Verwendung eines Proteins mit einer NAD⁺-abhängige Dehydrogenase-Aktivität zur Umsetzung von *myo*-Inositol zu 1-Keto-D-*chiro*-Inositol gemeinsam mit einem weiteren Protein mit einer NADPH-abhängige Dehydrogenase-Aktivität zur Umsetzung von 1-Keto-D-*chiro*-Inositol zur D-*chiro*-Inositol für die in-vitro-Herstellung von D-*chiro*-Inositol.

25. Verwendung der Proteine gemäß Anspruch 24, wobei die Proteine eine Aminosäuresequenz mit einer Identität von wenigstens 80% zur Sequenz gemäß SEQ ID NO. 1 und eine Aminosäuresequenz mit einer Identität von wenigstens 80% zur Aminosäuresequenz gemäß SEQ ID NO. 2 aufweisen.

26. Verwendung von Nukleinsäuresequenzen für die biotechnologische oder in-vitro-Herstellung von D-*chiro*-Inositol, ausgewählt aus der Gruppe enthaltend
a) Nukleinäuresequenzen mit mindestens 70 % Sequenzidentität zu den Gensequenzen gemäß SEQ. ID. NO. 3 bzw. SEQ. ID.NO.4, oder
b) Nukleinsäuresequenzen, die unter stringenten Bedingungen mit komplementären Sequenzen zu den Nukleinsäuresequenzen gemäß SEQ ID NO. 3 bzw. SEQ. ID. NO. 4 oder mit Fragmenten davon hybridisiert, oder
c) Nukleinsäuresequenzen gemäß SEQ ID NO. 3 bzw. SEQ. ID. NO. 4, die sich jedoch von diesen durch die Degeneriertheit des genetischen Codes oder durch funktionsneutrale Mutationen unterscheiden, oder
d) Nukleinsäuresequenzen gemäß SEQ ID NO.5 bzw. gemäß SEQ ID NO. 6

27. Verwendung von Nukleinsäuresequenzen gemäß Anspruch 26 zur Herstellung von Proteinen mit einer NAD⁺-abhängige Dehydrogenase-Aktivität zur Umsetzung von *myo*-Inositol zu 1-Keto-D-*chiro*-Inositol, und mit einer NADPH-abhängige Dehydrogenase-Aktivität zur Umsetzung von 1-Keto-D-*chiro*-Inositol zur D-*chiro*-Inositol, für den Einsatz in einem in-vitro-Verfahren zur Herstellung von D-*chiro*-Inositol.

28. Verwendung von Nukleinsäuresequenzen gemäß Anspruch 26 zur Herstellung von genetisch modifizierten Mikroorganismen gemäß einem der Ansprüche 1 - 13 und 22 und/oder zur Herstellung von Vektoren gemäß einem der Ansprüche 14 - 21 zum Einsatz bei der biotechnologischen Herstellung von D-*chiro*-Inositol.

29. Verfahren zur biotechnologischen Herstellung von D-*chiro*-Inositol, umfassend
a) die Bereitstellung eines genetisch modifizierten Mikroorganismus gemäß einem der Ansprüche 1 - 13,
b) die Kultivierung des genetisch modifizierten Mikroorganismus aus a) in einem Medium welches ein geeignetes Ausgangssubstrat für die Bildung von D-*chiro*-Inositol enthält, ausgewählt aus *myo*-Inositol, Glukose oder Glukosebausteine enthaltenden Zuckern,
c) die Isolierung, und optional die Aufreinigung, des gebildeten D-*chiro*-Inositols.

30. Verfahren gemäß Anspruch 29, umfassend eine enzymatische Umsetzung ausgehend von Glukosebausteinen enthaltenden Zuckern, wie Saccharose, Glukose und Maltose, zu *myo*-Inositol, die während Schritt b) stattfindet.
